(19) Europäisches Patentamt
European Patent Office
Office européen des brevets

(11) **EP 3 961 217 A1**

(12) **EUROPEAN PATENT APPLICATION**
published in accordance with Art. 153(4) EPC

(43) Date of publication:
**02.03.2022 Bulletin 2022/09**

(21) Application number: **20796111.1**

(22) Date of filing: **24.04.2020**

(51) International Patent Classification (IPC):
*G01N 33/68* *(2006.01)*

(52) Cooperative Patent Classification (CPC):
**G01N 33/68**

(86) International application number:
**PCT/JP2020/017595**

(87) International publication number:
**WO 2020/218465 (29.10.2020 Gazette 2020/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.04.2019 JP 2019086223**

(71) Applicants:
• **CHUGAI SEIYAKU KABUSHIKI KAISHA
Tokyo 115-8543 (JP)**
• **Jichi Medical University
Tokyo 102-0093 (JP)**
• **National Institutes of Biomedical Innovation,
Health and Nutrition
Ibaraki-shi, Osaka 567-0085 (JP)**

(72) Inventors:
• **KOBAYASHI, Shinta
Kamakura-shi, Kanagawa 247-8530 (JP)**
• **NAGANO, Kohji
Kamakura-shi, Kanagawa 247-8530 (JP)**
• **OHMORI, Hiroshi
Kamakura-shi, Kanagawa 247-8530 (JP)**
• **KAKIUCHI, Ayako
Tokyo 103-8324 (JP)**
• **KONNO, Ryo
Saitama-shi, Saitama 330-8503 (JP)**
• **SANKAI, Tadashi
Tukuba-shi, Ibaraki 305-0843 (JP)**

(74) Representative: **Vossius & Partner
Patentanwälte Rechtsanwälte mbB
Siebertstraße 3
81675 München (DE)**

(54) **METHOD FOR DIAGNOSING ENDOMETRIOSIS, DISEASE STATE MONITORING METHOD, AND KIT**

(57) It has been discovered that plasma samples from endometriosis patients contain a number of markers whose abundance is different from those in healthy individuals. It has also been discovered that, by measuring the abundance of those markers, whether or not a subject is affected by endometriosis can be diagnosed, the extent of endometrial fibrosis or adhesion in a subject can be determined, pain of a patient affected or suspected of being affected by endometriosis can be predicted, and pathological conditions of the patient can be monitored.

FIG. 3

EP 3 961 217 A1

## EP 3 961 217 A1

SEQUENCE LISTING

<110>  CHUGAI SEIYAKU KABUSHIKI KAISHA
       JICHI MEDICAL UNIVERSITY
       NATIONAL INSTITUTES OF BIOMEDICAL INNOVATION, HEALTH AND
       NUTRITION

<120>  Diagnostic method, monitoring method of endometriosis, and kit
       thereof

<130>  C1-A1825P

<150>  JP 2019-086223
<151>  2019-04-26

<160>  7

<170>  PatentIn version 3.5

<210>  1
<211>  1745
<212>  PRT
<213>  Homo sapiens

<400>  1

Met Gly Asn Arg Arg Asp Leu Gly Gln Pro Arg Ala Gly Leu Cys Leu
1               5                   10                  15


Leu Leu Ala Ala Leu Gln Leu Leu Pro Gly Thr Gln Ala Asp Pro Val
            20                  25                  30


Asp Val Leu Lys Ala Leu Gly Val Gln Gly Gly Gln Ala Gly Val Pro
            35                  40                  45


Glu Gly Pro Gly Phe Cys Pro Gln Arg Thr Pro Glu Gly Asp Arg Ala
        50                  55                  60


Phe Arg Ile Gly Gln Ala Ser Thr Leu Gly Ile Pro Thr Trp Glu Leu
65                  70                  75                  80


Phe Pro Glu Gly His Phe Pro Glu Asn Phe Ser Leu Leu Ile Thr Leu
                85                  90                  95


Arg Gly Gln Pro Ala Asn Gln Ser Val Leu Leu Ser Ile Tyr Asp Glu
                100                 105                 110


Arg Gly Ala Arg Gln Leu Gly Leu Ala Leu Gly Pro Ala Leu Gly Leu
            115                 120                 125


Leu Gly Asp Pro Phe Arg Pro Leu Pro Gln Gln Val Asn Leu Thr Asp
        130                 135                 140


Gly Arg Trp His Arg Val Ala Val Ser Ile Asp Gly Glu Met Val Thr

```
      145                     150                     155                     160


      Leu Val Ala Asp Cys Glu Ala Gln Pro Pro Val Leu Gly His Gly Pro
                      165                 170                 175


      Arg Phe Ile Ser Ile Ala Gly Leu Thr Val Leu Gly Thr Gln Asp Leu
                      180                 185                 190


      Gly Glu Lys Thr Phe Glu Gly Asp Ile Gln Glu Leu Leu Ile Ser Pro
                  195                 200                 205


      Asp Pro Gln Ala Ala Phe Gln Ala Cys Glu Arg Tyr Leu Pro Asp Cys
          210                 215                 220


      Asp Asn Leu Ala Pro Ala Ala Thr Val Ala Pro Gln Gly Glu Pro Glu
      225                 230                 235                 240


      Thr Pro Arg Pro Arg Arg Lys Gly Lys Gly Lys Gly Arg Lys Lys Gly
                      245                 250                 255


      Arg Gly Arg Lys Gly Lys Gly Arg Lys Lys Asn Lys Glu Ile Trp Thr
                  260                 265                 270


      Ser Ser Pro Pro Pro Asp Ser Ala Glu Asn Gln Thr Ser Thr Asp Ile
                  275                 280                 285


      Pro Lys Thr Glu Thr Pro Ala Pro Asn Leu Pro Pro Thr Pro Thr Pro
          290                 295                 300


      Leu Val Val Thr Ser Thr Val Thr Thr Gly Leu Asn Ala Thr Ile Leu
      305                 310                 315                 320


      Glu Arg Ser Leu Asp Pro Asp Ser Gly Thr Glu Leu Gly Thr Leu Glu
                      325                 330                 335


      Thr Lys Ala Ala Arg Glu Asp Glu Glu Gly Asp Asp Ser Thr Met Gly
                  340                 345                 350


      Pro Asp Phe Arg Ala Ala Glu Tyr Pro Ser Arg Thr Gln Phe Gln Ile
                  355                 360                 365


      Phe Pro Gly Ala Gly Glu Lys Gly Ala Lys Gly Glu Pro Ala Val Ile
                  370                 375                 380


      Glu Lys Gly Gln Gln Phe Glu Gly Pro Pro Gly Ala Pro Gly Pro Gln
      385                 390                 395                 400
```

Gly Val Val Gly Pro Ser Gly Pro Pro Gly Pro Pro Gly Phe Pro Gly
                    405                 410                 415

Asp Pro Gly Pro Pro Gly Pro Ala Gly Leu Pro Gly Ile Pro Gly Ile
            420                 425                 430

Asp Gly Ile Arg Gly Pro Pro Gly Thr Val Ile Met Met Pro Phe Gln
            435                 440                 445

Phe Ala Gly Gly Ser Phe Lys Gly Pro Pro Val Ser Phe Gln Gln Ala
    450                 455                 460

Gln Ala Gln Ala Val Leu Gln Gln Thr Gln Leu Ser Met Lys Gly Pro
465                 470                 475                 480

Pro Gly Pro Val Gly Leu Thr Gly Arg Pro Gly Pro Val Gly Leu Pro
            485                 490                 495

Gly His Pro Gly Leu Lys Gly Glu Glu Gly Ala Glu Gly Pro Gln Gly
            500                 505                 510

Pro Arg Gly Leu Gln Gly Pro His Gly Pro Pro Gly Arg Val Gly Lys
            515                 520                 525

Met Gly Arg Pro Gly Ala Asp Gly Ala Arg Gly Leu Pro Gly Asp Thr
    530                 535                 540

Gly Pro Lys Gly Asp Arg Gly Phe Asp Gly Leu Pro Gly Leu Pro Gly
545                 550                 555                 560

Glu Lys Gly Gln Arg Gly Asp Phe Gly His Val Gly Gln Pro Gly Pro
            565                 570                 575

Pro Gly Glu Asp Gly Glu Arg Gly Ala Glu Gly Pro Pro Gly Pro Thr
            580                 585                 590

Gly Gln Ala Gly Glu Pro Gly Pro Arg Gly Leu Leu Gly Pro Arg Gly
            595                 600                 605

Ser Pro Gly Pro Thr Gly Arg Pro Gly Val Thr Gly Ile Asp Gly Ala
    610                 615                 620

Pro Gly Ala Lys Gly Asn Val Gly Pro Pro Gly Glu Pro Gly Pro Pro
625                 630                 635                 640

Gly Gln Gln Gly Asn His Gly Ser Gln Gly Leu Pro Gly Pro Gln Gly
            645                 650                 655

```
Leu Ile Gly Thr Pro Gly Glu Lys Gly Pro Pro Gly Asn Pro Gly Ile
        660             665             670

Pro Gly Leu Pro Gly Ser Asp Gly Pro Leu Gly His Pro Gly His Glu
        675             680             685

Gly Pro Thr Gly Glu Lys Gly Ala Gln Gly Pro Pro Gly Ser Ala Gly
        690             695             700

Pro Pro Gly Tyr Pro Gly Pro Arg Gly Val Lys Gly Thr Ser Gly Asn
705             710             715             720

Arg Gly Leu Gln Gly Glu Lys Gly Glu Lys Gly Glu Asp Gly Phe Pro
            725             730             735

Gly Phe Lys Gly Asp Val Gly Leu Lys Gly Asp Gln Gly Lys Pro Gly
            740             745             750

Ala Pro Gly Pro Arg Gly Glu Asp Gly Pro Glu Gly Pro Lys Gly Gln
            755             760             765

Ala Gly Gln Ala Gly Glu Glu Gly Pro Pro Gly Ser Ala Gly Glu Lys
    770             775             780

Gly Lys Leu Gly Val Pro Gly Leu Pro Gly Tyr Pro Gly Arg Pro Gly
785             790             795             800

Pro Lys Gly Ser Ile Gly Phe Pro Gly Pro Leu Gly Pro Ile Gly Glu
            805             810             815

Lys Gly Lys Ser Gly Lys Thr Gly Gln Pro Gly Leu Glu Gly Glu Arg
            820             825             830

Gly Pro Pro Gly Ser Arg Gly Glu Arg Gly Gln Pro Gly Ala Thr Gly
            835             840             845

Gln Pro Gly Pro Lys Gly Asp Val Gly Gln Asp Gly Ala Pro Gly Ile
    850             855             860

Pro Gly Glu Lys Gly Leu Pro Gly Leu Gln Gly Pro Pro Gly Phe Pro
865             870             875             880

Gly Pro Lys Gly Pro Pro Gly His Gln Gly Lys Asp Gly Arg Pro Gly
            885             890             895

His Pro Gly Gln Arg Gly Glu Leu Gly Phe Gln Gly Gln Thr Gly Pro
            900             905             910
```

```
Pro Gly Pro Ala Gly Val Leu Gly Pro Gln Gly Lys Thr Gly Glu Val
        915                 920                 925

Gly Pro Leu Gly Glu Arg Gly Pro Pro Gly Pro Pro Gly Pro Pro Gly
        930                 935                 940

Glu Gln Gly Leu Pro Gly Leu Glu Gly Arg Glu Gly Ala Lys Gly Glu
945                     950                 955                 960

Leu Gly Pro Pro Gly Pro Leu Gly Lys Glu Gly Pro Ala Gly Leu Arg
                965                 970                 975

Gly Phe Pro Gly Pro Lys Gly Gly Pro Gly Asp Pro Gly Pro Thr Gly
            980                 985                 990

Leu Lys Gly Asp Lys Gly Pro Pro  Gly Pro Val Gly Ala  Asn Gly Ser
        995                 1000                1005

Pro Gly  Glu Arg Gly Pro Leu  Gly Pro Ala Gly Gly  Ile Gly Leu
    1010                1015                1020

Pro Gly  Gln Ser Gly Ser Glu  Gly Pro Val Gly Pro  Ala Gly Lys
    1025                1030                1035

Lys Gly  Ser Arg Gly Glu Arg  Gly Pro Pro Gly Pro  Thr Gly Lys
    1040                1045                1050

Asp Gly  Ile Pro Gly Pro Leu  Gly Pro Leu Gly Pro  Pro Gly Ala
    1055                1060                1065

Ala Gly  Pro Ser Gly Glu Glu  Gly Asp Lys Gly Asp  Val Gly Ala
    1070                1075                1080

Pro Gly  His Lys Gly Ser Lys  Gly Asp Lys Gly Asp  Ala Gly Pro
    1085                1090                1095

Pro Gly  Gln Pro Gly Ile Arg  Gly Pro Ala Gly His  Pro Gly Pro
    1100                1105                1110

Pro Gly  Ala Asp Gly Ala Gln  Gly Arg Arg Gly Pro  Pro Gly Leu
    1115                1120                1125

Phe Gly  Gln Lys Gly Asp Asp  Gly Val Arg Gly Phe  Val Gly Val
    1130                1135                1140

Ile Gly  Pro Pro Gly Leu Gln  Gly Leu Pro Gly Pro  Pro Gly Glu
```

48

1145                          1150                          1155

Lys Gly  Glu Val Gly Asp Val  Gly Ser Met Gly Pro  His Gly Ala
    1160              1165              1170

Pro Gly  Pro Arg Gly Pro Gln  Gly Pro Thr Gly Ser  Glu Gly Thr
    1175              1180              1185

Pro Gly  Leu Pro Gly Gly Val  Gly Gln Pro Gly Ala  Val Gly Glu
    1190              1195              1200

Lys Gly  Glu Arg Gly Asp Ala  Gly Asp Pro Gly Pro  Pro Gly Ala
    1205              1210              1215

Pro Gly  Ile Pro Gly Pro Lys  Gly Asp Ile Gly Glu  Lys Gly Asp
    1220              1225              1230

Ser Gly  Pro Ser Gly Ala Ala  Gly Pro Pro Gly Lys  Lys Gly Pro
    1235              1240              1245

Pro Gly  Glu Asp Gly Ala Lys  Gly Ser Val Gly Pro  Thr Gly Leu
    1250              1255              1260

Pro Gly  Asp Leu Gly Pro Pro  Gly Asp Pro Gly Val  Ser Gly Ile
    1265              1270              1275

Asp Gly  Ser Pro Gly Glu Lys  Gly Asp Pro Gly Asp  Val Gly Gly
    1280              1285              1290

Pro Gly  Pro Pro Gly Ala Ser  Gly Glu Pro Gly Ala  Pro Gly Pro
    1295              1300              1305

Pro Gly  Lys Arg Gly Pro Ser  Gly His Met Gly Arg  Glu Gly Arg
    1310              1315              1320

Glu Gly  Glu Lys Gly Ala Lys  Gly Glu Pro Gly Pro  Asp Gly Pro
    1325              1330              1335

Pro Gly  Arg Thr Gly Pro Met  Gly Ala Arg Gly Pro  Pro Gly Arg
    1340              1345              1350

Val Gly  Pro Glu Gly Leu Arg  Gly Ile Pro Gly Pro  Val Gly Glu
    1355              1360              1365

Pro Gly  Leu Leu Gly Ala Pro  Gly Gln Met Gly Pro  Pro Gly Pro
    1370              1375              1380

```
Leu Gly Pro Ser Gly Leu Pro Gly Leu Lys Gly Asp Thr Gly Pro
    1385             1390             1395

Lys Gly Glu Lys Gly His Ile Gly Leu Ile Gly Leu Ile Gly Pro
    1400             1405             1410

Pro Gly Glu Ala Gly Glu Lys Gly Asp Gln Gly Leu Pro Gly Val
    1415             1420             1425

Gln Gly Pro Pro Gly Pro Lys Gly Asp Pro Gly Pro Pro Gly Pro
    1430             1435             1440

Ile Gly Ser Leu Gly His Pro Gly Pro Pro Gly Val Ala Gly Pro
    1445             1450             1455

Leu Gly Gln Lys Gly Ser Lys Gly Ser Pro Gly Ser Met Gly Pro
    1460             1465             1470

Arg Gly Asp Thr Gly Pro Ala Gly Pro Pro Gly Pro Pro Gly Ala
    1475             1480             1485

Pro Ala Glu Leu His Gly Leu Arg Arg Arg Arg Arg Phe Val Pro
    1490             1495             1500

Val Pro Leu Pro Val Val Glu Gly Gly Leu Glu Glu Val Leu Ala
    1505             1510             1515

Ser Leu Thr Ser Leu Ser Leu Glu Leu Glu Gln Leu Arg Arg Pro
    1520             1525             1530

Pro Gly Thr Ala Glu Arg Pro Gly Leu Val Cys His Glu Leu His
    1535             1540             1545

Arg Asn His Pro His Leu Pro Asp Gly Glu Tyr Trp Ile Asp Pro
    1550             1555             1560

Asn Gln Gly Cys Ala Arg Asp Ser Phe Arg Val Phe Cys Asn Phe
    1565             1570             1575

Thr Ala Gly Gly Glu Thr Cys Leu Tyr Pro Asp Lys Lys Phe Glu
    1580             1585             1590

Ile Val Lys Leu Ala Ser Trp Ser Lys Glu Lys Pro Gly Gly Trp
    1595             1600             1605

Tyr Ser Thr Phe Arg Arg Gly Lys Lys Phe Ser Tyr Val Asp Ala
    1610             1615             1620
```

```
Asp Gly  Ser Pro Val Asn Val  Val Gln Leu Asn Phe  Leu Lys Leu
    1625             1630              1635

Leu Ser  Ala Thr Ala Arg Gln  Asn Phe Thr Tyr Ser  Cys Gln Asn
    1640             1645              1650

Ala Ala  Ala Trp Leu Asp Glu  Ala Thr Gly Asp Tyr  Ser His Ser
    1655             1660              1665

Ala Arg  Phe Leu Gly Thr Asn  Gly Glu Glu Leu Ser  Phe Asn Gln
    1670             1675              1680

Thr Thr  Ala Ala Thr Val Ser  Val Pro Gln Asp Gly  Cys Arg Leu
    1685             1690              1695

Arg Lys  Gly Gln Thr Lys Thr  Leu Phe Glu Phe Ser  Ser Ser Arg
    1700             1705              1710

Ala Gly  Phe Leu Pro Leu Trp  Asp Val Ala Ala Thr  Asp Phe Gly
    1715             1720              1725

Gln Thr  Asn Gln Lys Phe Gly  Phe Glu Leu Gly Pro  Val Cys Phe
    1730             1735              1740

Ser Ser
    1745


<210>  2
<211>  429
<212>  PRT
<213>  Artificial Sequence

<220>
<223>  an artificially synthesized sequence

<400>  2

His Met Gly Arg Glu Gly Arg Glu Gly Glu Lys Gly Ala Lys Gly Glu
1                   5                   10                  15

Pro Gly Pro Asp Gly Pro Pro Gly Arg Thr Gly Pro Met Gly Ala Arg
            20                  25                  30

Gly Pro Pro Gly Arg Val Gly Pro Glu Gly Leu Arg Gly Ile Pro Gly
            35                  40                  45

Pro Val Gly Glu Pro Gly Leu Leu Gly Ala Pro Gly Gln Met Gly Pro
        50                  55                  60
```

Pro Gly Pro Leu Gly Pro Ser Gly Leu Pro Gly Leu Lys Gly Asp Thr
65                   70                   75                   80

Gly Pro Lys Gly Glu Lys Gly His Ile Gly Leu Ile Gly Leu Ile Gly
                    85                   90                   95

Pro Pro Gly Glu Ala Gly Glu Lys Gly Asp Gln Gly Leu Pro Gly Val
                    100                  105                  110

Gln Gly Pro Pro Gly Pro Lys Gly Asp Pro Gly Pro Pro Gly Pro Ile
                    115                  120                  125

Gly Ser Leu Gly His Pro Gly Pro Pro Gly Val Ala Gly Pro Leu Gly
                    130                  135                  140

Gln Lys Gly Ser Lys Gly Ser Pro Gly Ser Met Gly Pro Arg Gly Asp
145                  150                  155                  160

Thr Gly Pro Ala Gly Pro Pro Gly Pro Pro Gly Ala Pro Ala Glu Leu
                    165                  170                  175

His Gly Leu Arg Arg Arg Arg Arg Phe Val Pro Val Pro Leu Pro Val
                    180                  185                  190

Val Glu Gly Gly Leu Glu Glu Val Leu Ala Ser Leu Thr Ser Leu Ser
                    195                  200                  205

Leu Glu Leu Glu Gln Leu Arg Arg Pro Pro Gly Thr Ala Glu Arg Pro
                    210                  215                  220

Gly Leu Val Cys His Glu Leu His Arg Asn His Pro His Leu Pro Asp
225                  230                  235                  240

Gly Glu Tyr Trp Ile Asp Pro Asn Gln Gly Cys Ala Arg Asp Ser Phe
                    245                  250                  255

Arg Val Phe Cys Asn Phe Thr Ala Gly Gly Glu Thr Cys Leu Tyr Pro
                    260                  265                  270

Asp Lys Lys Phe Glu Ile Val Lys Leu Ala Ser Trp Ser Lys Glu Lys
                    275                  280                  285

Pro Gly Gly Trp Tyr Ser Thr Phe Arg Arg Gly Lys Lys Phe Ser Tyr
                    290                  295                  300

Val Asp Ala Asp Gly Ser Pro Val Asn Val Val Gln Leu Asn Phe Leu
305                  310                  315                  320

```
Lys Leu Leu Ser Ala Thr Ala Arg Gln Asn Phe Thr Tyr Ser Cys Gln
            325             330             335

Asn Ala Ala Ala Trp Leu Asp Glu Ala Thr Gly Asp Tyr Ser His Ser
            340             345             350

Ala Arg Phe Leu Gly Thr Asn Gly Glu Glu Leu Ser Phe Asn Gln Thr
            355             360             365

Thr Ala Ala Thr Val Ser Val Pro Gln Asp Gly Cys Arg Leu Arg Lys
    370             375             380

Gly Gln Thr Lys Thr Leu Phe Glu Phe Ser Ser Ser Arg Ala Gly Phe
385             390             395             400

Leu Pro Leu Trp Asp Val Ala Ala Thr Asp Phe Gly Gln Thr Asn Gln
            405             410             415

Lys Phe Gly Phe Glu Leu Gly Pro Val Cys Phe Ser Ser
            420             425
```

```
<210>  3
<211>  1316
<212>  PRT
<213>  Artificial sequnce

<220>
<223>  an artificially synthesized sequence

<400>  3
```

```
Met Gly Asn Arg Arg Asp Leu Gly Gln Pro Arg Ala Gly Leu Cys Leu
1               5               10              15

Leu Leu Ala Ala Leu Gln Leu Leu Pro Gly Thr Gln Ala Asp Pro Val
            20              25              30

Asp Val Leu Lys Ala Leu Gly Val Gln Gly Gly Gln Ala Gly Val Pro
            35              40              45

Glu Gly Pro Gly Phe Cys Pro Gln Arg Thr Pro Glu Gly Asp Arg Ala
    50              55              60

Phe Arg Ile Gly Gln Ala Ser Thr Leu Gly Ile Pro Thr Trp Glu Leu
65              70              75              80

Phe Pro Glu Gly His Phe Pro Glu Asn Phe Ser Leu Leu Ile Thr Leu
            85              90              95
```

53

```
Arg Gly Gln Pro Ala Asn Gln Ser Val Leu Leu Ser Ile Tyr Asp Glu
            100                 105                 110

Arg Gly Ala Arg Gln Leu Gly Leu Ala Leu Gly Pro Ala Leu Gly Leu
            115                 120                 125

Leu Gly Asp Pro Phe Arg Pro Leu Pro Gln Gln Val Asn Leu Thr Asp
            130                 135                 140

Gly Arg Trp His Arg Val Ala Val Ser Ile Asp Gly Glu Met Val Thr
145                 150                 155                 160

Leu Val Ala Asp Cys Glu Ala Gln Pro Pro Val Leu Gly His Gly Pro
                165                 170                 175

Arg Phe Ile Ser Ile Ala Gly Leu Thr Val Leu Gly Thr Gln Asp Leu
            180                 185                 190

Gly Glu Lys Thr Phe Glu Gly Asp Ile Gln Glu Leu Leu Ile Ser Pro
            195                 200                 205

Asp Pro Gln Ala Ala Phe Gln Ala Cys Glu Arg Tyr Leu Pro Asp Cys
    210                 215                 220

Asp Asn Leu Ala Pro Ala Ala Thr Val Ala Pro Gln Gly Glu Pro Glu
225                 230                 235                 240

Thr Pro Arg Pro Arg Arg Lys Gly Lys Gly Lys Gly Arg Lys Lys Gly
                245                 250                 255

Arg Gly Arg Lys Gly Lys Gly Arg Lys Lys Asn Lys Glu Ile Trp Thr
            260                 265                 270

Ser Ser Pro Pro Pro Asp Ser Ala Glu Asn Gln Thr Ser Thr Asp Ile
            275                 280                 285

Pro Lys Thr Glu Thr Pro Ala Pro Asn Leu Pro Pro Thr Pro Thr Pro
    290                 295                 300

Leu Val Val Thr Ser Thr Val Thr Thr Gly Leu Asn Ala Thr Ile Leu
305                 310                 315                 320

Glu Arg Ser Leu Asp Pro Asp Ser Gly Thr Glu Leu Gly Thr Leu Glu
                325                 330                 335

Thr Lys Ala Ala Arg Glu Asp Glu Glu Gly Asp Asp Ser Thr Met Gly
            340                 345                 350
```

54

```
Pro Asp Phe Arg Ala Ala Glu Tyr Pro Ser Arg Thr Gln Phe Gln Ile
    355                 360             365

Phe Pro Gly Ala Gly Glu Lys Gly Ala Lys Gly Glu Pro Ala Val Ile
    370                 375             380

Glu Lys Gly Gln Gln Phe Glu Gly Pro Pro Gly Ala Pro Gly Pro Gln
385                 390             395                 400

Gly Val Val Gly Pro Ser Gly Pro Pro Gly Pro Gly Phe Pro Gly
                405             410             415

Asp Pro Gly Pro Pro Gly Pro Ala Gly Leu Pro Gly Ile Pro Gly Ile
            420             425             430

Asp Gly Ile Arg Gly Pro Pro Gly Thr Val Ile Met Met Pro Phe Gln
            435             440             445

Phe Ala Gly Gly Ser Phe Lys Gly Pro Pro Val Ser Phe Gln Gln Ala
    450             455             460

Gln Ala Gln Ala Val Leu Gln Gln Thr Gln Leu Ser Met Lys Gly Pro
465             470             475             480

Pro Gly Pro Val Gly Leu Thr Gly Arg Pro Gly Pro Val Gly Leu Pro
            485             490             495

Gly His Pro Gly Leu Lys Gly Glu Glu Gly Ala Glu Gly Pro Gln Gly
            500             505             510

Pro Arg Gly Leu Gln Gly Pro His Gly Pro Pro Gly Arg Val Gly Lys
    515             520             525

Met Gly Arg Pro Gly Ala Asp Gly Ala Arg Gly Leu Pro Gly Asp Thr
    530             535             540

Gly Pro Lys Gly Asp Arg Gly Phe Asp Gly Leu Pro Gly Leu Pro Gly
545             550             555             560

Glu Lys Gly Gln Arg Gly Asp Phe Gly His Val Gly Gln Pro Gly Pro
            565             570             575

Pro Gly Glu Asp Gly Glu Arg Gly Ala Glu Gly Pro Pro Gly Pro Thr
            580             585             590

Gly Gln Ala Gly Glu Pro Gly Pro Arg Gly Leu Leu Gly Pro Arg Gly
    595             600             605
```

Ser Pro Gly Pro Thr Gly Arg Pro Gly Val Thr Gly Ile Asp Gly Ala
        610                 615                 620

Pro Gly Ala Lys Gly Asn Val Gly Pro Pro Gly Glu Pro Gly Pro Pro
    625                 630                 635                 640

Gly Gln Gln Gly Asn His Gly Ser Gln Gly Leu Pro Gly Pro Gln Gly
                    645                 650                 655

Leu Ile Gly Thr Pro Gly Glu Lys Gly Pro Pro Gly Asn Pro Gly Ile
            660                 665                 670

Pro Gly Leu Pro Gly Ser Asp Gly Pro Leu Gly His Pro Gly His Glu
            675                 680                 685

Gly Pro Thr Gly Glu Lys Gly Ala Gln Gly Pro Pro Gly Ser Ala Gly
    690                 695                 700

Pro Pro Gly Tyr Pro Gly Pro Arg Gly Val Lys Gly Thr Ser Gly Asn
705                 710                 715                 720

Arg Gly Leu Gln Gly Glu Lys Gly Glu Lys Gly Glu Asp Gly Phe Pro
                725                 730                 735

Gly Phe Lys Gly Asp Val Gly Leu Lys Gly Asp Gln Gly Lys Pro Gly
            740                 745                 750

Ala Pro Gly Pro Arg Gly Glu Asp Gly Pro Glu Gly Pro Lys Gly Gln
    755                 760                 765

Ala Gly Gln Ala Gly Glu Glu Gly Pro Pro Gly Ser Ala Gly Glu Lys
    770                 775                 780

Gly Lys Leu Gly Val Pro Gly Leu Pro Gly Tyr Pro Gly Arg Pro Gly
785                 790                 795                 800

Pro Lys Gly Ser Ile Gly Phe Pro Gly Pro Leu Gly Pro Ile Gly Glu
                805                 810                 815

Lys Gly Lys Ser Gly Lys Thr Gly Gln Pro Gly Leu Glu Gly Glu Arg
            820                 825                 830

Gly Pro Pro Gly Ser Arg Gly Glu Arg Gly Gln Pro Gly Ala Thr Gly
    835                 840                 845

Gln Pro Gly Pro Lys Gly Asp Val Gly Gln Asp Gly Ala Pro Gly Ile

56

850                    855                    860

Pro Gly Glu Lys Gly Leu Pro Gly Leu Gln Gly Pro Pro Gly Phe Pro
865                    870                    875                    880

Gly Pro Lys Gly Pro Pro Gly His Gln Gly Lys Asp Gly Arg Pro Gly
                 885                    890                    895

His Pro Gly Gln Arg Gly Glu Leu Gly Phe Gln Gly Gln Thr Gly Pro
                 900                    905                    910

Pro Gly Pro Ala Gly Val Leu Gly Pro Gln Gly Lys Thr Gly Glu Val
                 915                    920                    925

Gly Pro Leu Gly Glu Arg Gly Pro Pro Gly Pro Pro Gly Pro Pro Gly
                 930                    935                    940

Glu Gln Gly Leu Pro Gly Leu Glu Gly Arg Glu Gly Ala Lys Gly Glu
945                    950                    955                    960

Leu Gly Pro Pro Gly Pro Leu Gly Lys Glu Gly Pro Ala Gly Leu Arg
                 965                    970                    975

Gly Phe Pro Gly Pro Lys Gly Gly Pro Gly Asp Pro Gly Pro Thr Gly
                 980                    985                    990

Leu Lys Gly Asp Lys Gly Pro Pro Gly Pro Val Gly Ala Asn Gly Ser
                 995                    1000                   1005

Pro Gly Glu Arg Gly Pro Leu Gly Pro Ala Gly Gly Ile Gly Leu
     1010                   1015                   1020

Pro Gly Gln Ser Gly Ser Glu Gly Pro Val Gly Pro Ala Gly Lys
     1025                   1030                   1035

Lys Gly Ser Arg Gly Glu Arg Gly Pro Pro Gly Pro Thr Gly Lys
     1040                   1045                   1050

Asp Gly Ile Pro Gly Pro Leu Gly Pro Leu Gly Pro Pro Gly Ala
     1055                   1060                   1065

Ala Gly Pro Ser Gly Glu Glu Gly Asp Lys Gly Asp Val Gly Ala
     1070                   1075                   1080

Pro Gly His Lys Gly Ser Lys Gly Asp Lys Gly Asp Ala Gly Pro
     1085                   1090                   1095

```
Pro Gly Gln Pro Gly Ile Arg  Gly Pro Ala Gly His  Pro Gly Pro
    1100             1105              1110

Pro Gly Ala Asp Gly Ala Gln  Gly Arg Arg Gly Pro  Pro Gly Leu
    1115             1120              1125

Phe Gly Gln Lys Gly Asp Asp  Gly Val Arg Gly Phe  Val Gly Val
    1130             1135              1140

Ile Gly Pro Pro Gly Leu Gln  Gly Leu Pro Gly Pro  Pro Gly Glu
    1145             1150              1155

Lys Gly Glu Val Gly Asp Val  Gly Ser Met Gly Pro  His Gly Ala
    1160             1165              1170

Pro Gly Pro Arg Gly Pro Gln  Gly Pro Thr Gly Ser  Glu Gly Thr
    1175             1180              1185

Pro Gly Leu Pro Gly Gly Val  Gly Gln Pro Gly Ala  Val Gly Glu
    1190             1195              1200

Lys Gly Glu Arg Gly Asp Ala  Gly Asp Pro Gly Pro  Pro Gly Ala
    1205             1210              1215

Pro Gly Ile Pro Gly Pro Lys  Gly Asp Ile Gly Glu  Lys Gly Asp
    1220             1225              1230

Ser Gly Pro Ser Gly Ala Ala  Gly Pro Pro Gly Lys  Lys Gly Pro
    1235             1240              1245

Pro Gly Glu Asp Gly Ala Lys  Gly Ser Val Gly Pro  Thr Gly Leu
    1250             1255              1260

Pro Gly Asp Leu Gly Pro Pro  Gly Asp Pro Gly Val  Ser Gly Ile
    1265             1270              1275

Asp Gly Ser Pro Gly Glu Lys  Gly Asp Pro Gly Asp  Val Gly Gly
    1280             1285              1290

Pro Gly Pro Pro Gly Ala Ser  Gly Glu Pro Gly Ala  Pro Gly Pro
    1295             1300              1305

Pro Gly Lys Arg Gly Pro Ser  Gly
    1310             1315


<210>   4
<211>   10
<212>   PRT
```

```
<213>   Artificial sequnce

<220>
<223>   an artificially synthesized sequence

<400>   4

His Met Gly Arg Glu Gly Arg Glu Gly Glu
1               5                   10


<210>   5
<211>   11
<212>   PRT
<213>   Artificial sequnce

<220>
<223>   an artificially synthesized sequence

<400>   5

Gly His Met Gly Arg Glu Gly Arg Glu Gly Glu
1               5                   10


<210>   6
<211>   10
<212>   PRT
<213>   Artificial sequnce

<220>
<223>   an artificially synthesized sequence

<400>   6

Gly Pro Pro Gly Lys Arg Gly Pro Ser Gly
1               5                   10


<210>   7
<211>   11
<212>   PRT
<213>   Artificial sequnce

<220>
<223>   an artificially synthesized sequence

<400>   7

Gly Pro Pro Gly Lys Arg Gly Pro Ser Gly His
1               5                   10
```

**Description**

[Technical Field]

The present disclosure relates to methods of diagnosing endometriosis or methods of determining whether or not a subject is affected by endometriosis, methods of determining the extent of endometrial fibrosis or adhesion in a subject affected by endometriosis, methods of predicting pain of a subject affected by endometriosis, methods of monitoring pathological conditions of a subject affected by endometriosis, and the like, and kits and the like for performing these methods.

[Background Art]

Endometriosis is an estrogen-dependent inflammatory disease observed in 6-10% of women capable of pregnancy, and infertility or pelvic pain is observed in more than 50% of patients (NPL 1). Various hypotheses have been proposed for the pathogenic mechanism of endometriosis (NPL 2) and the relationship with various factors including inflammatory cytokines and chemokines, growth factors, and hormones has been reported (NPL 3). Drugs that target hormones have already been developed, and hormone agents such as GnRH antagonists and progesterone formulations have been shown to alleviate pain and improve pathological conditions (NPLs 4 and 5). However, the strong side effects and difficulty in long-term use of hormone agent treatment present therapeutic problems (NPL 6). Meanwhile, drugs targeting inflammatory cytokines and chemokines are under development and anti-IL-8 antibodies have shown a strong effect of alleviating pathological conditions in a simian endometriosis model (PTL 1).

In the diagnosis of endometriosis, a definitive diagnosis can only be carried out by invasive means such as endoscopic observation or laparotomy. Furthermore, it is difficult for patients to notice the difference between symptoms associated with regular menstruation (e.g., pain) and symptoms of early endometriosis. As such, the hurdle to diagnosing a group of candidate patients is high and it is considered to require 7 to 11 years on average from onset to definitive diagnosis. The delay in definitive diagnosis leads to a delay in the period to start treatment of endometriosis, which is a great clinical problem (NPLs 7 and 8). Therefore, non-invasive diagnostic methods with less burden for patients, such as blood markers, are strongly desired, but precise diagnostic methods have not been established (NPL 9).

[Citation List]

[Patent Literature]

[PTL 1] WO2018/025982

[Non-Patent Literature]

[NPL 1] Giudice LC. Endometriosis. N Engl J Med 2010; 362:2389-98.
[NPL 2] Rogers PA et al. Research priorities for endometriosis. Reprod Sci 2017; 24:202-226.
[NPL 3] Beste MT et al. Molecular network analysis of endometriosis reveals a role for c-Jun-regulated macrophage activation. Sci Transl Med. 2014; 6:222ra216.
[NPL 4] Taylor HS et al. Treatment of Endometriosis-Associated Pain with Elagolix, an Oral GnRH Antagonist. N Engl J Med. 2017; 377:28-40.
[NPL 5] Kohler G et al. A dose-ranging study to determine the efficacy and safety of 1, 2, and 4mg of dienogest daily for endometriosis. Int J Gynaecol Obstet. 2010; 108:21-5.
[NPL 6] Treatment of Endometriosis-Associated Pain with Elagolix, an Oral GnRH Antagonist.
[NPL 7] Greene R et al. Diagnostic experience among 4,334 women reporting surgically diagnosed endometriosis. Fertility and Sterility, 2009; 91:32-39.
[NPL 8] Manderson L et al. Circuit breaking: Pathways of treatment seeking for women with endometriosis in Australia. Qualitative Health Research, 2008; 18:522-534.
[NPL 9] Fassbender A et al. Update on biomarkers for the detection of endometriosis. Biomed Res Int 2015; 2015:130854.

[Summary of Invention]

[Technical Problem]

The present disclosure was achieved in view of the above circumstances. An objective of the present disclosure is to provide methods of diagnosing endometriosis or methods of determining whether or not a subject is affected by endometriosis, methods of determining the extent of endometrial fibrosis or adhesion in a subject affected by endometriosis, methods of predicting pain of a subject affected by endometriosis, methods of monitoring pathological conditions of a subject affected by endometriosis, and the like, and kits for performing these methods.

[Solution to Problem]

As a result of conducting dedicated research on methods of diagnosing and methods of monitoring pathological conditions of endometriosis, the inventors of the present disclosure have discovered that there are a number of markers whose abundance in patients with endometriosis is different from those in healthy individuals, and also discovered that by measuring the abundance of those markers, whether or not a subject is affected by endometriosis can be diagnosed, the extent of endometrial fibrosis or adhesion can be determined, pain of a subject affected by endometriosis can be predicted, and pathological conditions of a subject affected by endometriosis can be monitored.

The present disclosure is based on these findings and specifically relates to the following inventions:

[1] A method of diagnosing endometriosis, comprising measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in a sample obtained from a subject.

[2] A method of determining whether or not a subject is affected by endometriosis, comprising measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in a sample obtained from the subject.

[3] The method of [1] or [2], further comprising a step in which if the abundance of at least one marker selected from the group consisting of the type V collagen MMP degradation product, the markers shown in Table 1A, the markers shown in Table 2A, the markers shown in Table 5A, and the markers shown in Table 6A is high or the abundance of at least one marker selected from the group consisting of the markers shown in Table 1B, the markers shown in Table 2B, the markers shown in Table 5B, and the markers shown in Table 6B is low in the sample obtained from the subject, the subject that the sample is derived from is shown to be affected or potentially affected by endometriosis.

[4] A method of determining the extent of endometrial fibrosis in a subject, comprising measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in a sample obtained from the subject.

[5] The method of [4], further comprising a step in which if the abundance of at least one marker selected from the group consisting of the type V collagen MMP degradation product, the markers shown in Table 1A, the markers shown in Table 2A, the markers shown in Table 5A, and the markers shown in Table 6A is high or the abundance of at least one marker selected from the group consisting of the markers shown in Table 1B, the markers shown in Table 2B, the markers shown in Table 5B, and the markers shown in Table 6B is low in the sample obtained from the subject, it is shown that endometrial fibrosis has occurred or potentially occurred in the subject that the sample is derived from.

[6] A method of determining the extent of endometrial adhesion in a subject, comprising measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in a sample obtained from the subject.

[7] The method of [6], further comprising a step in which if the abundance of at least one marker selected from the group consisting of the type V collagen MMP degradation product, the markers shown in Table 1A, the markers shown in Table 2A, the markers shown in Table 5A, and the markers shown in Table 6A is high or the abundance of at least one marker selected from the group consisting of the markers shown in Table 1B, the markers shown in Table 2B, the markers shown in Table 5B, and the markers shown in Table 6B is low in the sample obtained from the subject, it is shown that endometrial adhesion has occurred or potentially occurred in the subject that the sample is derived from.

[8] A method of predicting the degree of pain due to endometriosis in a subject affected or suspected of being affected by endometriosis, comprising measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in a sample obtained from the subject.

[9] The method of [8], further comprising a step in which if the abundance of at least one marker selected from the group consisting of the type V collagen MMP degradation product, the markers shown in Table 1A, the markers shown in Table 2A, the markers shown in Table 5A, and the markers shown in Table 6A is high or the abundance of at least one marker selected from the group consisting of the markers shown in Table 1B, the markers shown in Table 2B, the markers shown in Table 5B, and the markers shown in Table 6B is low in the sample obtained from the subject, the subject that the sample is derived from is shown to develop or potentially develop pain due to endometriosis.

[10] A method of determining the degree of progression of endometriosis in a subject affected or suspected of being affected by endometriosis, comprising measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in a sample obtained from the subject.

[11] The method of [10], further comprising a step in which if the abundance of at least one marker selected from the group consisting of the type V collagen MMP degradation product, the markers shown in Table 1A, the markers shown in Table 2A, the markers shown in Table 5A, and the markers shown in Table 6A is high or the abundance of at least one marker selected from the group consisting of the markers shown in Table 1B, the markers shown in Table 2B, the markers shown in Table 5B, and the markers shown in Table 6B is low in the sample obtained from the subject, it is shown that endometriosis is progressing or potentially progressing in the subject that the sample is derived from.

[12] The method of [3], [5], [7], [9], or [11], wherein when the abundance of at least one marker selected from the group consisting of the type V collagen MMP degradation product, the markers shown in Table 1A, the markers shown in Table 2A, the markers shown in Table 5A, and the markers shown in Table 6A is higher than the abundance of the same marker in a sample obtained from a healthy individual, the abundance is determined to be high.

[13] The method of [3], [5], [7], [9], or [11], wherein when the abundance of at least one marker selected from the group consisting of the type V collagen MMP degradation product, the markers shown in Table 1A, the markers shown in Table 2A, and the markers shown in Table 5A is two-fold or higher than the abundance of the same marker in a sample obtained from a healthy individual and/or when the abundance of at least one marker selected from the markers shown in Table 6A is 1.6-fold or higher than the abundance of the same marker in a sample obtained from a healthy individual, the abundance is determined to be high.

[14] The method of [3], [5], [7], [9], or [11], wherein when the abundance of at least one marker selected from the group consisting of the markers shown in Table 1B, the markers shown in Table 2B, the markers shown in Table 5B, and the markers shown in Table 6B is lower than the abundance of the same marker in a sample obtained from a healthy individual, the abundance is determined to be low.

[15] The method of [3], [5], [7], [9], or [11], wherein when the abundance of at least one marker selected from the group consisting of the markers shown in Table 1B, the markers shown in Table 2B, and the markers shown in Table 5B is 0.5-fold or lower than the abundance of the same marker in a sample obtained from a healthy individual and/or when the abundance of at least one marker selected from the markers shown in Table 6B is 0.625-fold or lower than the abundance of the same marker in a sample obtained from a healthy individual, the abundance is determined to be low.

[16] A method of monitoring pathological conditions of endometriosis in a subject affected or suspected of being affected by endometriosis, comprising measuring each of the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in each of a plurality of samples collected from the subject at different points of time.

[17] The method of [16], further comprising a step in which if the abundance of at least one marker selected from the group consisting of the type V collagen MMP degradation product, the markers shown in Table 1A, the markers shown in Table 2A, the markers shown in Table 5A, and the markers shown in Table 6A decreases over time or the abundance of at least one marker selected from the group consisting of the markers shown in Table 1B, the markers shown in Table 2B, the markers shown in Table 5B, and the markers shown in Table 6B increases over time, it is shown that the pathological conditions of endometriosis are improving or potentially improving in the subject that the sample is derived from.

[18] The method of [16], further comprising a step in which if the abundance of at least one marker selected from the group consisting of the type V collagen MMP degradation product, the markers shown in Table 1A, the markers shown in Table 2A, the markers shown in Table 5A, and the markers shown in Table 6A increases over time or the abundance of at least one marker selected from the group consisting of the markers shown in Table 1B, the markers shown in Table 2B, the markers shown in Table 5B, and the markers shown in Table 6B decreases over time, it is shown that the pathological conditions of endometriosis are worsening or potentially worsening in the subject that the sample is derived from.

[19] The method of any one of [1] to [18], wherein the marker is measured as a polypeptide.

[20] The method of any one of [1] to [19], wherein the sample is a blood sample.

[21] The method of [20], wherein the abundance of the marker in the sample is a concentration of a polypeptide of the marker in the blood sample.

[21-1] The method of [20] or [21], wherein the concentration of the type V collagen MMP degradation product in the blood sample is measured by specifically recognizing the peptide represented by SEQ ID NO: 4 or SEQ ID NO: 6.

[22] A kit for diagnosing endometriosis, comprising a reagent for measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B.

[23] A kit for determining whether or not a subject is affected by endometriosis, comprising a reagent for measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B.

[24] The kit of [22] or [23], further comprising instructions stating that if the abundance of at least one marker selected from the group consisting of the type V collagen MMP degradation product, the markers shown in Table 1A, the markers shown in Table 2A, the markers shown in Table 5A, and the markers shown in Table 6A is high or the abundance of at least one marker selected from the group consisting of the markers shown in Table 1B, the markers shown in Table 2B, the markers shown in Table 5B, and the markers shown in Table 6B is low in a sample obtained from a subject, the subject that the sample is derived from is shown to be affected or potentially affected by endometriosis.

[25] A kit for determining the extent of endometrial fibrosis in a subject, comprising a reagent for measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B.

[26] The kit of [25], further comprising instructions stating that if the abundance of at least one marker selected from the group consisting of the type V collagen MMP degradation product, the markers shown in Table 1A, the markers shown in Table 2A, the markers shown in Table 5A, and the markers shown in Table 6A is high or the abundance of at least one marker selected from the group consisting of the markers shown in Table 1B, the markers shown in Table 2B, the markers shown in Table 5B, and the markers shown in Table 6B is low in a sample obtained from a subject, it is shown that endometrial fibrosis has occurred or potentially occurred in the subject that the sample is derived from.

[27] A kit for determining the extent of endometrial adhesion in a subject, comprising a reagent for measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B.

[28] The kit of [27], further comprising instructions stating that if the abundance of at least one marker selected from the group consisting of the type V collagen MMP degradation product, the markers shown in Table 1A, the markers shown in Table 2A, the markers shown in Table 5A, and the markers shown in Table 6A is high or the abundance of at least one marker selected from the group consisting of the markers shown in Table 1B, the markers shown in Table 2B, the markers shown in Table 5B, and the markers shown in Table 6B is low in a sample obtained from a subject, it is shown that endometrial adhesion has occurred or potentially occurred in the subject that the sample is derived from.

[29] A kit for predicting the degree of pain due to endometriosis in a subject affected or suspected of being affected by endometriosis, comprising a reagent for measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B.

[30] The kit of [29], further comprising instructions stating that if the abundance of at least one marker selected from the group consisting of the type V collagen MMP degradation product, the markers shown in Table 1A, the markers shown in Table 2A, the markers shown in Table 5A, and the markers shown in Table 6A is high or the abundance of at least one marker selected from the group consisting of the markers shown in Table 1B, the markers shown in Table 2B, the markers shown in Table 5B, and the markers shown in Table 6B is low in a sample obtained from a subject, the subject that the sample is derived from is shown to develop or potentially develop pain due to endometriosis.

[31] A kit for determining the degree of progression of endometriosis in a subject affected or suspected of being affected by endometriosis, comprising a reagent for measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B.

[32] The kit of [31], further comprising instructions stating that if the abundance of at least one marker selected from the group consisting of the type V collagen MMP degradation product, the markers shown in Table 1A, the markers shown in Table 2A, the markers shown in Table 5A, and the markers shown in Table 6A is high or the abundance of at least one marker selected from the group consisting of the markers shown in Table 1B, the markers shown in Table 2B, the markers shown in Table 5B, and the markers shown in Table 6B is low in a sample obtained from a subject, it is shown that endometriosis is progressing or potentially progressing in the subject that the sample is derived from.

[33] The kit of [24], [26], [28], [30], or [32], wherein if the abundance of at least one marker selected from the group consisting of the type V collagen MMP degradation product, the markers shown in Table 1A, the markers shown in Table 2A, the markers shown in Table 5A, and the markers shown in Table 6A is higher than the abundance of the same marker in a sample obtained from a healthy individual, the abundance is determined to be high.

[34] The kit of [24], [26], [28], [30], or [32], wherein if the abundance of at least one marker selected from the group consisting of the type V collagen MMP degradation product, the markers shown in Table 1A, the markers shown in Table 2A, and the markers shown in Table 5A is two-fold or higher than the abundance of the same marker in a sample obtained from a healthy individual and/or if the abundance of at least one marker selected from the markers shown in Table 6A is 1.6-fold or higher than the abundance of the same marker in a sample obtained from a healthy individual, the abundance is determined to be high.

[35] The kit of [24], [26], [28], [30], or [32], wherein if the abundance of at least one marker selected from the group consisting of the markers shown in Table 1B, the markers shown in Table 2B, the markers shown in Table 5B, and the markers shown in Table 6B is lower than the abundance of the same marker in a sample obtained from a healthy individual, the abundance is determined to be low.

[36] The kit of [24], [26], [28], [30], or [32], wherein if the abundance of at least one marker selected from the group consisting of the markers shown in Table 1B, the markers shown in Table 2B, and the markers shown in Table 5B that is 0.5-fold or lower than the abundance of the same marker present in a sample obtained from a healthy individual and/or the abundance of at least one marker selected from the markers shown in Table 6B that is 0.625-fold or lower than the abundance of the same marker in a sample obtained from a healthy individual, the abundance is determined to be low.

[37] A kit for monitoring pathological conditions of endometriosis in a subject affected or suspected of being affected by endometriosis, comprising a reagent for measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B.

[38] The kit of [37], further comprising instructions stating that the abundance of at least one marker selected from the group consisting of the type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in each of a plurality of samples collected from the subject at different points of time is compared.

[39] The kit of [37] or [38], further comprising instructions stating that if the abundance of at least one marker selected from the group consisting of the type V collagen MMP degradation product, the markers shown in Table 1A, the markers shown in Table 2A, the markers shown in Table 5A, and the markers shown in Table 6A decreases over time or the abundance of at least one marker selected from the group consisting of the markers shown in Table 1B, the markers shown in Table 2B, the markers shown in Table 5B, and the markers shown in Table 6B increases over time, it is shown that the pathological conditions of endometriosis are improving or potentially improving in the subject that the sample is derived from.

[40] The kit of [37] or [38], further comprising instructions stating that if the abundance of at least one marker selected from the group consisting of the type V collagen MMP degradation product, the markers shown in Table 1A, the markers shown in Table 2A, the markers shown in Table 5A, and the markers shown in Table 6A increases over time or the abundance of at least one marker selected from the group consisting of the markers shown in Table 1B, the markers shown in Table 2B, the markers shown in Table 5B, and the markers shown in Table 6B decreases over time, it is shown that the pathological conditions of endometriosis are worsening or potentially worsening in the subject that the sample is derived from.

[41] The kit of any one of [22] to [40], wherein the marker is measured as a polypeptide.

[42] The kit of any one of [22] to [41], wherein the sample is a blood sample.

[43] The kit of [42], wherein the abundance of the marker in the sample is a concentration of a polypeptide of the marker in the blood sample.

[43-1] The kit of [42] or [43], wherein the concentration of the type V collagen MMP degradation product in the blood sample is measured by specifically recognizing the peptide represented by SEQ ID NO: 4 or SEQ ID NO: 6.

[44] A reagent for measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B, for use in the diagnosis of endometriosis.

[45] A reagent for measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B, for use in the determination of whether or not a subject is affected by endometriosis.

[46] A reagent for measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B, for use in the determination of the extent of endometrial fibrosis in a subject.

[47] A reagent for measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B, for use in the determination of the extent of endometrial adhesion in a subject.

[48] A reagent for measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B, for use in the prediction of the degree of pain due to endometriosis in a subject affected or suspected of being affected by endometriosis.

[49] A reagent for measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B, for use in the determination of the degree of progression of endometriosis in a subject affected or suspected of being affected by endometriosis.

[50] A reagent for measuring the abundance of at least one marker selected from the group consisting of a type V collagen MMP degradation product, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B, for use in the monitoring of pathological conditions of endometriosis in a subject affected or suspected of being affected by endometriosis.

[51] The reagent of any one of [44] to [50], wherein the marker is measured as a polypeptide.

[52] The reagent of any one of [44] to [51], wherein the abundance of the marker is measured by measuring a concentration of a polypeptide of the marker in a blood sample obtained from the subject.

[53] The reagent of [51], wherein the concentration of the type V collagen MMP degradation product in the blood sample is measured by specifically recognizing the peptide represented by SEQ ID NO: 4 or SEQ ID NO: 6 present in the blood sample.

[Brief Description of Drawings]

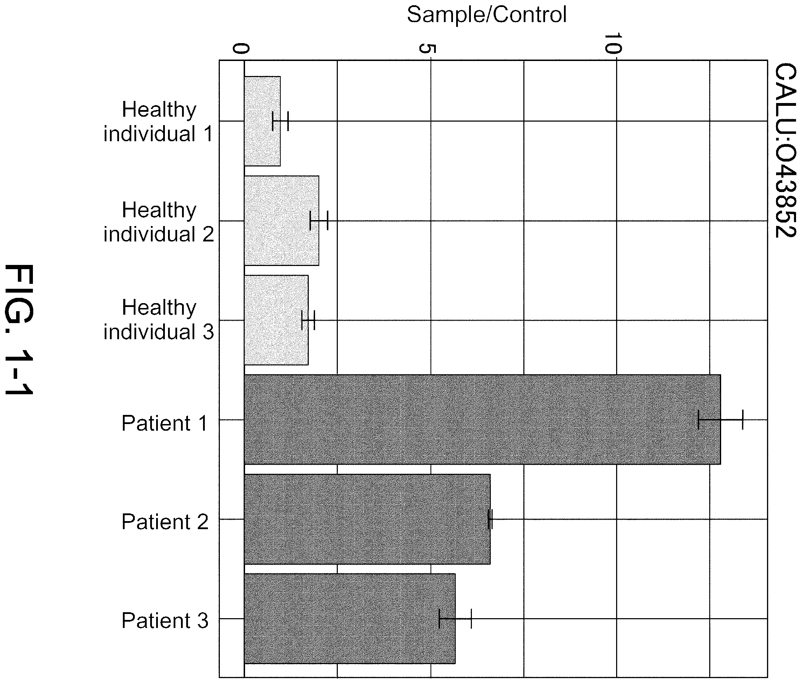

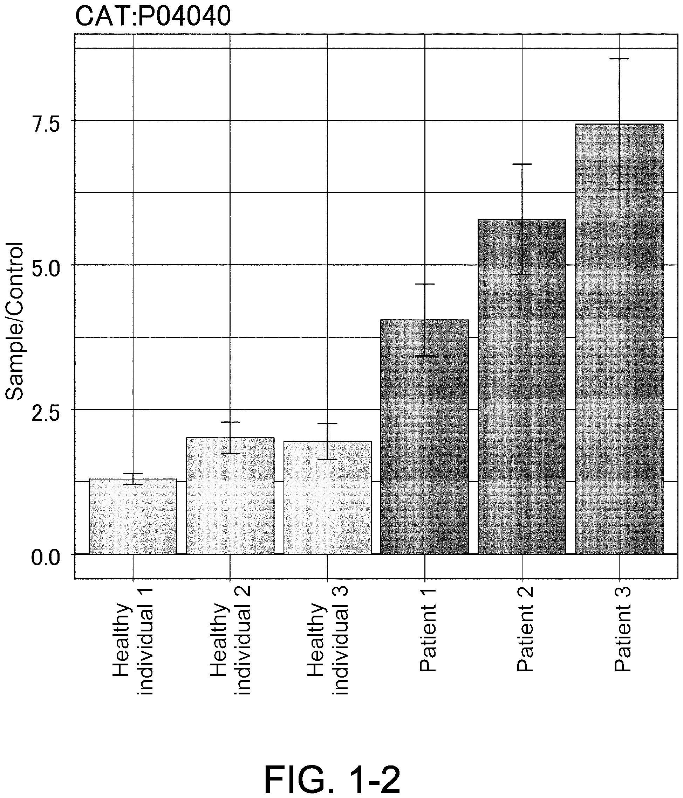

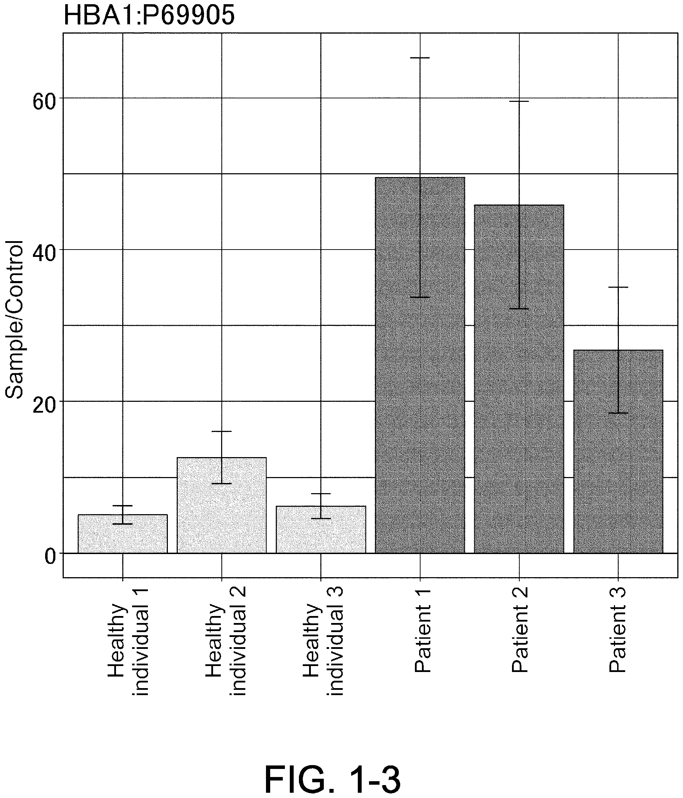

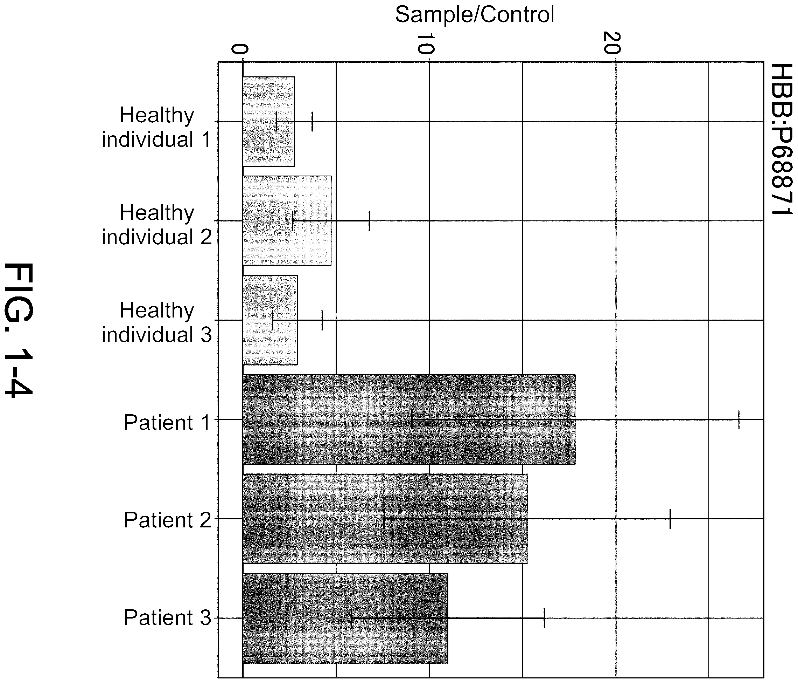

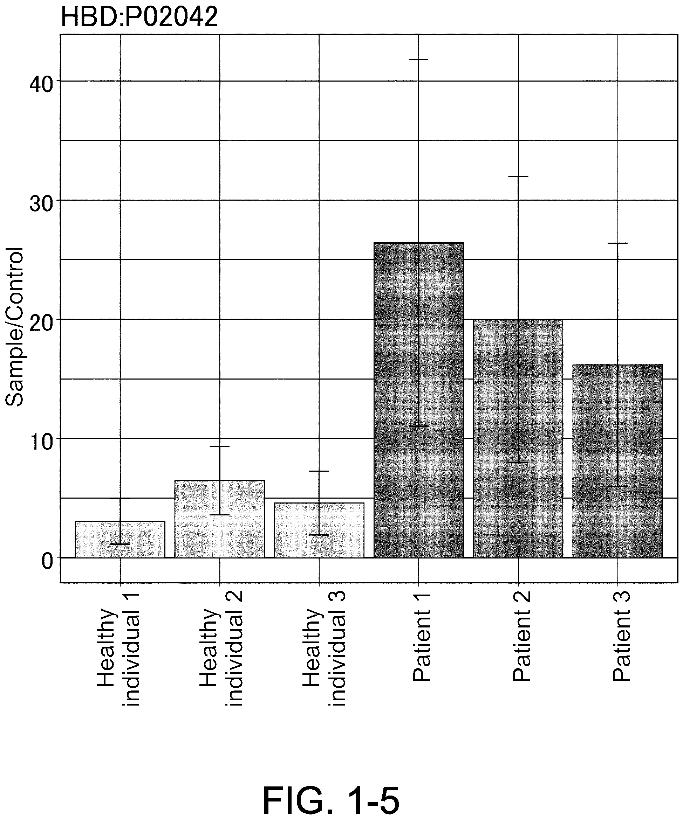

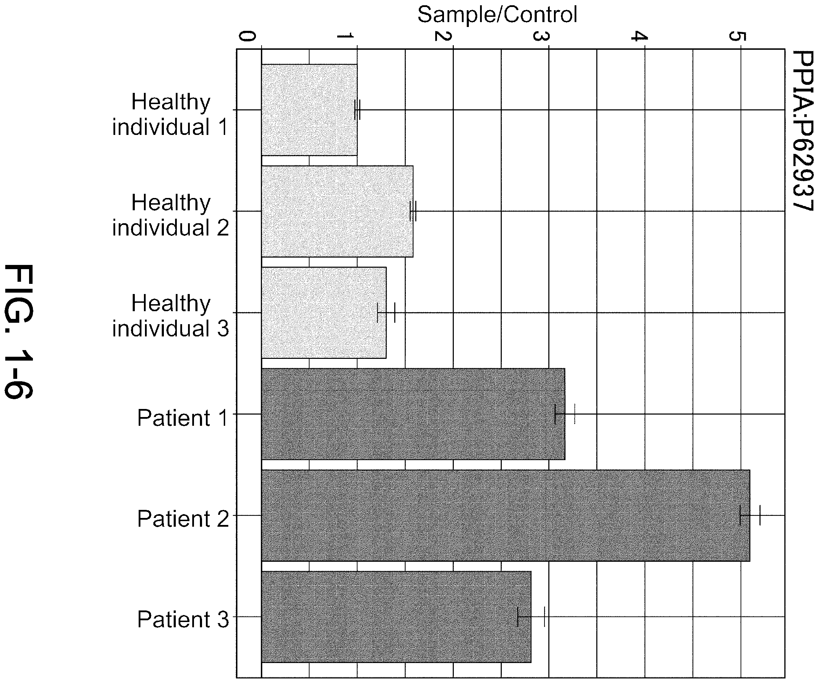

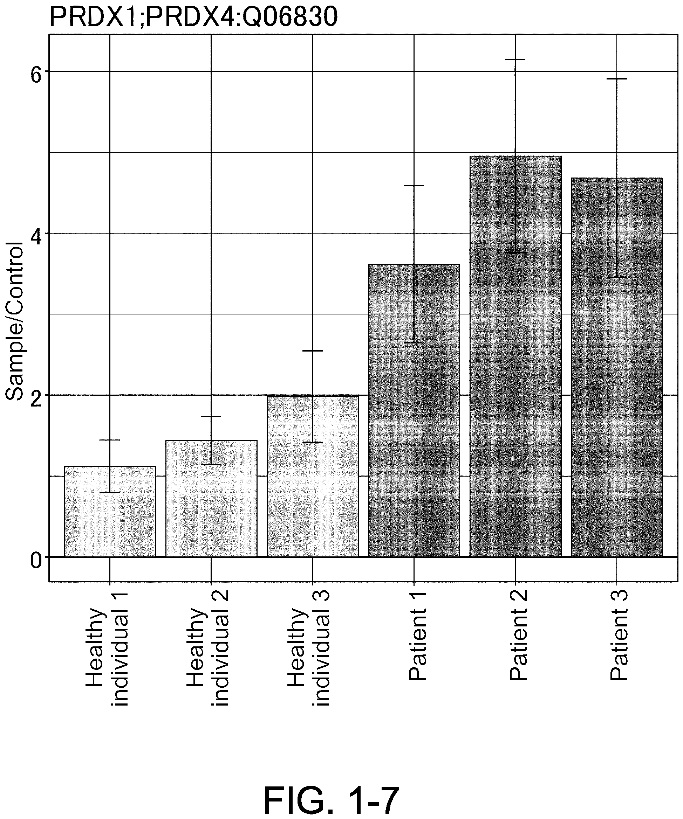

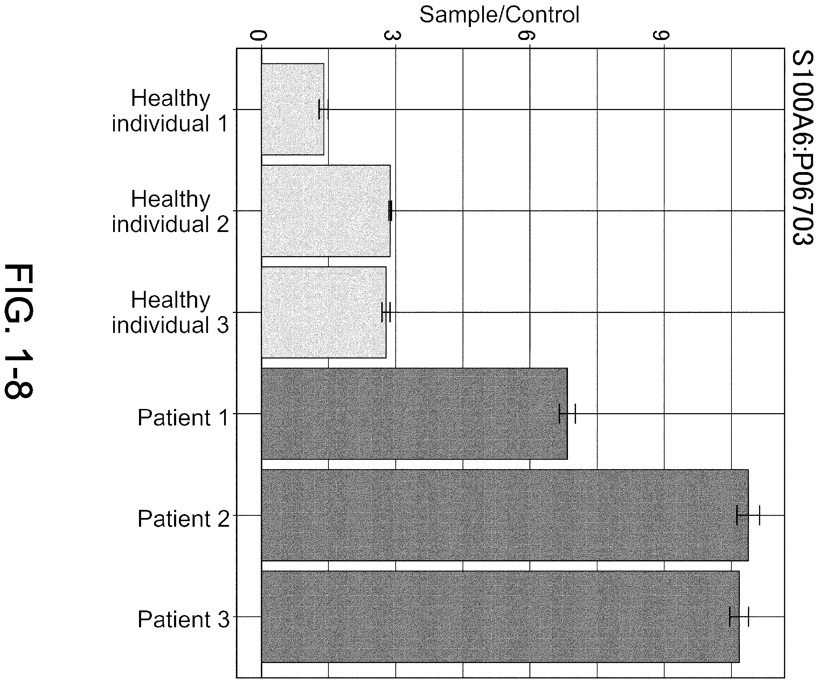

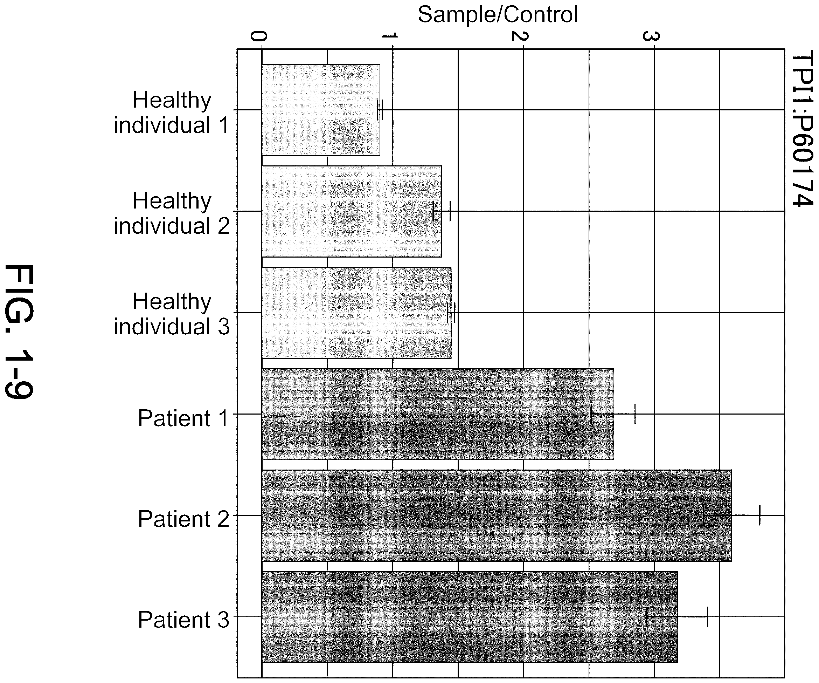

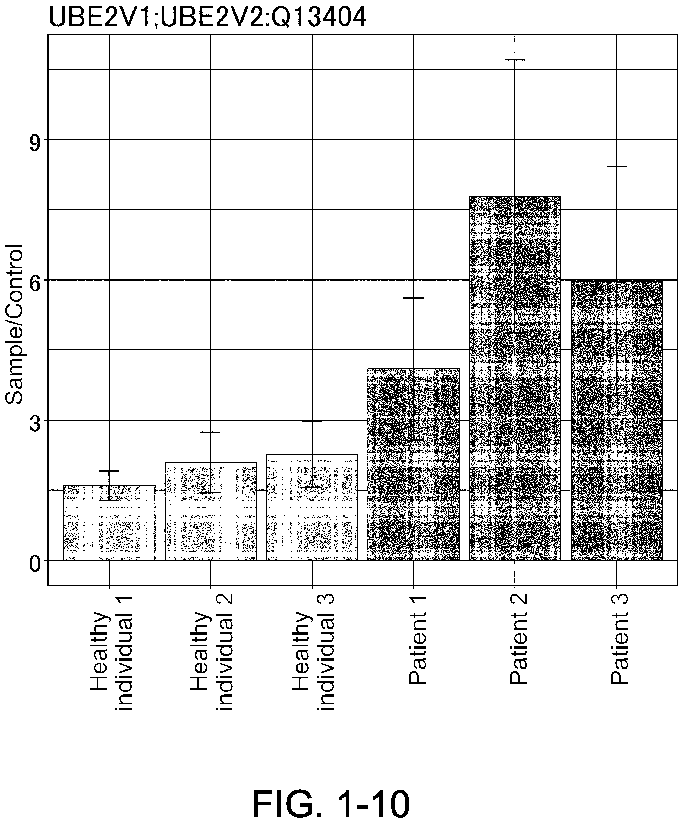

Fig. 1-1 to Fig. 1-10 show the results of analyzing the relative expression levels of proteins in the plasma of healthy individuals/endometriosis patients by LC-MS. The title of each graph shows Gene Symbol:UniProt ID of the target protein. The X axis represents the name of the sample and the Y axis represents the relative expression level of the protein. The bars in the graph represent the standard deviation of the relative expression level of the protein in three data sets obtained by three analyses. Fig. 1-1 shows the data on Gene Symbol: CALU and UniProt ID: 043852.
Fig. 1-2 shows the data on Gene Symbol: CAT and UniProt ID: P04040.
Fig. 1-3 shows the data on Gene Symbol: HBA1 and UniProt ID: P69905.
Fig. 1-4 shows the data on Gene Symbol: HBB and UniProt ID: P68871.
Fig. 1-5 shows the data on Gene Symbol: HBD and UniProt ID: P02042.
Fig. 1-6 shows the data on Gene Symbol: PPIA and UniProt ID: P62937.
Fig. 1-7 shows the data on Gene Symbol: PRDX1;PRDX4 and UniProt ID: Q06830.
Fig. 1-8 shows the data on Gene Symbol: S100A6 and UniProt ID: P06703.
Fig. 1-9 shows the data on Gene Symbol: TPI1 and UniProt ID: P60174.
Fig. 1-10 shows the data on Gene Symbol: UBE2V1;UBE2V2 and UniProt ID: Q13404.
Fig. 2-1 to Fig. 2-10 show the results of analyzing the expression levels of proteins in the plasma of healthy individuals/endometriosis patients by SOMAscan®. The title of each graph shows Gene Symbol:UniProt ID of the target protein. The X axis represents the name of the sample and the Y axis represents the aptamer signal value of the target protein in SOMAscan®. Fig. 2-1 shows the data on Gene Symbol: BGN and UniProt ID: P21810.
Fig. 2-2 shows the data on Gene Symbol: CDH12 and UniProt ID: P55289.

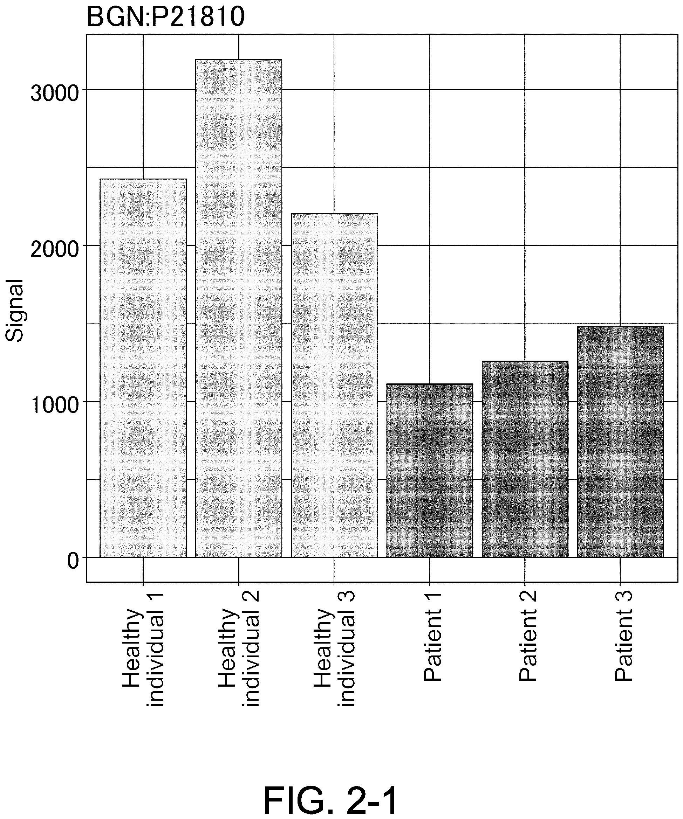

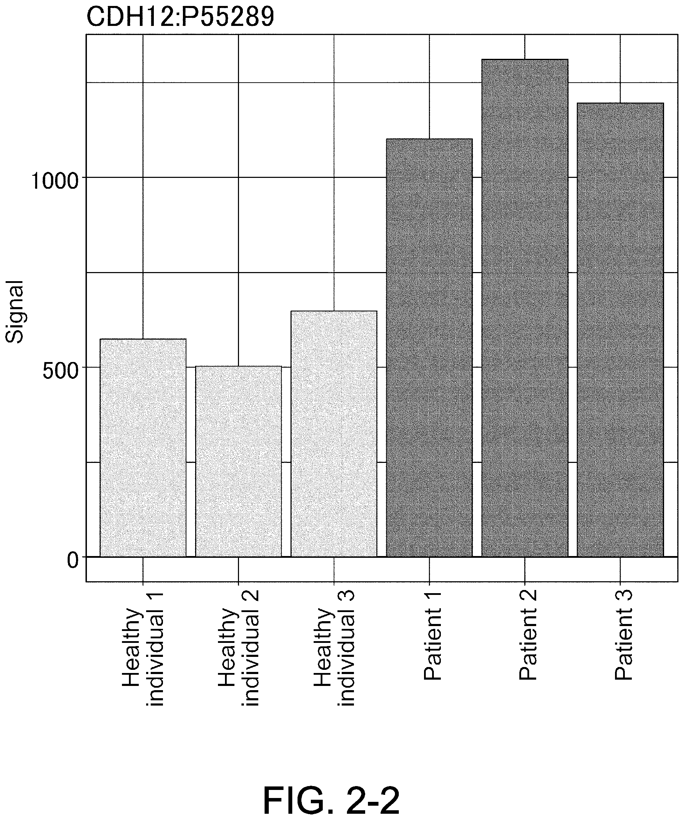

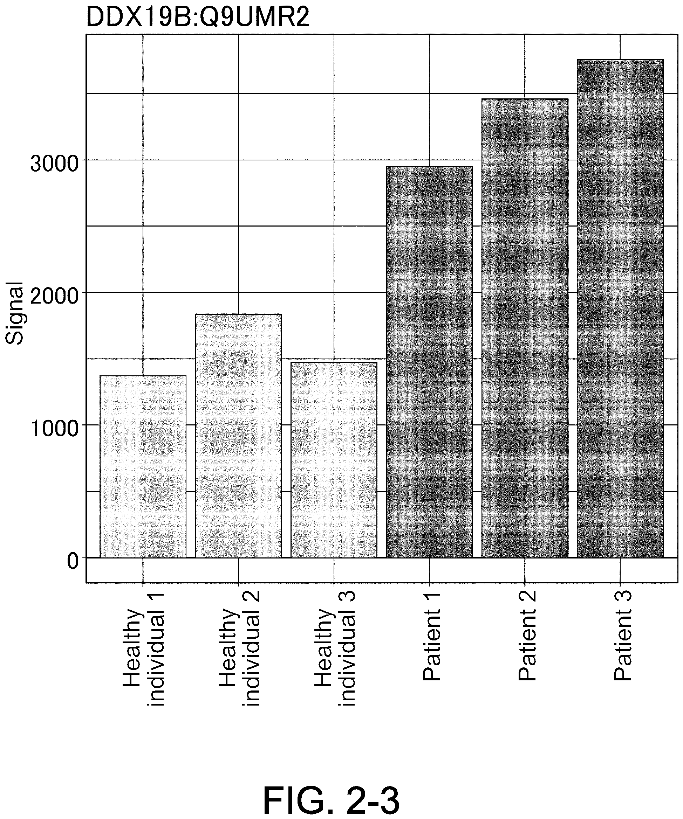

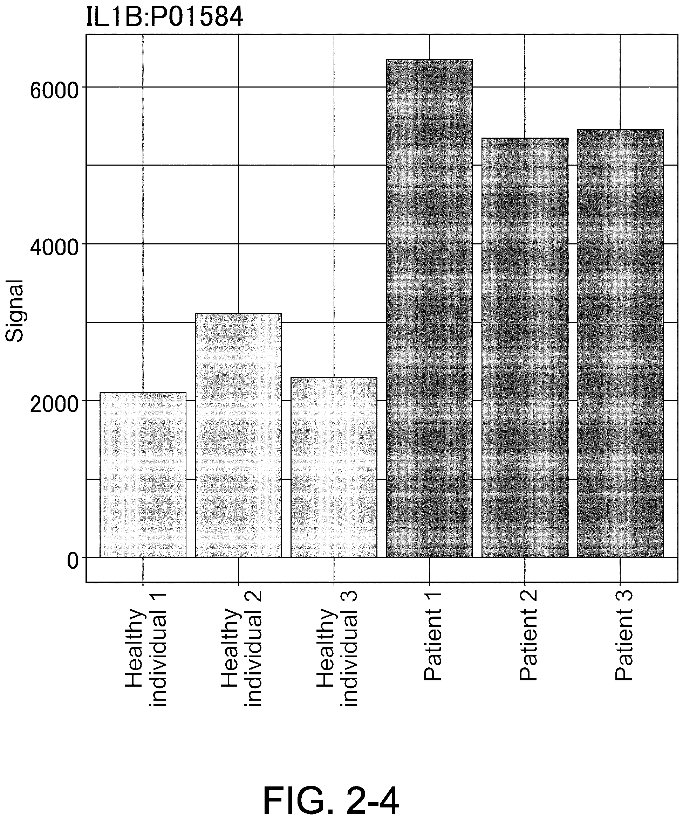

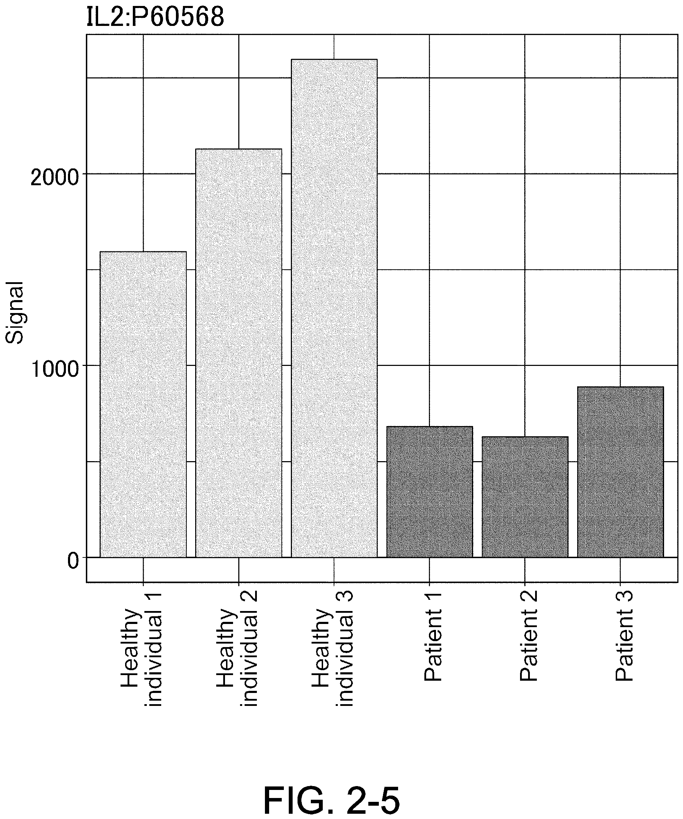

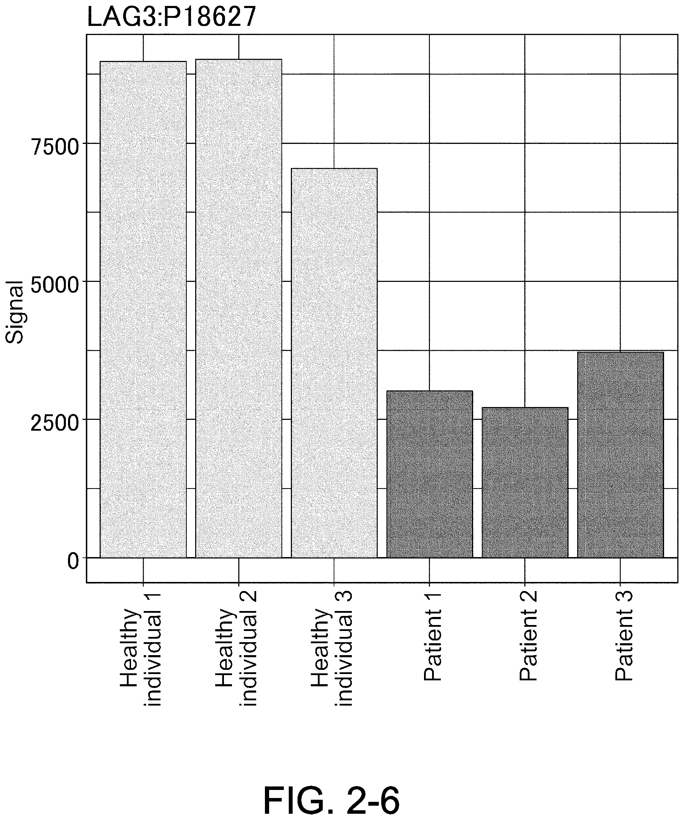

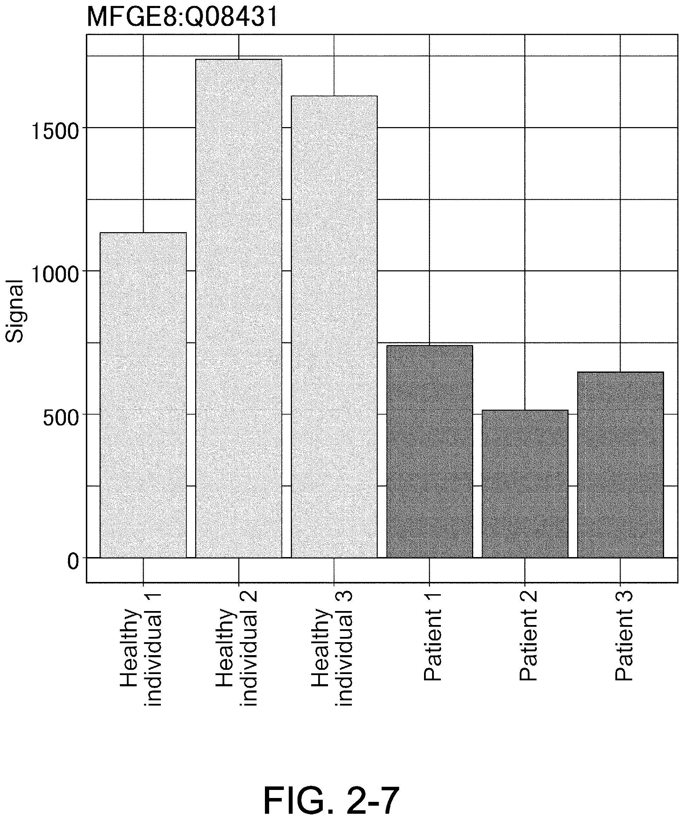

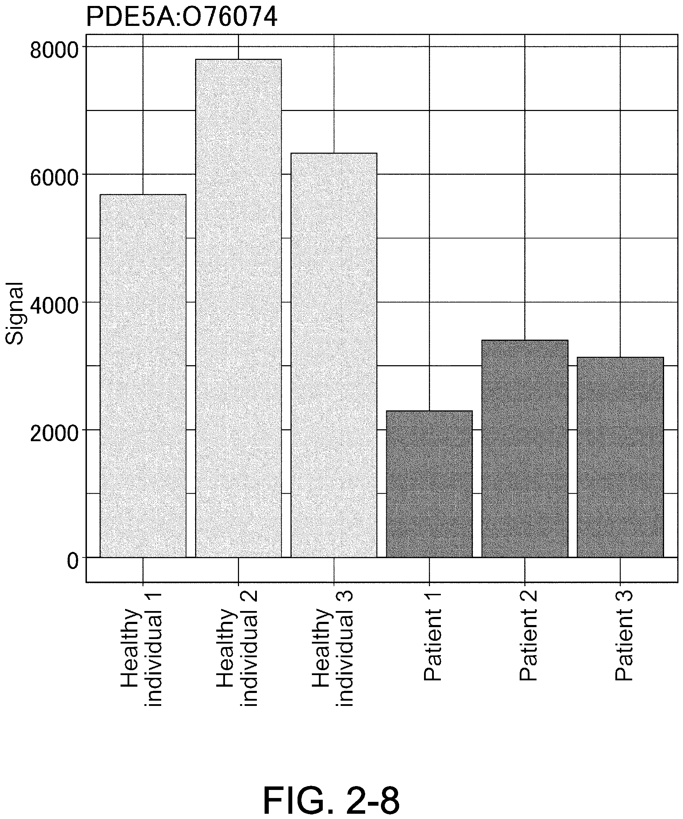

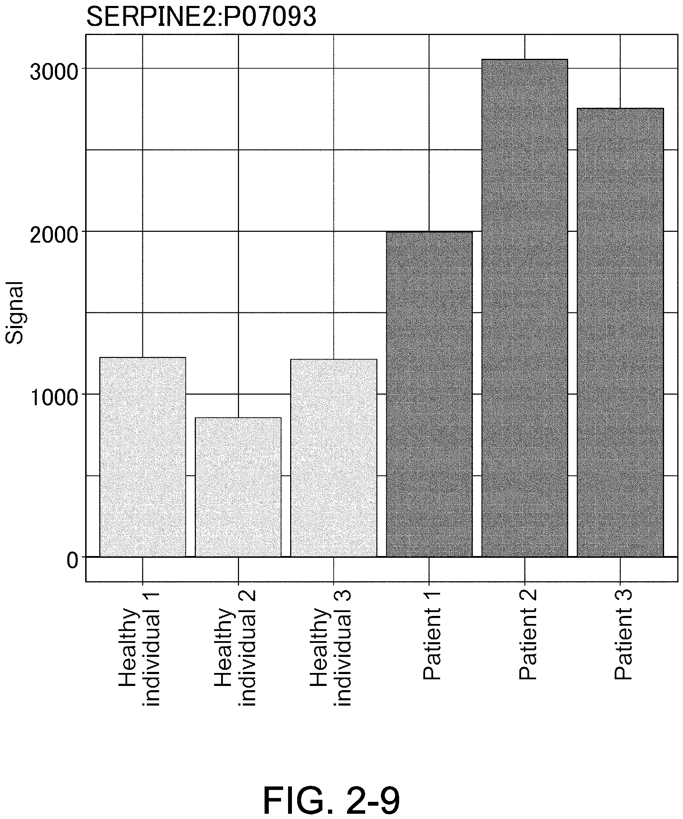

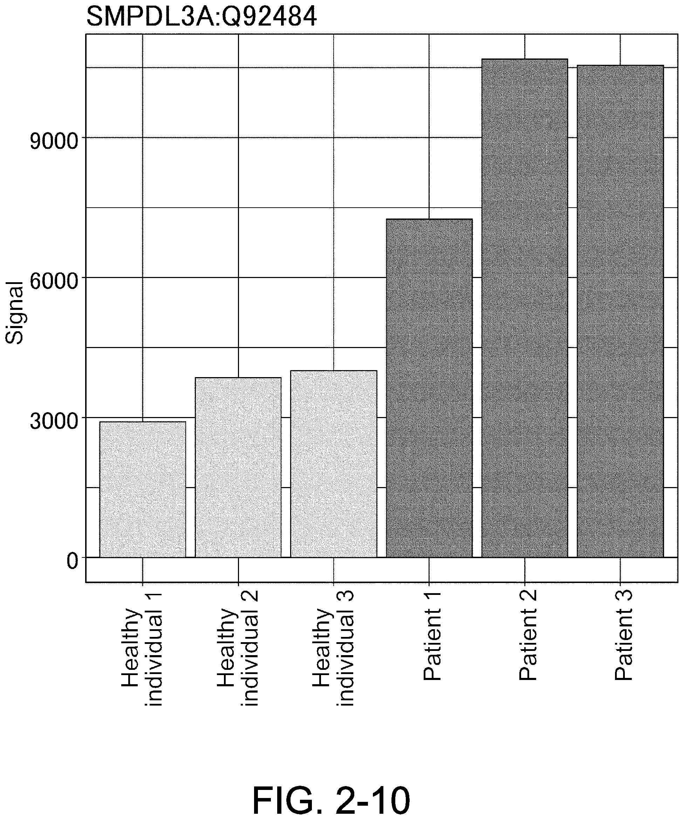

Fig. 2-3 shows the data on Gene Symbol: DDX19B and UniProt ID: Q9UMR2
Fig. 2-4 shows the data on Gene Symbol: IL1B and UniProt ID: P01584.
Fig. 2-5 shows the data on Gene Symbol: IL2 and UniProt ID: P60568.
Fig. 2-6 shows the data on Gene Symbol: LAG3 and UniProt ID: P18627.
Fig. 2-7 shows the data on Gene Symbol: MFGE8 and UniProt ID: Q08431.
Fig. 2-8 shows the data on Gene Symbol: PDE5A and UniProt ID: O76074.
Fig. 2-9 shows the data on Gene Symbol: SERPINE2 and UniProt ID: P07093.
Fig. 2-10 shows the data on Gene Symbol: SMPDL3A and UniProt ID: Q92484.

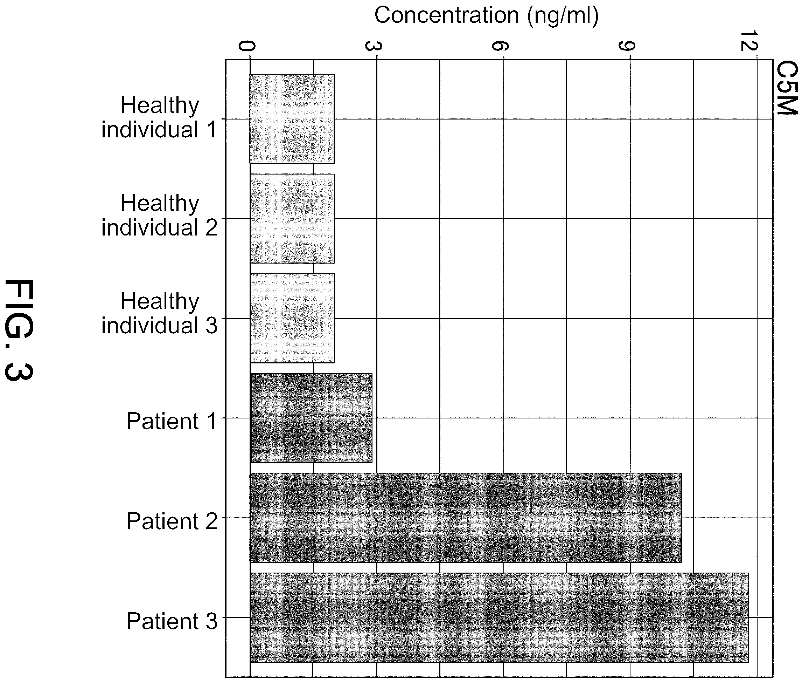

Fig. 3 shows the result of analyzing the expression level of a type V collagen degradation product (C5M) in the plasma of healthy individuals/endometriosis patients. The X axis represents the name of the sample and the Y axis represents the concentration of the target degradation product in the plasma (ng/ml).

[Description of Embodiments]

Markers

Markers in the present disclosure can be reworded as biomarkers and refer to specific chemical substances in the body that can be measured and evaluated objectively as indices for normal biological processes, disease development processes, or pharmacological responsiveness to treatment. The markers are useful for evaluating the presence or absence of diseases, progress of diseases, or susceptibility to diseases; evaluating or predicting the effects, the optimal dose, or safety of pharmaceutical agents; predicting prognosis; or such. The markers in the present disclosure are specified by protein names, protein fragment names, or gene names. Genes serving as markers are preferably measured as polypeptides or polynucleotides (including the form of DNA and the form of mRNA), and proteins or protein fragments serving as markers are preferably measured as polypeptides.

Methods of measuring the abundance of markers

The abundance of a marker can be measured by selecting an appropriate method according to the form of the marker or the type of a sample in which the abundance of the marker is to be measured. When the marker is in the form of a polypeptide, it can be measured by immunological methods that use antibodies specifically binding to the polypeptide. Examples of such methods include enzyme immunoassays (ELISA, EIA), fluoroimmunoassays (FIA), radioimmunoassays (RIA), luminescent immunoassays (LIA), electrochemical luminescence (ECL) methods, Western blotting methods, surface plasmon resonance methods, methods that use antibody arrays, immunohistochemical staining methods, fluorescence activated cell sorting (FACS) methods, immunochromatography methods, immunoprecipitation methods, immunonephelometry methods, and latex agglutination methods. When the marker is in the form of a polynucleotide, it can be measured by genetic engineering methods that use oligonucleotides specifically binding to the polynucleotide. Examples of such methods include polymerase chain reaction (PCR) methods, reverse transcription PCR (RT-PCR) methods, real-time quantitative PCR (Q-PCR) methods, northern blotting, and hybridization methods (including methods that use oligonucleotide arrays such as DNA microarrays).

When a marker to be measured is a protein expressed from a gene, the abundance can be reworded as expression level. In the present disclosure, the "abundance" includes the expression level of a protein, the expression level of a gene, and the concentration of a protein fragment.

In an embodiment of the present disclosure, the abundance of a marker can be a relative abundance. A relative abundance can be measured by comparing the level of a specific marker and the level of other proteins/metabolites in a sample obtained from a subject with a control. A relative abundance can also be measured by LC/MS (liquid chromatography/mass spectrometry).

Criteria for the abundance of markers

In the present disclosure, "the abundance of a marker is high or large" means that the measured value of the marker is higher or larger than a predetermined value (control level) for the marker. "The abundance of a marker is low or small" means that it is lower or smaller than a predetermined value (control level) for the marker or not greater than the control level.

The predetermined value in the present disclosure means a value that is predetermined based on a certain scientific basis. It may be any value as long as it can be used as a reference to determine the presence or absence of endometriosis, determine the extent of endometrial fibrosis in a subject, determine the extent of endometrial adhesion in a subject, predict the degree of pain of a subject affected by endometriosis, determine the degree of progression of endometriosis, or monitor pathological conditions of endometriosis. The predetermined value in the present disclosure may be determined for each marker.

The predetermined value in the present disclosure can be determined from the measured value of a marker in a sample (control sample) obtained from a healthy subject, for example, a healthy adult. It has been found in the present disclosure that the measured value of a marker in a sample obtained from a subject affected by endometriosis is increased or decreased compared to the measured value of the marker in a sample obtained from a healthy subject. Thus, one possible approach may be to use as a predetermined value the average of the measured values of a marker in samples obtained from multiple healthy subjects. Another possible approach may be to use as a predetermined value the average of the measured values of a marker in samples obtained from multiple healthy subjects plus a value of 0.1, 0.2, 0.3, 0.4, 0.5, 1.0, 1.5, 2.0, 2.5, or 3.0 times the standard deviation. Accordingly, in one embodiment of the present disclosure, it is shown that determination of whether a test subject is affected by endometriosis, determination of the extent of endometrial fibrosis in a subject, determination of the extent of endometrial adhesion in a subject, prediction of the degree of pain of a subject affected or suspected of being affected by endometriosis, determination of the degree of progression of endometriosis, or monitoring of pathological conditions of endometriosis, is carried out by comparing the abundance of a marker (control level) measured in a sample (control sample) obtained from a healthy individual and the measured value of the marker in a sample obtained from a test subject.

In the present disclosure, the measured values and predetermined values of markers in the present disclosure may be measurement results of the abundance of the markers quantified by any method. In the present disclosure, values obtained as a result of measurement (*e.g.*, color development intensity) may be directly used as measured values or predetermined values of markers, or values converted from measurement results (*e.g.*, concentration) by comparing them with a separately prepared positive control sample that contains a known amount of the marker may be used. Alternatively, values given by, for example, grouping the values obtained as described above into certain intervals and scoring them (*e.g.*, grades 1, 2, and 3) may be used.

Samples

Samples in the present disclosure can be reworded as biological samples and refer to organs, tissues, cells, body fluids, or mixtures thereof contained in living bodies. Specific examples include skin, respiratory tract, intestinal tract, urogenital tract, nerve, tumor, bone marrow, blood cells, blood (whole blood, plasma, serum), lymph, cerebrospinal fluid, intraperitoneal fluid, synovial fluid, intrapulmonary fluid, saliva, sputum, urine, and such. Samples obtained by washing these or obtained by culturing these *ex vivo* are also included in the samples of the present disclosure. A preferred sample in the present disclosure is blood, and a particularly preferred sample is plasma or serum.

In the present disclosure, samples obtained from subjects may be processed by methods such as concentration, purification, extraction, isolation, or physical/chemical treatment before subjected to measurement of the abundance of markers. For example, blood cells or plasma components may be isolated from blood samples, and DNA or RNA may be extracted from tissue/cell samples. Alternatively, unwanted components may be denatured/removed by heating or chemical reagents. Such processing is performed mainly for improving the sensitivity and specificity of measurement of the abundance of markers.

In determination of the extent of endometrial fibrosis, determination of the extent of endometrial adhesion, prediction of the degree of pain due to endometriosis, determination of the degree of progression of endometriosis, and monitoring of pathological conditions of endometriosis in the present disclosure, subjects from which samples are obtained may be subjects already diagnosed as being affected by endometriosis or subjects suspected of being affected by endometriosis. Subjects affected by endometriosis may be any subjects as long as they are affected by endometriosis. Subjects may be subjects that have not received, or have already been receiving, treatment for endometriosis.

Subjects in the present disclosure are mammals. Mammals include, but are not limited to, domesticated animals (for example, cows, sheep, cats, dogs, and horses), primates (for example, humans and non-human primates like monkeys), rabbits, and rodents (such as mice and rats). In a certain embodiment, subjects are humans.

Markers in the present disclosure

Markers in the present disclosure include type V collagen MMP (matrix metalloproteinase) degradation products (type V collagen-derived polypeptides comprising the amino acid sequence set forth in SEQ ID NO: 4 at its N-terminus and/or comprising the amino acid sequence set forth in SEQ ID NO: 6 at its C-terminus, such as protein fragments set forth in SEQ ID NOs: 2 and 3), the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B.

[Table 1A]

| UniProt ID | Protein name | Gene name |
|---|---|---|
| P02042 | Hemoglobin subunit delta | HBD |
| P68871 | Hemoglobin subunit beta;LVV-hemorphin-7;Spinorphin | HBB |
| P55072 | Transitional endoplasmic reticulum ATPase | VCP |
| P50395; P50395-2 | Rab GDP dissociation inhibitor beta | GDI2 |
| O43852-9;O43852-5;O43852-2; O43852;043852-4;043852-3; 043852-12;043852-13; 043852-14;043852-7;043852-15; 043852-11;O43852-8;O43852-10; 043852-6 | Calumenin | CALU |
| P04040 | Catalase | CAT |
| Q06830;Q13162 | Peroxiredoxin-1 ;Peroxiredoxin-4 | PRDX1; PRDX 4 |
| P69905 | Hemoglobin subunit alpha | HBA1 |
| Q13404-8;Q15819;Q13404; Q13404-7;Q13404-2;Q13404-1 | Ubiquitin-conjugating enzyme E2 variant 1;Ubiquitin-conjugating enzyme E2 variant 2 | UBE2V1; UBE2 V2 |
| P06703 | Protein S100-A6 | S100A6 |
| P60174-1;P60174;P60174-4 | Triosephosphate isomerase | TPI1 |
| P62937;P62937-2 | Peptidyl-prolyl cis-trans isomerase A;Peptidyl-prolyl cis-trans isomerase A, N-terminally processed | PPIA |
| P49746-2;P49746 | Thrombospondin-3 | THBS3 |
| P09486 | SPARC | SPARC |
| Q14766-3;Q14766-2;Q14766-5; Q14766;Q14766-4 | Latent-transforming growth factor beta-binding protein 1 | LTBP1 |
| P01137 | Transforming growth factor beta-1;Latency-associated peptide | TGFB1 |
| P61088;Q5JXB2 | Ubiquitin-conjugating enzyme E2 N;Putative ubiquitin-conjugating enzyme E2 N-like | UBE2N; UBE2 NL |
| P00558-2;P00558 | Phosphoglycerate kinase 1 | PGK1 |
| P07996;P07996-2 | Thrombospondin-1 | THBS1 |

(continued)

| UniProt ID | Protein name | Gene name |
|---|---|---|
| P02776 | Platelet factor 4;Platelet factor 4, short form | PF4 |
| P05067-7;P05067-11;P05067-8; P05067-9;P05067;P05067-10; P05067-3;P05067-4;P05067-5; P05067-6 | Amyloid beta A4 protein;N-APP;Soluble APP-alpha;Soluble APP-beta;C99;Beta-amyloid protein 42;Beta-amyloid protein 40;C83;P3(42);P3(40);C80;Gamm a-secretase C-terminal fragment 59;Gamma-secretase C-terminal fragment 57; Gamma-secretase C-terminal fragment 50;C31 | APP |
| P00568 | Adenylate kinase isoenzyme 1 | AK1 |
| P00918 | Carbonic anhydrase 2 | CA2 |
| P32119 | Peroxiredoxin-2 | PRDX2 |
| P16070-18;P16070-12; P16070-14;P16070-13; P16070-11;P16070-10; P16070-16;P16070-8;P16070-17; P16070-6;P16070-4;P16070-3; P16070-7;P16070-5;P16070; P16070-15;P16070-9 | CD44 antigen | CD44 |
| Q13201 | Multimerin-1;Platelet glycoprotein la*;155 kDa platelet multimerin | MMRN1 |
| P27348 | 14-3-3 protein theta | YWHAQ |
| O00592-2;O00592 | Podocalyxin | PODXL |
| P10599-2;P10599 | Thioredoxin | TXN |
| P00915 | Carbonic anhydrase 1 | CA1 |
| P62158 | Calmodulin | CALM1 |
| P58546 | Myotrophin | MTPN |
| P62258;P62258-2 | 14-3-3 protein epsilon | YWHAE |
| O94919 | Endonuclease domain-containing 1 protein | ENDOD1 |
| P37837 | Transaldolase | TALDO1 |
| P07738 | Bisphosphoglycerate mutase | BPGM |
| P30043 | Flavin reductase (NADPH) | BLVRB |
| P02775 | Platelet basic protein;Connective tissue-activating peptide III; TC-2;Connective tissue-activating peptide III(1-81);Beta-thromboglobulin;Neutrophil-activating peptide 2(74); Neutrophil-activating peptide 2(73);Neutrophil-activating peptide 2;TC-1;Neutrophil-activating peptide 2(1-66); Neutrophil-activating peptide 2(1-63) | PPBP |
| P15311 | Ezrin | EZR |
| P0DJI8;P0DJI9-2 | Serum amyloid A-1 protein;Amyloid protein A;Serum amyloid protein A(2-104);Serum amyloid protein A(3-104);Serum amyloid protein A(2-103);Serum amyloid protein A(2-102); Serum amyloid protein A(4-101);Serum amyloid A-2 protein | SAA1;SAA2 |
| P10124 | Serglycin | SRGN |
| P13798 | Acylamino-acid-releasing enzyme | APEH |
| P23528 | Cofilin-1 | CFL1 |

(continued)

| UniProt ID | Protein name | Gene name |
|---|---|---|
| P30041 | Peroxiredoxin-6 | PRDX6 |
| Q8WUM4;Q8WUM4-2 | Programmed cell death 6-interacting protein | PDCD6IP |
| P05090 | Apolipoprotein D | APOD |
| Q99497 | Protein deglycase DJ-1 | PARK7 |
| P35579;P35579-2 | Myosin-9 | MYH9 |
| Q13228;Q13228-4;Q13228-2; Q13228-3 | Selenium-binding protein 1 | SELENBP1 |
| P13716;P13716-2 | Delta-aminolevulinic acid dehydratase | ALAD |
| Q9UL13-2;Q9UL13 | Protein HEG homolog 1 | HEG1 |
| P07384 | Calpain-1 catalytic subunit | CAPN1 |
| P18065 | Insulin-like growth factor-binding protein 2 | IGFBP2 |
| P04083 | Annexin A1 | ANXA1 |
| P0DMV8-2;P0DMV9;P0DMV8 | Heat shock 70 kDa protein 1A;Heat shock 70 kDa protein 1B | HSPA1A; HSP A1B |
| P07911-3;P07911;P07911-5; P07911-4 | Uromodulin;Uromodulin, secreted form | UMOD |
| P15924;P15924-2;P15924-3 | Desmoplakin | DSP |

[Table 1B]

| UniProt ID | Protein name | Gene name |
|---|---|---|
| Q9C0H2-3 | Protein tweety homolog 3 | TTYH3 |

[Table 2A]

| Protein name | UniProt ID | Entrez Gene ID | Entrez Gene Symbol |
|---|---|---|---|
| Pulmonary surfactant-associated protein D | P35247 | 6441 | SFTPD |
| Histone H1.2 | P16403 | 3006 | HIST1H1C |
| Sialic acid-binding Ig-like lectin 9 | Q9Y336 | 27180 | SIGLEC9 |
| Heterogeneous nuclear ribonucleoproteins A2/B1 | P22626 | 3181 | HNRNPA2B1 |
| Hexokinase-2 | P52789 | 3099 | HK2 |
| High mobility group protein B1 | P09429 | 3146 | HMGB1 |
| Thrombospondin-1 | P07996 | 7057 | THBS1 |
| 3-hydroxy-3-methylglutaryl-coenzyme A reductase | P04035 | 3156 | HMGCR |
| Platelet factor 4 | P02776 | 5196 | PF4 |
| Protein S100-A9 | P06702 | 6280 | S100A9 |
| 40S ribosomal protein S3a | P61247 | 6189 | RPS3A |
| Annexin A6 | P08133 | 309 | ANXA6 |
| Inosine-5'-monophosphate dehydrogenase 1 | P20839 | 3614 | IMPDH1 |

(continued)

| Protein name | UniProt ID | Entrez Gene ID | Entrez Gene Symbol |
|---|---|---|---|
| Tyrosine-protein kinase Fgr | P09769 | 2268 | FGR |
| Serine/threonine-protein kinase 17B | O94768 | 9262 | STK17B |
| Neutrophil-activating peptide 2 | P02775 | 5473 | PPBP |
| Estradiol 17-beta-dehydrogenase 1 | P14061 | 3292 | HSD17B1 |
| Connective tissue-activating peptide III | P02775 | 5473 | PPBP |
| Prostaglandin G/H synthase 2 | P35354 | 5743 | PTGS2 |
| Amyloid beta A4 protein | P05067 | 351 | APP |
| GTP-binding nuclear protein Ran | P62826 | 5901 | RAN |
| NAD-dependent protein deacetylase sirtuin-2 | Q8IXJ6 | 22933 | SIRT2 |
| Protein S100-A12 | P80511 | 6283 | S100A12 |
| Plasminogen activator inhibitor 1 | P05121 | 5054 | SERPINE1 |
| SUMO-conjugating enzyme UBC9 | P63279 | 7329 | UBE2I |
| Bactericidal permeability-increasing protein | P17213 | 671 | BPI |
| 6-phosphogluconate dehydrogenase, decarboxylating | P52209 | 5226 | PGD |
| Platelet-derived growth factor subunit B | P01127 | 5155 | PDGFB |
| Tyrosine-protein phosphatase non-receptor type 6 | P29350 | 5777 | PTPN6 |
| Metalloproteinase inhibitor 3 | P35625 | 7078 | TIMP3 |
| NudC domain-containing protein 3 | Q8IVD9 | 23386 | NUDCD3 |
| SPARC | P09486 | 6678 | SPARC |
| Protein 4.1 | P11171 | 2035 | EPB41 |
| Sex hormone-binding globulin | P04278 | 6462 | SHBG |
| Death-associated protein kinase 2 | Q9UIK4 | 23604 | DAPK2 |
| Hepatoma-derived growth factor-related protein 2 | Q7Z4V5 | 84717 | HDGFRP2 |
| Proto-oncogene vav | P15498 | 7409 | VAV1 |
| Serine/threonine-protein kinase pim-1 | P11309 | 5292 | PIM1 |
| Heterogeneous nuclear ribonucleoprotein A/B | Q99729 | 3182 | HNRNPAB |
| Proliferation-associated protein 2G4 | Q9UQ80 | 5036 | PA2G4 |
| Extracellular superoxide dismutase [Cu-Zn] | P08294 | 6649 | SOD3 |
| Angiopoietin-1 | Q15389 | 284 | ANGPT1 |
| Acid sphingomyelinase-like phosphodiesterase 3a | Q92484 | 10924 | SMPDL3A |
| Peroxiredoxin-6 | P30041 | 9588 | PRDX6 |
| AMP Kinase (alpha1beta1gamma1) | Q13131 Q9Y478 P54619 | 5562 5564 5571 | PRKAA1 PRKAB1 PRKAG1 |
| Serum amyloid A-1 protein | P0DJI8 | 6288 | SAA1 |
| Insulin-like growth factor-binding protein 2 | P18065 | 3485 | IGFBP2 |
| Histone H2B type 2-E | Q16778 | 8349 | HIST2H2BE |

(continued)

| Protein name | UniProt ID | Entrez Gene ID | Entrez Gene Symbol |
|---|---|---|---|
| Fibroblast growth factor 5 | P12034 | 2250 | FGF5 |
| Non-histone chromosomal protein HMG-14 | P05114 | 3150 | HMGN1 |
| Calcium/calmodulin-dependent protein kinase type II subunit delta | Q13557 | 817 | CAMK2D |
| Interstitial collagenase | P03956 | 4312 | MMP1 |
| ICOS ligand | O75144 | 23308 | ICOSLG |
| Calcium/calmodulin-dependent protein kinase type II subunit beta | Q13554 | 816 | CAMK2B |
| Inosine-5'-monophosphate dehydrogenase 2 | P12268 | 3615 | IMPDH2 |
| Dickkopf-related protein 4 | Q9UBT3 | 27121 | DKK4 |
| Glia-derived nexin | P07093 | 5270 | SERPINE2 |
| 14-3-3 protein beta/alpha | P31946 | 7529 | YWHAB |
| Macrophage-capping protein | P40121 | 822 | CAPG |
| ADP-ribosyl cyclase/cyclic ADP-ribose hydrolase 1 | P28907 | 952 | CD38 |
| SH2 domain-containing protein 1A | O60880 | 4068 | SH2D1A |
| Vacuolar protein sorting-associated protein VTA1 homolog | Q9NP79 | 51534 | VTA1 |
| Interleukin-1 beta | P01584 | 3553 | IL1B |
| Chloride intracellular channel protein 1 | O00299 | 1192 | CLIC1 |
| MAP kinase-activated protein kinase 3 | Q16644 | 7867 | MAPKAPK3 |
| Mitogen-activated protein kinase 1 | P28482 | 5594 | MAPK1 |
| Choline/ethanolamine kinase | Q9Y259 | 1120 | CHKB |
| Creatine kinase B-type | P12277 | 1152 | CKB |
| DNA topoisomerase 1 | P11387 | 7150 | TOP1 |
| C-C motif chemokine 5 | P13501 | 6352 | CCL5 |
| C3a anaphylatoxin | P01024 | 718 | C3 |
| Copine-1 | Q99829 | 8904 | CPNE1 |
| Chymase | P23946 | 1215 | CMA1 |
| Mitogen-activated protein kinase kinase kinase 7:TGF-beta-activated kinase 1 and MAP3K7-binding protein 1 fusion | O43318 Q15750 | 6885 10454 | MAP3K7 TAB1 |
| Dickkopf-related protein 1 | O94907 | 22943 | DKK1 |
| Ephrin type-B receptor 4 | P54760 | 2050 | EPHB4 |
| 40S ribosomal protein S7 | P62081 | 6201 | RPS7 |
| Intercellular adhesion molecule 1 | P05362 | 3383 | ICAM1 |
| Ubiquitin+1, truncated mutation for UbB | P62979 | 6233 | RPS27A |
| Ubiquitin-conjugating enzyme E2 N | P61088 | 7334 | UBE2N |
| Moesin | P26038 | 4478 | MSN |
| Small ubiquitin-related modifier 3 | P55854 | 6613 | SUMO3 |
| G2/mitotic-specific cyclin-B1 | P14635 | 891 | CCNB1 |

(continued)

| Protein name | UniProt ID | Entrez Gene ID | Entrez Gene Symbol |
|---|---|---|---|
| ATP-dependent RNA helicase DDX19B | Q9UMR2 | 11269 | DDX19B |
| Tyrosine-protein kinase CSK | P41240 | 1445 | CSK |
| Alpha-1-antitrypsin | P01009 | 5265 | SERPINA1 |
| Adenylate kinase isoenzyme 1 | P00568 | 203 | AK1 |
| Mothers against decapentaplegic homolog 3 | P84022 | 4088 | SMAD3 |
| Brain-derived neurotrophic factor | P23560 | 627 | BDNF |
| Atrial natriuretic factor | P01160 | 4878 | NPPA |
| Annexin A1 | P04083 | 301 | ANXA1 |
| Signal transducer and activator of transcription 1-alpha/beta | P42224 | 6772 | STAT1 |
| Angiopoietin-related protein 4 | Q9BY76 | 51129 | ANGPTL4 |
| Cadherin-12 | P55289 | 1010 | CDH12 |
| Stress-induced-phosphoprotein 1 | P31948 | 10963 | STIP1 |
| Glycylpeptide N-tetradecanoyltransferase 1 | P30419 | 4836 | NMT1 |
| Peroxiredoxin-1 | Q06830 | 5052 | PRDX1 |
| Oxidized low-density lipoprotein receptor 1 | P78380 | 4973 | OLR1 |
| VPS10 domain-containing receptor SorCS2 | Q96PQ0 | 57537 | SORCS2 |
| FACT complex subunit SSRP1 | Q08945 | 6749 | SSRP1 |
| Carbonic anhydrase 1 | P00915 | 759 | CA1 |
| Mitogen-activated protein kinase 11 | Q15759 | 5600 | MAPK11 |
| Triosephosphate isomerase | P60174 | 7167 | TPI1 |
| Neurexophilin-1 | P58417 | 30010 | NXPH1 |
| Platelet-derived growth factor subunit A | P04085 | 5154 | PDGFA |
| Interleukin-22 receptor subunit alpha-1 | Q8N6P7 | 58985 | IL22RA1 |

[Table 2B]

| Protein name | UniProt ID | Entrez Gene ID | Entrez Gene Symbol |
|---|---|---|---|
| Cystatin-SN | P01037 | 1469 | CST1 |
| Interleukin-22 | Q9GZX6 | 50616 | IL22 |
| Tyrosine-protein kinase Fyn | P06241 | 2534 | FYN |
| Biglycan | P21810 | 633 | BGN |
| Sonic hedgehog protein | Q15465 | 6469 | SHH |
| Cathepsin L2 | O60911 | 1515 | CTSV |
| Interferon alpha/beta receptor 1 | P17181 | 3454 | IFNAR1 |
| Ectodysplasin-A, secreted form | Q92838 | 1896 | EDA |
| Dynein light chain 1, cytoplasmic | P63167 | 8655 | DYNLL1 |

(continued)

| Protein name | UniProt ID | Entrez Gene ID | Entrez Gene Symbol |
|---|---|---|---|
| Cytochrome c | P99999 | 54205 | CYCS |
| Lactoperoxidase | P22079 | 4025 | LPO |
| Tumor necrosis factor receptor superfamily member 19L | Q969Z4 | 84957 | RELT |
| Growth factor receptor-bound protein 2 | P62993 | 2885 | GRB2 |
| Ectonucleotide pyrophosphatase/phosphodiesterase family member 7 | Q6UWV6 | 339221 | ENPP7 |
| Glucagon | P01275 | 2641 | GCG |
| Eukaryotic translation initiation factor 4 gamma 2 | P78344 | 1982 | EIF4G2 |
| C-X-C motif chemokine 10 | P02778 | 3627 | CXCL10 |
| Platelet-derived growth factor receptor beta | P09619 | 5159 | PDGFRB |
| von Willebrand factor | P04275 | 7450 | VWF |
| Iduronate 2-sulfatase | P22304 | 3423 | IDS |
| cGMP-specific 3',5'-cyclic phosphodiesterase | O76074 | 8654 | PDE5A |
| Small glutamine-rich tetratricopeptide repeat-containing protein alpha | O43765 | 6449 | SGTA |
| Interleukin-25 | Q9H293 | 64806 | IL25 |
| MHC class I polypeptide-related sequence B | Q29980 | 4277 | MICB |
| C-C motif chemokine 7 | P80098 | 6354 | CCL7 |
| Lactadherin | Q08431 | 4240 | MFGE8 |
| Macrophage metalloelastase | P39900 | 4321 | MMP12 |
| Ubiquitin-like protein ISG15 | P05161 | 9636 | ISG15 |
| Fatty acid-binding protein, liver | P07148 | 2168 | FABP1 |
| Properdin | P27918 | 5199 | CFP |
| Stromelysin-2 | P09238 | 4319 | MMP10 |
| Protein FAM107B | Q9H098 | 83641 | FAM107B |
| Myosin-binding protein C, slow-type | Q00872 | 4604 | MYBPC1 |
| Serine/threonine-protein kinase Chk2 | O96017 | 11200 | CHEK2 |
| Xaa-Pro aminopeptidase 1 | Q9NQW7 | 7511 | XPNPEP1 |
| NT-3 growth factor receptor | Q16288 | 4916 | NTRK3 |
| Interleukin-5 receptor subunit alpha | Q01344 | 3568 | IL5RA |
| Ephrin type-A receptor 2 | P29317 | 1969 | EPHA2 |
| Peptidyl-prolyl cis-trans isomerase F, mitochondrial | P30405 | 10105 | PPIF |
| B-cell lymphoma 6 protein | P41182 | 604 | BCL6 |
| Cell adhesion molecule 1 | Q9BY67 | 23705 | CADM1 |
| Kinesin-like protein KIF23 | Q02241 | 9493 | KIF23 |
| Netrin receptor UNC5D | Q6UXZ4 | 137970 | UNC5D |
| Lymphocyte activation gene 3 protein | P18627 | 3902 | LAG3 |

(continued)

| Protein name | UniProt ID | Entrez Gene ID | Entrez Gene Symbol |
|---|---|---|---|
| Serine protease HTRA2, mitochondrial | O43464 | 27429 | HTRA2 |
| Interleukin-22 receptor subunit alpha-2 | Q969J5 | 116379 | IL22RA2 |
| Caspase-3 | P42574 | 836 | CASP3 |
| Platelet-derived growth factor receptor alpha | P16234 | 5156 | PDGFRA |
| Interleukin-20 | Q9NYY1 | 50604 | IL20 |
| Ferritin | P02794 P02792 | 2495 2512 | FTH1 FTL |
| Ephrin-A5 | P52803 | 1946 | EFNA5 |
| Amphoterin-induced protein 2 | Q86SJ2 | 347902 | AMIGO2 |
| Interleukin-2 | P60568 | 3558 | IL2 |
| Phospholipase A2 | P04054 | 5319 | PLA2G1B |
| Protein kinase C alpha type | P17252 | 5578 | PRKCA |
| Tumor necrosis factor ligand superfamily member 8 | P32971 | 944 | TNFSF8 |
| Endoglin | P17813 | 2022 | ENG |
| Gamma-enolase | P09104 | 2026 | ENO2 |
| C-X-C motif chemokine 9 | Q07325 | 4283 | CXCL9 |
| beta-nerve growth factor | P01138 | 4803 | NGF |
| Hepcidin | P81172 | 57817 | HAMP |
| Inorganic pyrophosphatase | Q15181 | 5464 | PPA1 |
| Kallikrein-8 | O60269 | 11202 | KLK8 |
| Semaphorin-6B | Q9H3T3 | 10501 | SEMA6B |
| Adapter molecule crk | P46108 | 1398 | CRK |
| Phosphoglycerate mutase 1 | P18669 | 5223 | PGAM1 |
| Proprotein convertase subtilisin/kexin type 7 | Q16549 | 9159 | PCSK7 |
| Pancreatic hormone | P01298 | 5539 | PPY |
| Interleukin-1 receptor type 1 | P14778 | 3554 | IL1R1 |
| C-X-C motif chemokine 11 | O14625 | 6373 | CXCL11 |
| Secreted frizzled-related protein 1 | Q8N474 | 6422 | SFRP1 |
| Inhibin beta A chain | P08476 | 3624 | INHBA |
| Immunoglobulin D | P01880 | 3495 50802 3535 | IGHD IGK@ IGL@ |
| Complement C3b | P01024 | 718 | C3 |
| Teratocarcinoma-derived growth factor 1 | P13385 | 6997 | TDGF1 |
| Brother of CDO | Q9BWV1 | 91653 | BOC |
| CD109 antigen | Q6YHK3 | 135228 | CD109 |
| Aminoacylase-1 | Q03154 | 95 | ACY1 |
| 60 kDa heat shock protein, mitochondrial | P10809 | 3329 | HSPD1 |

(continued)

| Protein name | UniProt ID | Entrez Gene ID | Entrez Gene Symbol |
|---|---|---|---|
| Importin subunit beta-1 | Q14974 | 3837 | KPNB1 |
| C-reactive protein | P02741 | 1401 | CRP |
| Ephrin type-A receptor 1 | P21709 | 2041 | EPHA1 |
| Semaphorin-6A | Q9H2E6 | 57556 | SEMA6A |
| SLIT and NTRK-like protein 5 | O94991 | 26050 | SLITRK5 |
| Leucine-rich repeat transmembrane protein FLRT3 | Q9NZU0 | 23767 | FLRT3 |
| dCTP pyrophosphatase 1 | Q9H773 | 79077 | DCTPP1 |
| Osteopontin | P10451 | 6696 | SPP1 |
| Form imidoyltransferase-cyclodeaminase | O95954 | 10841 | FTCD |
| Ephrin-B2 | P52799 | 1948 | EFNB2 |
| T-lymphocyte surface antigen Ly-9 | Q9HBG7 | 4063 | LY9 |
| Sialic acid-binding Ig-like lectin 14 | Q08ET2 | 100049587 | SIGLEC14 |
| N-acylethanolamine-hydrolyzing acid amidase | Q02083 | 27163 | NAAA |
| Endoplasmic reticulum aminopeptidase 1 | Q9NZ08 | 51752 | ERAP1 |
| Low affinity immunoglobulin gamma Fc region receptor II-a | P12318 | 2212 | FCGR2A |

[Table 5A]

| UniProt ID | Protein name | Gene name |
|---|---|---|
| Q15389 | Angiopoietin-1 (ANG-1) | ANGPT1 |
| P23560 | Brain-Derived Neurotrophic Factor (BDNF) | BDNF |
| O94907 | Dickkopf-related protein 1 (DKK-1) | DKK1 |
| P80511 | S100-A12 | S100A12 |
| P42830 | Epithelial-Derived Neutrophil-Activating Protein 78 (ENA-78) | CXCL5 |
| P02794 | Ferritin (FRTN) | FTH1 |
| P09341 | Growth-Regulated alpha protein (GRO-alpha) | CXCL1 |
| P01137 | Latency-Associated Peptide of Transforming Growth Factor beta 1 (LAP TGF-b1) | TGFB1 |
| Q8WXL0 | Luteinizing Hormone (LH) | LHB |
| G3CBL7 | MHC class I chain-related protein A (MICA) | MICA |
| P02775 | Platelet basic protein | PPBP |
| P01127 | Platelet-Derived Growth Factor BB (PDGF-BB) | PDGFB |
| P01236 | Prolactin (PRL) | PRL |
| PODJI8; PODJI9-2 | Serum amyloid A-1 protein;Amyloid protein A;Serum amyloid protein A(2-104); Serum amyloid protein A(3-104);Serum amyloid protein A(2-103);Serum amyloid protein A(2-102);Serum amyloid protein A(4-101);Serum amyloid A-2 protein | SAA1;SAA2 |
| P13501 | T-Cell-Specific Protein RANTES (RANTES) | CCL5 |

[Table 5B]

| UniProt ID | Protein name | Gene name |
|---|---|---|
| P10645 | Chromogranin-A (CgA) | CHGA |
| Q9GZV9 | Fibroblast growth factor 23 (FGF-23) | FGF23 |
| P01266 | Thyroglobul in (TG) | TG |

[Table 6A]

| UniProt ID | Protein name | Gene name |
|---|---|---|
| P07996 | Thrombospondin-1 0S=Homo sapiens 0X=9606 GN=THBS1 PE=1 SV=2 | THBS1 |
| P69905 | Hemoglobin subunit alpha 0S=Homo sapiens 0X=9606 GN=HBA1 PE=1 SV=2 | HBA1 |
| P32119 | Peroxiredoxin-2 0S=Homo sapiens 0X=9606 GN=PRDX2 PE=1 SV=5 | PRDX2 |
| P00918 | Carbonic anhydrase 2 0S=Homo sapiens 0X=9606 GN=CA2 PE=1 SV=2 | CA2 |
| P30043 | Flavin reductase (NADPH) 0S=Homo sapiens 0X=9606 GN=BLVRB PE=1 SV=3 | BLVRB |
| 076011 | Keratin, type I cuticular Ha4 0S=Homo sapiens 0X=9606 GN=KRT34 PE=1 SV=2 | KRT34 |
| P13716 | Delta-aminolevulinic acid dehydratase 0S=Homo sapiens 0X=9606 GN=ALAD PE=1 SV=1 | ALAD |
| P00568 | Adenylate kinase isoenzyme 1 0S=Homo sapiens 0X=9606 GN=AK1 PE=1 SV=3 | AK1 |
| P07738 | Bisphosphoglycerate mutase 0S=Homo sapiens 0X=9606 GN=BPGM PE=1 SV=2 | BPGM |
| P02775 | Platelet basic protein 0S=Homo sapiens 0X=9606 GN=PPBP PE=1 SV=3 | PPBP |
| P22392 | Nucleoside diphosphate kinase B 0S=Homo sapiens 0X=9606 GN=NME2 PE=1 SV=1 | NME2 |
| P10124 | Serglycin 0S=Homo sapiens 0X=9606 GN=SRGN PE=1 SV=3 | SRGN |
| A0A0C4DH67 | Immunoglobulin kappa variable 1-8 0S=Homo sapiens 0X=9606 GN=IGKV1-8 PE=3 SV=1 | IGKV1-8 |
| P63241 | Eukaryotic translation initiation factor 5A-1 0S=Homo sapiens 0X=9606 GN=EIF5A PE=1 SV=2 | EIF5A |
| P02776 | Platelet factor 4 0S=Homo sapiens 0X=9606 GN=PF4 PE=1 SV=2 | PF4 |
| A0A075B6S2 | Immunoglobulin kappa variable 2D-29 0S=Homo sapiens 0X=9606 GN=IGKV2D-29 PE=3 SV=1 | IGKV2D-29 |
| Q9UKU6 | Thyrotropin-releasing hormone-degrading ectoenzyme 0S=Homo sapiens 0X=9606 GN=TRHDE PE=2 SV=1 | TRHDE |
| Q8WUM4 | Programmed cell death 6-interacting protein 0S=Homo sapiens 0X=9606 GN=PDCD6IP PE=1 SV=1 | PDCD6IP |
| P00441 | Superoxide dismutase [Cu-Zn] 0S=Homo sapiens 0X=9606 GN=SOD1 PE=1 SV=2 | SOD1 |

[Table 6B]

| UniProt ID | Protein name | Gene name |
|---|---|---|
| P36980 | Complement factor H-related protein 2 0S=Homo sapiens 0X=9606 GN=CFHR2 PE=1 SV=1 | CFHR2 |
| PODOX3 | Immunoglobulin delta heavy chain 0S=Homo sapiens 0X=9606 PE=1 SV=1 | N/A |

(continued)

| UniProt ID | Protein name | Gene name |
|---|---|---|
| P01880 | Immunoglobulin heavy constant delta 0S=Homo sapiens 0X=9606 GN=IGHD PE=1 SV=3 | IGHD |
| Q8N6C8 | Leukocyte immunoglobulin-like receptor subfamily A member 3 0S=Homo sapiens 0X=9606 GN=LILRA3 PE=1 SV=3 | LILRA3 |
| P47929 | Galectin-7 0S=Homo sapiens 0X=9606 GN=LGALS7 PE=1 SV=2 | LGALS7 |
| 075339 | Cartilage intermediate layer protein 1 0S=Homo sapiens 0X=9606 GN=CILP PE=1 SV=4 | CILP |
| 015389 | Sialic acid-binding Ig-like lectin 5 0S=Homo sapiens 0X=9606 GN=SIGLEC5 PE=1 SV=1 | SIGLEC5 |
| P35247 | Pulmonary surfactant-associated protein D 0S=Homo sapiens 0X=9606 GN=SFTPD PE=1 SV=3 | SFTPD |

The sequences of the markers (mRNA and protein) listed in these tables can be easily retrieved from the database known to those skilled in the art (Uniprot: https://www.uniprot.org/) on the basis of the Uniprot IDs shown in the above tables. Specifically, a Uniprot ID can be used to search for the corresponding Uniprot entry to retrieve sequences provided in the "sequences" section. It is also possible to obtain IDs/accession numbers for other databases (for example, RefSeq, EMBL, GenBank, DDBJ, CCDS, PIR, UniGene, Ensemble, GeneID, KEGG, and USCS) from the description of the "sequences" section, and retrieve more sequences from those databases. In the present disclosure, the sequence of each marker refers to a sequence and an ID/accession number provided in the latest version of the corresponding Uniprot entry with a release number earlier than 2018-09 (for example, 2018-08; if 2018-08 does not exist, then 2018_07; if neither 2018_08 nor 2018_07 exists, then 2018_06; the same applies to numbers earlier than 2018_06).

Furthermore, in the FTP where the archives of Uniprot release data are stored (ftp://ftp.uniprot.org/pub/databases/uniprot/previous_releases/), the amino acid sequences of the markers in the above tables can also be retrieved from the data included in directory "release-2018_08/knowledgebase" (the "knowledgebase" directory included in "release-2018_08") by using the Uniprot IDs shown in the tables.

The groups of markers listed in the above tables include markers indicating fibrosis (TGFB1, SPARC, *etc*.). Furthermore, the groups of markers listed in the above tables include many molecules considered to be derived from platelets. For example, uniprot says that PPBP, THBS1, PF4, SERPINA1, TIMP3, APP, CALU, CASP3, MMRN1, SAA1, SRGN, VWF, and others are involved in platelet degranulation and such. Zhang *et al*. demonstrated in an *in vitro* experimental system using endometrial-derived cells that TGFb1 released from activated platelets induced epithelial-mesenchymal transition (EMT) and fibroblast-to-myofibroblast transdifferentiation (FMT), increased collagen production, and induced fibrosis (Molecular and Cellular Endocrinology, 428, 1-16, 2016). This is consistent with the fact that TGFb1 and platelet-derived proteins were selected as markers in the present disclosure, suggesting that changes in these proteins may reflect changes in platelet activation and such.

Type V collagen MMP degradation products are also called MMP mediated type V collagen degradation and are products resulting from degradation of the alpha 3 chain (Refseq: NP_056534.2, SEQ ID NO: 1) of type V collagen by MMP Specifically, the type V collagen MMP degradation products are type V collagen degradation products produced by cleaving type V collagen shown in SEQ ID NO: 1 between amino acids 1316 (G) and 1317 (H) (Clin Biochem. 2012 May;45(7-8):541-6). Specifically, any polypeptides are included in the type V collagen MMP degradation products as long as they are derived from type V collagen and comprise the amino acid sequence set forth in SEQ ID NO: 4 at the N-terminus and/or comprise the amino acid sequence set forth in SEQ ID NO: 6 at the C-terminus. Examples of the type V collagen MMP degradation products include protein fragments shown in SEQ ID NO: 2 or 3. Among the type V collagen MMP degradation products, the peptide fragment of SEQ ID NO: 4 (HMGREGREGE) is sometimes referred to as C5M.

The presence of a type V collagen MMP degradation product is detected by any method that can detect a type V collagen fragment after cleavage between amino acids 1316 (G) and 1317 (H) set forth in SEQ ID NO: 1. In a certain embodiment, an antibody that specifically recognizes peptide HMGREGREGE (SEQ ID NO: 4) located at the N-terminus of the type V collagen fragment shown in SEQ ID NO: 2 can be used to detect the abundance of a type V collagen MMP degradation product. In a more specific embodiment, an antibody that can recognize peptide HMGREGREGE (SEQ ID NO: 4) located at the N-terminus of the type V collagen fragment shown in SEQ ID NO: 2 and that does not recognize GHMGREGREGE (SEQ ID NO: 5) can be used to detect the abundance of a type V collagen MMP degradation product (an ELISA detection method that uses such an antibody was established in Clin Biochem. 2012 May;45(7-8):541-6.). In another embodiment, an antibody that specifically recognizes peptide GPPGKRGPSG (SEQ ID NO: 6) located at the C-terminus of the type V collagen fragment set forth in SEQ ID NO: 3 can be used to detect the abundance of a type V collagen MMP degradation product. In a more specific embodiment, an antibody that can recognize peptide GPPGKRGPSG (SEQ ID NO: 6) located at the C-terminus of the type V collagen fragment shown in SEQ ID NO: 3 and that does not recognize GPPGKRGPSGH (SEQ ID NO: 7) can be used to detect the abundance of a type V collagen MMP degradation product.

In the present disclosure, a protein selected from the group consisting of type V collagen MMP degradation products, the markers shown in Table 1A, the markers shown in Table 2A, the markers shown in Table 1B, the markers shown in Table 2B, the markers shown in Table 5A, the markers shown in in Table 6A, the markers shown in Table 5B, and the markers shown in Table 6B may be used alone as a marker, or a combination of selected proteins may be used as markers. When more than one protein is used as markers, each of the markers may be measured individually and then the results may be combined, or the markers may be measured together.

The abundance of the markers of the present disclosure can be measured using the measuring methods described in the present disclosure or using commercially available measuring reagents. Specific measured values and predetermined values of each marker described herein can be construed as values measured using the above-mentioned measuring reagents.

The predetermined values (control levels) determined for the markers of the present disclosure can vary depending on the type of a sample of a subject for which the abundance of each marker is measured, but can be determined, for example, from the range of 0.1 to 100 ng/mL. Alternatively, it can be determined from the range of 0.2 to 90 ng/mL, 0.3 to 80 ng/mL, 0.4 to 70 ng/mL, 0.5 to 60 ng/mL, 1.0 to 50 ng/mL, 1.5 to 40 ng/mL, 2.0 to 30 ng/mL, 2.5 to 20 ng/mL, 3.0 to 10 ng/mL, and so on, but is not limited thereto.

The predetermined value determined for each marker of the present disclosure can be determined from values such as 0.1 ng/mL, 0.2 ng/mL, 0.3 ng/mL, 0.4 ng/mL, 0.5 ng/mL, 0.6 ng/mL, 0.7 ng/mL, 0.8 ng/mL, 0.9 ng/mL, 1.0 ng/mL, 1.5 ng/mL, 2.0 ng/mL, 2.5 ng/mL, 3.0 ng/mL, 3.5 ng/mL, 4.0 ng/mL, 4.5 ng/mL, 5.0 ng/mL, 5.5 ng/mL, 6.0 ng/mL, 6.5 ng/mL, 7.0 ng/mL, 7.5 ng/mL, 8.0 ng/mL, 8.5 ng/mL, 9.0 ng/mL, 9.5 ng/mL, 10 ng/mL, 15 ng/mL, 20 ng/mL, 25 ng/mL, 30 ng/mL, 35 ng/mL, 40 ng/mL, 45 ng/mL, 50 ng/mL, 60 ng/mL, 70 ng/mL, 80 ng/mL, 90 ng/mL, and 100 ng/mL, but is not limited thereto.

In the present disclosure, when the abundance of each marker of the present disclosure is greater than the predetermined value (control level), it can be decided that "the abundance is high" or "the abundance is large." For example, in the present disclosure, it can be decided that "the abundance is high" or "the abundance is large" when, without limitation, the abundance of at least one marker selected from the group consisting of type V collagen MMP degradation products, the markers shown in Table 1A, the markers shown in Table 2A, and the markers shown in Table 5A is, for example, 1.1 times or more, 1.2 times or more, 1.3 times or more, 1.4 times or more, 1.5 times or more, 1.6 times or more, 1.7 times or more, 1.8 times or more, 1.9 times or more, 2.0 times or more, 2.1 times or more, 2.2 times or more, 2.3 times or more, 2.4 times or more, 2.5 times or more, 2.6 times or more, 2.7 times or more, 2.8 times or more, 2.9 times or more, or 3.0 times or more the abundance of the same marker in a sample obtained from a healthy individual, or more. Alternatively, in the present disclosure, it can be decided that "the abundance is high" or "the abundance is large" when, without limitation, the abundance of at least one marker selected from the markers shown in Table 6A is, for example, 1.10 times or more, 1.15 times or more, 1.20 times or more, 1.25 times or more, 1.30 times or more, 1.35 times or more, 1.40 times or more, 1.45 times or more, 1.50 times or more, 1.55 times or more, 1.60 times or more, 1.65 times or more, 1.70 times or more, 1.75 times or more, 1.80 times or more, 1.85 times or more, 1.90 times or more, 1.95 times or more, or 2.00 times or more the abundance of the same marker in a sample obtained from a healthy individual, or more.

In the present disclosure, when the abundance of each marker of the present disclosure is less than the predetermined value (control level), it can be decided that "the abundance is low" or "the abundance is small." For example, in the present disclosure, it can be decided that "the abundance is low" or "the abundance is small" when, without limitation, the abundance of at least one marker selected from the group consisting of type V collagen MMP degradation products, the markers shown in Table 1B, the markers shown in Table 2B, and the markers shown in Table 5B is, for example, 0.9 times or less, 0.8 times or less, 0.7 times or less, 0.6 times or less, 0.5 times or less, 0.4 times or less, 0.3 times or less, 0.2 times or less, or 0.1 times or less the abundance of the same marker in a sample obtained from a healthy individual, or less. Alternatively, in the present disclosure, it can be decided that "the abundance is low" or "the abundance is small" when, without limitation, the abundance of at least one marker selected from the markers shown in Table 6B is, for example, 0.9 times or less, 0.8 times or less, 0.7 times or less, 0.6 times or less, 0.5 times or less, 0.4 times or less, 0.3 times or less, 0.2 times or less, or 0.1 times or less the abundance of the same marker in a sample obtained from a healthy individual, or less.

Diagnosis of endometriosis and determination of whether or not a subject is affected by endometriosis

One aspect of the present disclosure relates to methods of diagnosing endometriosis or methods of determining whether or not a subject is affected by endometriosis. One aspect of the present disclosure also relates to methods of detecting endometriosis or markers thereof. One aspect of the present disclosure also relates to methods of assisting the diagnosis or determination. One aspect of the present disclosure also relates to methods of providing instructions for the diagnosis or determination. One aspect of the present disclosure relates to reagents and kits for use in these methods.

The above respective methods comprise measuring the abundance of at least one marker selected from the group consisting of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in a sample obtained from the subject. The methods can also comprise comparing the abundance with a predetermined value (control level).

The above reagents are reagents for measuring the abundance of at least one marker selected from the group consisting of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B. The above kits contain such reagents.

The above respective methods may comprise a step in which if the abundance of at least one marker selected from the group consisting of type V collagen MMP degradation products, the markers shown in Table 1A, the markers shown in Table 2A, the markers shown in Table 5A, and the markers shown in Table 6A in the sample obtained from the subject is higher than a predetermined value (control level) or the abundance of at least one marker selected from the group consisting of the markers shown in Table 1B, the markers shown in Table 2B, the markers shown in Table 5B, and the markers shown in Table 6B in the sample obtained from the subject is lower than a predetermined value (control level), the subject that the sample is derived from is shown to be affected or potentially affected by endometriosis.

The above respective methods can be performed both *in vivo* and *in vitro,* but are preferably performed *in vitro.*

Furthermore, the above respective methods may also comprise obtaining a sample from the subject.

Moreover, the above respective methods may also comprise treating a subject that has been shown to be affected or potentially affected by endometriosis. The above respective methods may also comprise administering a known therapeutic agent for endometriosis to a subject that has been shown to be affected or potentially affected by endometriosis. Thus, the present invention relates to methods of treating endometriosis in a subject that has been shown to be affected or potentially affected by endometriosis by each of the above methods. Various therapeutic agents for endometriosis are known to those skilled in the art, and examples thereof include, but are not limited to, estrogen/progesterone mixtures, progesterone preparations, GnRH agonists, GnRH antagonists, and danazol.

The above-mentioned kits may further comprise instructions stating that if the abundance of at least one marker selected from the group consisting of type V collagen MMP degradation products, the markers shown in Table 1A, the markers shown in Table 2A, the markers shown in Table 5A, and the markers shown in Table 6A in the sample obtained from the subject is higher than a predetermined value (control level) or if the abundance of at least one marker selected from the group consisting of the markers shown in Table 1B, the markers shown in Table 2B, the markers shown in Table 5B, and the markers shown in Table 6B is lower than a predetermined value (control level), the subject that the sample is derived from is shown to be affected or potentially affected by endometriosis.

Determination of the extent of endometrial fibrosis in a subject

One aspect of the present disclosure relates to methods of determining the extent of endometrial fibrosis in a subject, methods of assisting the determination, methods of providing instructions for the determination, and reagents and kits for use in these.

The above respective methods comprise measuring the abundance of at least one marker selected from the group consisting of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in a sample obtained from the subject. The methods can also comprise comparing the abundance with a predetermined value (control level).

The above reagents are reagents for measuring the abundance of at least one marker selected from the group consisting of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B. The above-mentioned kits contain such reagents.

The above respective methods may comprise a step in which if the abundance of at least one marker selected from the group consisting of type V collagen MMP degradation products, the markers shown in Table 1A, the markers shown in Table 2A, the markers shown in Table 5A, and the markers shown in Table 6A in the sample obtained from the subject is higher than a predetermined value (control level) or if the abundance of at least one marker selected from the group consisting of the markers shown in Table 1B, the markers shown in Table 2B, the markers shown in Table 5B, and the markers shown in Table 6B in the sample obtained from the subject is lower than a predetermined value (control level), it is shown that endometrial fibrosis is occurring or potentially occurring in the subject that the sample is derived from.

The above respective methods can be performed both *in vivo* and *in vitro,* but is preferably performed *in vitro.*

Furthermore, the above respective methods may comprise obtaining a sample from the subject.

Moreover, the above respective methods may also comprise treating a subject in whom or which it has been shown that endometrial fibrosis has occurred or potentially occurred. The above respective methods may also comprise administering a known therapeutic agent for suppressing endometrial fibrosis to a subject in whom or which it has been shown that endometrial fibrosis has occurred or potentially occurred. Thus, the present invention relates to methods of suppressing fibrosis in a subject in whom or which it has been shown by the above respective methods that endometrial fibrosis has occurred or potentially occurred. Examples of therapeutic agents for suppressing endometrial fibrosis include the above-mentioned therapeutic agents for endometriosis.

The above kits may further comprise instructions stating that if the abundance of at least one marker selected from the group consisting of type V collagen MMP degradation products, the markers shown in Table 1A, the markers shown in Table 2A, the markers shown in Table 5A, and the markers shown in Table 6A is higher than a predetermined value (control level) or the abundance of at least one marker selected from the group consisting of the markers shown in Table 1B, the markers shown in Table 2B, the markers shown in Table 5B, and the markers shown in Table 6B is lower than a predetermined value (control level) in a sample obtained from the subject, it is shown that endometrial fibrosis has occurred or potentially occurred in the subject that the sample is derived from.

Determination of the extent of endometrial adhesion in a subject

One aspect of the present disclosure relates to methods of determining the extent of endometrial adhesion in a subject, methods of assisting the determination, methods of providing instructions for the determination, and reagents and kits for use in these methods.

The above respective methods comprise measuring the abundance of at least one marker selected from the group consisting of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in a sample obtained from the subject. The methods can also comprise comparing the abundance with a predetermined value (control level).

The above reagents are reagents for measuring the abundance of at least one marker selected from the group consisting of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B. The above kits contain such reagents.

The above respective methods may comprise a step in which if the abundance of at least one marker selected from the group consisting of type V collagen MMP degradation products, the markers shown in Table 1A, the markers shown in Table 2A, the markers shown in Table 5A, and the markers shown in Table 6A in a sample obtained from the subject is higher than a predetermined value (control level) or if the abundance of at least one marker selected from the group consisting of the markers shown in Table 1B, the markers shown in Table 2B, the markers shown in Table 5B, and the markers shown in Table 6B in a sample obtained from the subject is lower than a predetermined value (control level), it is shown that endometrial adhesion has occurred or potentially occurred in the subject that the sample is derived from.

The above respective methods can be performed both *in vivo* and *in vitro,* but are preferably performed *in vitro.*

Furthermore, The above respective methods may comprise obtaining a sample from the subject.

Moreover, the above respective methods may also comprise treating a subject in whom or which it has been shown that endometrial adhesion has occurred or potentially occurred. Each of the above methods may also comprise administering a known therapeutic agent for suppressing endometrial adhesion to a subject in whom or which it has been shown that endometrial adhesion has occurred or potentially occurred. Thus, the present invention relates to methods of suppressing endometrial adhesion in a subject in whom or which it has been shown by the above respective methods that endometrial adhesion has occurred or potentially occurred. Examples of therapeutic agents for suppressing endometrial adhesion include the above-mentioned therapeutic agents for endometriosis.

The above kits may further comprise instructions stating that if the abundance of at least one marker selected from the group consisting of the concentration of type V collagen MMP degradation products, the markers shown in Table 1A, the markers shown in Table 2A, the markers shown in Table 5A, and the markers shown in Table 6A is higher than a predetermined value (control level) or the abundance of at least one marker selected from the group consisting of the markers shown in Table 1B, the markers shown in Table 2B, the markers shown in Table 5B, and the markers shown in Table 6B is lower than a predetermined value (control level) in a sample obtained from the subject, it is shown that endometrial adhesion has occurred or potentially occurred in the subject that the sample is derived from.

Prediction of the degree of pain due to endometriosis in a subject affected or suspected of being affected by endometriosis

One aspect of the present disclosure relates to methods of predicting the degree of pain due to endometriosis in a subject affected or suspected of being affected by endometriosis, methods of assisting the prediction, methods of providing instructions for the prediction, and reagents and kits for use in these methods.

The above respective methods comprise measuring the abundance of at least one marker selected from the group consisting of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in a sample obtained from the subject. The methods can also comprise comparing the abundance with a predetermined value (control level).

The above reagents are reagents for measuring the abundance of at least one marker selected from the group consisting of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B. The above kits contain such reagents.

The above respective methods may comprise a step in which if the abundance of at least one marker selected from the group consisting of type V collagen MMP degradation products, the markers shown in Table 1A, the markers shown in Table 2A, the markers shown in Table 5A, and the markers shown in Table 6A in a sample obtained from the subject is higher than a predetermined value (control level) or if the abundance of at least one marker selected from the group consisting of the markers shown in Table 1B, the markers shown in Table 2B, the markers shown in Table 5B, and the markers shown in Table 6B in a sample obtained from the subject is lower than a predetermined value (control level), the subject that the sample is derived from is shown to develop or potentially develop pain due to endometriosis.

The above respective methods can be performed both *in vivo* and *in vitro,* but is preferably performed *in vitro.*

Furthermore, the above respective methods may comprise obtaining a sample from the subject.

Moreover, the above respective methods may also comprise alleviating pain due to endometriosis in a subject that has been shown to develop or potentially develop pain due to endometriosis. The above respective methods may also comprise administering a known therapeutic agent for suppressing pain due to endometriosis to a subject that has been shown to develop or potentially develop pain due to endometriosis. Thus, the present invention relates to methods of suppressing pain due to endometriosis in a subject that has been shown to develop or potentially develop pain due to endometriosis by the above respective methods. Examples of therapeutic agents for suppressing pain due to endometriosis include the above-mentioned therapeutic agents for endometriosis.

The above kits may further comprise instructions stating that if the abundance of at least one marker selected from the group consisting of type V collagen MMP degradation products, the markers shown in Table 1A, the markers shown in Table 2A, the markers shown in Table 5A, and the markers shown in Table 6A is higher than a predetermined value (control level) or the abundance of at least one marker selected from the group consisting of the markers shown in Table 1B, the markers shown in Table 2B, the markers shown in Table 5B, and the markers shown in Table 6B is lower than a predetermined value (control level) in a sample obtained from the subject, the subject that the sample is derived from is shown to develop or potentially develop pain due to endometriosis.

Determination of the degree of progression of endometriosis

One aspect of the present disclosure relates to methods of determining the degree of progression of endometriosis in a subject affected or suspected of being affected by endometriosis, methods of assisting the determination, methods of providing instructions for the determination, and reagents and kits for use in these methods.

The above respective methods comprise measuring the abundance of at least one marker selected from the group consisting of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in a sample obtained from the subject. The methods can also comprise comparing the abundance with a predetermined value (control level).

The above reagents are reagents for measuring the abundance of at least one marker selected from the group consisting of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B. The above kits contain such reagents.

The above respective methods may comprise a step in which if the abundance of at least one marker selected from the group consisting of type V collagen MMP degradation products, the markers shown in Table 1A, the markers shown in Table 2A, the markers shown in Table 5A, and the markers shown in Table 6A in a sample obtained from the subject is higher than a predetermined value (control level) or if the abundance of at least one marker selected from the group consisting of the markers shown in Table 1B, the markers shown in Table 2B, the markers shown in Table 5B, and the markers shown in Table 6B in a sample obtained from the subject is lower than a predetermined value (control level), it is shown that endometriosis is progressing or potentially progressing in the subject that the sample is derived from.

The above respective methods can be performed both *in vivo* and *in vitro,* but are preferably performed *in vitro.*

Furthermore, the above respective methods may comprise obtaining a sample from the subject.

Moreover, the above respective methods may also comprise treating a subject in whom or which it has been shown that endometriosis is progressing or potentially progressing. The above respective methods may also comprise administering a known therapeutic agent for suppressing progression of endometriosis to a subject in whom or which it has been shown that endometriosis is progressing or potentially progressing. Thus, the present invention relates to methods of suppressing progression of endometriosis in a subject in whom or which it has been shown by the above respective methods that endometriosis is progressing or potentially progressing. Examples of therapeutic agents for suppressing progression of endometriosis include the above-mentioned therapeutic agents for endometriosis.

The above kits may further comprise instructions stating that if the abundance of at least one marker selected from the group consisting of type V collagen MMP degradation products, the markers shown in Table 1A, the markers shown in Table 2A, the markers shown in Table 5A, and the markers shown in Table 6A is higher than a predetermined value (control level) or the abundance of at least one marker selected from the group consisting of the markers shown in Table 1B, the markers shown in Table 2B, the markers shown in Table 5B, and the markers shown in Table 6B is lower than a predetermined value (control level) in a sample obtained from the subject, it is shown that endometriosis is progressing or potentially progressing in the subject that the sample is derived from.

Monitoring of pathological conditions of endometriosis

One aspect of the present disclosure relates to methods of monitoring pathological conditions of endometriosis in a subject affected or suspected of being affected by endometriosis, methods of assisting the monitoring, methods of providing instructions for the monitoring, and reagents and kits for use in these methods.

The monitoring in the present disclosure includes evaluating a therapeutic effect and/or providing information on a future therapeutic regimen or therapeutic policy.

The above respective methods comprise measuring the abundance of at least one marker selected from the group consisting of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in a sample obtained from the subject.

The above reagents are reagents for measuring the abundance of at least one marker selected from the group consisting of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B. The above kits contain such reagents.

The above respective methods may comprise measuring each of the abundance of at least one marker selected from the group consisting of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in each of a plurality of samples collected from a subject at different points of time. The methods may also comprise comparing the abundance of the marker in each sample in a plurality of samples collected at different points of time. In the present disclosure, the timing and number of times of collecting samples are not limited, and samples can be collected at various points of time before treatment, during treatment, and after treatment as necessary. Samples can also be collected at various timings within each period of before treatment, during treatment, and after treatment. In the present disclosure, "plurality" is not particularly limited, and examples thereof include 2, 3, 4, 5, 6, 7, 8, 9, 10, or more times.

The above kits may comprise instructions stating that the abundance of at least one marker selected from the group consisting of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B in each of a plurality of samples obtained from a subject collected at different points of time is compared.

The above respective methods may comprise a step in which if the abundance of at least one marker selected from the group consisting of type V collagen MMP degradation products, the markers shown in Table 1A, the markers shown in Table 2A, the markers shown in Table 5A, and the markers shown in Table 6A decreased over time or if the abundance of at least one marker selected from the group consisting of the markers shown in Table 1B, the markers shown in Table 2B, the markers shown in Table 5B, and the markers shown in Table 6B increased over time, it is shown that the pathological conditions of endometriosis are improving or potentially improving in the subject that the sample is derived from.

The above respective methods may comprise a step in which if the abundance of at least one marker selected from the group consisting of type V collagen MMP degradation products, the markers shown in Table 1A, the markers shown in Table 2A, the markers shown in Table 5A, and the markers shown in Table 6A increased over time or if the abundance of at least one marker selected from the group consisting of the markers shown in Table 1B, the markers shown in Table 2B, the markers shown in Table 5B, and the markers shown in Table 6B decreased over time, it is shown that the pathological conditions of endometriosis are worsening or potentially worsening in the subject the sample is derived from.

The above respective methods can be performed both *in vivo* and *in vitro,* but are preferably performed *in vitro.*

Furthermore, the above respective methods may comprise obtaining a sample from the subject.

Moreover, the above respective methods may comprise treating a subject in whom or which it has been shown that the pathological conditions of endometriosis are worsening or potentially worsening. The above respective methods may also comprise administering a known therapeutic agent for endometriosis to a subject in whom or which it has been shown that the pathological conditions of endometriosis are worsening or potentially worsening. Thus, the present invention relates to methods of treating endometriosis in a subject in whom or which it has been indicated by each of the above methods that the pathological conditions of endometriosis are worsening or potentially worsening. The above-mentioned agents can be used as therapeutic agents for endometriosis.

The above kits may comprise instructions stating that if the abundance of at least one marker selected from the group consisting of type V collagen MMP degradation products, the markers shown in Table 1A, the markers shown in Table 2A, the markers shown in Table 5A, and the markers shown in Table 6A decreased over time or if the abundance of at least one marker selected from the group consisting of the markers shown in Table 1B, the markers shown in Table 2B, the markers shown in Table 5B, and the markers shown in Table 6B increased over time, it is shown that the pathological conditions of endometriosis are improving or potentially improving in the subject that the sample is derived from.

The above kits may comprise instructions stating that if the abundance of at least one marker selected from the group consisting of type V collagen MMP degradation products, the markers shown in Table 1A, the markers shown in Table 2A, the markers shown in Table 5A, and the markers shown in Table 6A increased over time or if the abundance of at least one marker selected from the group consisting of the markers shown in Table 1B, the markers shown in Table 2B, the markers shown in Table 5B, and the markers shown in Table 6B decreased over time, it is shown that the pathological conditions of endometriosis are worsening or potentially worsening in the subject that the sample is derived from.

Reagents/kits

Reagents contained in the kits of the present disclosure are not particularly limited as long as the abundance of markers can be measured. Reagents can be appropriately selected depending on the form of markers. When the form of a marker is a polypeptide, reagents comprising an antibody that specifically binds to the polypeptide are preferable. When the form of a marker is a polynucleotide, reagents comprising an oligonucleotide that specifically binds to the polynucleotide are preferable. In the present disclosure, the antibody or the polynucleotide itself may be a reagent. Polynucleotides in the present disclosure include those in the form of DNA and in the form of mRNA.

Antibodies in the present disclosure may be chimeric antibodies, humanized antibodies, or human antibodies. In some embodiments, antibodies may be multispecific antibodies, *e.g.*, bispecific antibodies. Alternatively, antibodies in the present disclosure may be antibody fragments or modified products thereof. For example, antibody fragments include Fab, F (ab')2, Fv, or single chain Fv (scFv) in which an H chain Fv and an L chain Fv are linked with an appropriate linker. These antibodies can be obtained by methods well known to those skilled in the art.

Antibodies can be labeled by commonly known methods. Labeling substances known to those skilled in the art, such as fluorescent dyes, enzymes, coenzymes, chemiluminescent substances, and radioactive substances, can be used.

Oligonucleotides in the present disclosure can be any oligonucleotide that specifically hybridizes to at least a portion of a polynucleotide that is a marker in the present disclosure or a complementary strand thereof. The nucleotide sequence of such an oligonucleotide for detecting a polynucleotide is selected from the nucleotide sequence complementary to the sense strand of a marker in the present disclosure. Oligonucleotides typically have a length of at least 15 bp or more.

Oligonucleotides in the present disclosure can also be used after being labeled by commonly known methods. Oligonucleotides in the present disclosure can be produced, for example, by commercially available oligonucleotide synthesizers.

Kits of the present disclosure preferably contain a positive control sample as a reference for measuring the abundance of markers. The positive control sample is not particularly limited as long as the amount of the markers contained therein has been determined in advance, and can be appropriately prepared depending on the form of markers measured by the kits. For example, when the form of a marker is a polypeptide, a positive control sample is preferably a sample comprising a polypeptide prepared by isolating, purifying, and quantifying the same polypeptide as the marker.

In addition to the above, kits of the present invention may include, for example, sterile water, saline, vegetable oil, surfactant, lipid, solubilizer, buffer, protein stabilizer (such as BSA and gelatin), preservative, blocking solution, reaction solution, reaction stop solution, and reagents for treating samples as necessary.

Sets of type V collagen MMP degradation products, the markers shown in Table 1A, the markers shown in Table 2A, the markers shown in Table 1B, the markers shown in Table 2B, the markers shown in Table 5A, the markers shown in Table 6A, the markers shown in Table 5B, and the markers shown in Table 6B are also included in the present disclosure. Such sets can be used for diagnosing endometriosis, determining the presence or absence of endometriosis, determining the extent of endometrial fibrosis or adhesion in patients affected by endometriosis, predicting pain of patients affected by endometriosis, monitoring pathological conditions of patients affected by endometriosis, and such.

All prior art documents cited herein are incorporated herein by reference.

[Examples]

The present disclosure will be further illustrated by the Examples below but is not limited thereto.

(Example 1) Search for biomarkers by LC-MS analysis using plasma of healthy human individuals and patients with endometriosis

(1-1) Obtainment of human plasma samples

Plasma from healthy individuals and endometriosis patients was purchased from Proteogenex.

(1-2) LC-MS analysis of plasma proteins

With the Seppro IgY14 column (Sigma aldrich), highly abundant proteins in each plasma sample were bound to the column and removed by the method recommended by the manufacturer. The proteins in the flow-through fraction that did not bind to the column were precipitated by the methanol/chloroform method and dissolved in 8 M urea/400 mM ammonium bicarbonate solution. The dissolved proteins were reduced and alkylated, and then digested into peptides with lysyl endopeptidase and trypsin. The peptide solution was demineralized with Monospin C18 (GL Science) by the method recommended by the manufacturer.

The peptides were labeled with TMT10plex Mass Tag Labeling Kits and Reagents (ThermoFisher Scientific) (hereinafter, referred to as TMT 10-plex) by the method recommended by the manufacturer. The labeled peptides were fractionated with Pierce™ High pH Reversed-Phase Peptide Fractionation Kit (ThermoFisher Scientific) by the method recommended by the manufacturer. Each fraction was analyzed by the LC-MS analysis system in which Orbitrap fusion Lumos (ThermoFisher scientific) was coupled to the nano-LC system (Ultimate3000, Dionex). The analysis samples included seven samples: the peptides obtained from the plasma of each of three healthy individuals and three endometriosis patients, and a sample (control) prepared by mixing an equal amount of these six peptide solutions. Each sample was analyzed three times, and an independent data set in each analysis was then analyzed (N = 3, hereinafter, data set TMT1, 2, and 3).

The proteins in the samples were identified from the raw data of the LC-MS analysis by the MaxQuant software (http://www.biochem.mpg.de/5111795/maxquant), and their relative expression levels were analyzed. The database search for protein identification was performed with the following parameters. Taxonomy: uniprot human, Fixed modification: carbamidomethylation (C), Variable modification: oxidation (M); deamidation (NQ), Acetyl (protein N-term), and FDR (protein, peptide) < 1%. The relative expression levels were calculated by the following formula:

$$\text{Relative expression level} = \frac{\text{Target protein signal in plasma samples (from patients with endometriosis or healthy individuals)}}{\text{Target protein signal in the control sample}}$$

The target protein signal value used in the above formula was calculated using the column of "Reporter intensity corrected" in Protein groups.txt, which is one of the output files of MaxQunat, where for each label of TMT 10-plex, the sum of the intensity was divided by the sum of the intensity of the control sample and multiplied by $1 \times 10^{11}$ using pipeline pilot (BIOVIA).

(1-3) Selection of biomarker candidate molecules

The LC-MS result data were analyzed for each data set of TMT1, 2, and 3 by statistical programming environment R and Microsoft Excel. From the relative expression level of each protein in each of the samples (three healthy individuals and three endometriosis patients), 58 proteins for which altered expression was found in endometriosis patients were selected according to the following criteria for each data set (Table 3A and Table 3B).

$$\frac{\text{The average value of the relative expression level of the protein in three patients with endometriosis}}{\text{The average value of the relative expression level of the protein in three healthy individuals}} \geqq 2 \text{ or} \leqq 0.5$$

- The relative expression level of the protein in three healthy individuals and three endometriosis patients was p < 0.05 in the Welch t-test.

[Table 3A]

| UniProt ID | Protein name | Gene name | Relative expression level of protein: Average value of 3 patients/Average value of 3 healthy individuals | | |
|---|---|---|---|---|---|
| | | | Data set TMT1 | Data set TMT2 | Data set TMT3 |
| P02042 | Hemoglobin subunit delta | HBD | 4.848 | 4.12 | 4.27 |
| P68871 | Hemoglobin subunit beta;LVV-hemorphin-7;Spinorphin | HBB | 3.662 | 4.58 | 4.153 |
| P55072 | Transitional endoplasmic reticulum ATPase | VCP | 2.915 | 3 | 2.306 |
| P50395; P50395-2 | Rab GDP dissociation inhibitor beta | GDI2 | 2.06 | 2.19 | 1.784 |
| O43852-9;O43852-5; O43852-2;O43852; O43852-4;O43852-3; 043852-12;043852-13; O43852-14;O43852-7; O43852-15;O43852-11; O43852-8;O43852-10; 043852-6 | Calumenin | CALU | 6.081 | 5.24 | 4.619 |
| P04040 | Catalase | CAT | 3.085 | 3.36 | 3.258 |
| Q06830;Q13162 | Peroxiredoxin-1 ;Peroxiredoxin-4 | PRDX1; PRDX4 | 3.006 | 2.87 | 3.052 |
| P69905 | Hemoglobin subunit alpha | HBA1 | 4.877 | 5.88 | 5.135 |
| Q13404-8;Q15819; Q13404;Q13404-7; Q13404-2;Q13404-1 | Ubiquitin-conjugating enzyme E2 variant 1;Ubiquitin-conjugating enzyme E2 variant 2 | UBE2V1; UBE2V 2 | 3.279 | 2.76 | 2.538 |
| P06703 | Protein S100-A6 | S100A6 | 4.041 | 4.37 | 4.052 |
| P60174-1;P60174; P60174-4 | Triosephosphate isomerase | TPI1 | 2.433 | 2.71 | 2.574 |
| P62937;P62937-2 | Peptidyl-prolyl cis-trans isomerase A; Peptidyl-prolyl cis-trans isomerase A, N-terminally processed | PPIA | 2.957 | 2.84 | 2.631 |
| P49746-2; P49746 | Thrombospondin-3 | THBS3 | 3.863 | 1.19 | 1.208 |

(continued)

| P09486 | SPARC | SPARC | 4.177 | 5.05 | 4.681 |
|---|---|---|---|---|---|
| Q14766-3;Q14766-2; Q14766-5;Q14766; Q14766-4 | Latent-transforming growth factor beta-binding protein 1 | LTBP1 | 6.967 | 2.71 | 6.079 |
| P01137 | Transforming growth factor beta-1; Latency-associated peptide | TGFB1 | 4.8 | 5.77 | 2.667 |
| P61088;Q5JXB2 | Ubiquitin-conjugating enzyme E2 N; Putative ubiquitin-conjugating enzyme E2 N-like | UBE2N; UBE2N L | 2.742 | 2.92 | 3.356 |
| P00558-2;P00558 | Phosphoglycerate kinase 1 | PGK1 | 2.278 | 2.02 | 2.685 |
| P07996;P07996-2 | Thrombospondin-1 | THBS1 | 8.012 | 8.27 | 6.402 |
| P02776 | Platelet factor 4;Platelet factor 4, short form | PF4 | 6.872 | 7.55 | 7.134 |
| P05067-7;P05067-11 ; P05067-8;P05067-9; P05067;P05067-10; P05067-3;P05067-4; P05067-5;P05067-6 | Amyloid beta A4 protein;N-APP;Soluble APP-alpha;Soluble APP-beta;C99; Beta-amyloid protein 42;Beta-amyloid protein 40;C83;P3(42);P3(40);C80;Gamm a-secretase C-terminal fragment 59; Gamma-secretase C-terminal fragment 57;Gamma-secretase C-terminal fragment 50;C31 | APP | 5.951 | 3.84 | 5.453 |
| P00568 | Adenylate kinase isoenzyme 1 | AK1 | 2.762 | 3.62 | 2.942 |
| P00918 | Carbonic anhydrase 2 | CA2 | 2.721 | 2.78 | 3.387 |
| P32119 | Peroxiredoxin-2 | PRDX2 | 3.697 | 3.66 | 3.939 |
| P16070-18;P16070-12; P16070-14;P16070-13; P16070-11;P16070-10; P16070-16;P16070-8; P16070-17;P16070-6; P16070-4;P16070-3; P16070-7;P16070-5; P16070;P16070-15; P16070-9 | CD44 antigen | CD44 | 2.047 | 1.83 | 1.814 |
| Q13201 | Multimerin-1 ;Platelet glycoprotein Ia*;155 kDa platelet multimerin | MMRN1 | 4.569 | 4.27 | 3.898 |
| P27348 | 14-3-3 protein theta | YWHAQ | 1.983 | 2.65 | 1.942 |
| O00592-2;O00592 | Podocalyxin | PODXL | 1.953 | 2.03 | 2.009 |
| P10599-2;P10599 | Thioredoxin | TXN | 2.744 | 1.94 | 2.597 |
| P00915 | Carbonic anhydrase 1 | CA1 | 3.741 | 3.62 | 4.046 |
| P62158 | Calmodulin | CALM1 | 2.102 | 3.05 | 2.229 |
| P58546 | Myotrophin | MTPN | 1.836 | 2.09 | 1.787 |
| P62258;P62258-2 | 14-3-3 protein epsilon | YWHAE | 2.002 | 1.16 | 1.448 |
| O94919 | Endonuclease domain-containing 1 protein | ENDOD1 | 3.075 | 2.53 | 3.181 |
| P37837 | Transaldolase | TALDO1 | 2.995 | 1.02 | 0.806 |
| P07738 | Bisphosphoglycerate mutase | BPGM | 2.761 | 3.14 | 2.939 |

(continued)

| | | | | | |
|---|---|---|---|---|---|
| Q13201 | Multimerin-1 ;Platelet glycoprotein Ia*;155 kDa platelet multimerin | MMRN1 | 4.569 | 4.27 | 3.898 |
| P30043 | Flavin reductase (NADPH) | BLVRB | 2.985 | 3.09 | 2.68 |
| P02775 | Platelet basic protein;Connective tissue-activating peptide III;TC-2;Connective tissue-activating peptide III(1-81);Beta-thromboglobulin;Neutrophil-activating peptide 2(74);Neutrophil-activating peptide 2(73);Neutrophil-activating peptide 2;TC-1;Neutrophil-activating peptide 2(1-66); Neutrophil-activating peptide 2(1-63) | PPBP | 12.41 | 9.69 | 11.867 |
| P15311 | Ezrin | EZR | 2.116 | 2.1 | 2.294 |
| P0DJI8;P0DJI9-2 | Serum amyloid A-1 protein;Amyloid protein A;Serum amyloid protein A(2-104); Serum amyloid protein A(3-104);Serum amyloid protein A(2-103);Serum amyloid protein A(2-102);Serum amyloid protein A (4-101);Serum amyloid A-2 protein | SAA1;SAA2 | 3.374 | 4.17 | 3.645 |
| P10124 | Serglycin | SRGN | 3.41 | 5.68 | 4.664 |
| P13798 | Acylamino-acid-releasing enzyme | APEH | 4.068 | 4.14 | 4.786 |
| P23528 | Cofilin-1 | CFL1 | 2.704 | 2.79 | 2.566 |
| P30041 | Peroxiredoxin-6 | PRDX6 | 2.111 | 2.01 | 1.81 |
| Q8WUM4;Q8WUM4-2 | Programmed cell death 6-interacting protein | PDCD6IP | 2.152 | 2.36 | 2.291 |
| P05090 | Apolipoprotein D | APOD | 2.213 | 2.51 | 2.381 |
| Q99497 | Protein deglycase DJ-1 | PARK7 | 3.668 | 2.22 | 2.939 |
| P35579;P35579-2 | Myosin-9 | MYH9 | 2.97 | 2.8 | 2.504 |
| Q13228;Q13228-4; Q13228-2;Q13228-3 | Selenium-binding protein 1 | SELENBP1 | 2.032 | 2.33 | 1.843 |
| P13716;P13716-2 | Delta-aminolevulinic acid dehydratase | ALAD | 1.983 | 2.28 | 2.524 |
| Q9ULI3-2;Q9ULI3 | Protein HEG homolog 1 | HEG1 | 2.16 | 1.95 | 2.819 |
| P07384 | Calpain-1 catalytic subunit | CAPN1 | 1.752 | 0.97 | 2.358 |
| P18065 | Insulin-like growth factor-binding protein 2 | IGFBP2 | 2.507 | 2.87 | 2.833 |
| P04083 | Annexin A1 | ANXA1 | 2.684 | 8.03 | 3.645 |
| P0DMV8-2;P0DMV9; P0DMV8 | Heat shock 70 kDa protein 1A;Heat shock 70 kDa protein 1B | HSPA1A; HSPA 1B | 2.188 | 2.3 | 2.203 |
| P07911-3;P07911; P07911-5;P07911-4 | Uromodulin;Uromodulin, secreted form | UMOD | 1.565 | 1.85 | 2.121 |
| P15924;P15924-2; P15924-3 | Desmoplakin | DSP | 1.636 | 3.38 | 1.968 |

**[0103]**  [0084]

[Table 3B]

| UniProt ID | Protein name | Gene name | Relative expression level of protein: Average value of 3 patients/ Average value of 3 healthy individuals | | |
|---|---|---|---|---|---|
| | | | Data set TMT1 | Data set TMT2 | Data set TMT3 |
| Q9C0H2-3 | Protein tweety homolog 3 | TTYH3 | 0.511 | 0.619 | 0.5 |

The selected proteins are promising as biomarkers for diagnosing endometriosis. Figs. 1-1 to 1-10 show the graphs of relative expression levels of representative proteins among them.

(Example 2) Search for biomarkers by aptamer-binding protein analysis (SOMAscan®) using plasma of healthy human individuals and patients with endometriosis

(2-1) Obtainment of human plasma samples

Plasma from healthy individuals and endometriosis patients was purchased from Proteogenex.

(2-2) SOMAscan® analysis of plasma proteins

Low-expressed proteins in plasma samples (three healthy individuals and three endometriosis patients) were analyzed by SOMAscan (registered trademark, Somalogic). Specifically, aptamers that each specifically binds to 1310 proteins were prepared, mixed with plasma, and allowed to bind to proteins in the plasma. The proteins bound to the aptamers were then biotinylated, and the aptamer-protein complexes were purified with streptavidin beads. Next, the aptamers were eluted and detected by a microarray, thereby the expression levels of the proteins bound to the aptamers in the plasma were analyzed.

(2-3) Selection of biomarker candidates

The aptamer signal data of SOMAscan® were analyzed by statistical programming environment R and Microsoft Excel. According to the following criterion, 200 proteins for which altered expression was found in endometriosis patients were selected (Table 4A and Table 4B).

$$\frac{\text{The average value of the aptamer signal of the target protein in three patients with endometriosis}}{\text{The average value of the aptamer signal of the target protein in three healthy individuals}} \geqq 2 \text{ or } \leqq 0.5$$

In the above calculation, proteins with a maximum aptamer signal value of less than 1000 were considered to be below the detection limit and excluded from analysis.

[Table 4A]

| Protein name | UniProt ID | Entrez Gene ID | Entrez Gene Symbol | Aptamer signal: Average value of 3 patients / Average value of 3 healthy individuals |
|---|---|---|---|---|
| Pulmonary surfactant-associated protein D | P35247 | 6441 | SFTPD | 12.382 |
| Histone H1.2 | P16403 | 3006 | HIST1H1C | 11.014 |
| Sialic acid-binding Ig-like lectin 9 | Q9Y336 | 27180 | SIGLEC9 | 9.473 |
| Heterogeneous nuclear ribonucleoproteins A2/B1 | P22626 | 3181 | HNRNPA2B1 | 7.516 |
| Hexokinase-2 | P52789 | 3099 | HK2 | 6.671 |
| High mobility group protein B1 | P09429 | 3146 | HMGB1 | 5.584 |

(continued)

| Protein name | UniProt ID | Entrez Gene ID | Entrez Gene Symbol | Aptamer signal: Average value of 3 patients / Average value of 3 healthy individuals |
|---|---|---|---|---|
| Thrombospondin-1 | P07996 | 7057 | THBS1 | 5.365 |
| 3-hydroxy-3-methylglutaryl-coenzyme A reductase | P04035 | 3156 | HMGCR | 4.718 |
| Platelet factor 4 | P02776 | 5196 | PF4 | 4.686 |
| Protein S100-A9 | P06702 | 6280 | S100A9 | 4.681 |
| 40S ribosomal protein S3a | P61247 | 6189 | RPS3A | 4.5 |
| Annexin A6 | P08133 | 309 | ANXA6 | 4.484 |
| Inosine-5'-monophosphate dehydrogenase 1 | P20839 | 3614 | IMPDH1 | 4.417 |
| Tyrosine-protein kinase Fgr | P09769 | 2268 | FGR | 4.238 |
| Serine/threonine-protein kinase 17B | O94768 | 9262 | STK17B | 3.957 |
| Neutrophil-activating peptide 2 | P02775 | 5473 | PPBP | 3.826 |
| Estradiol 17-beta-dehydrogenase 1 | P14061 | 3292 | HSD17B1 | 3.787 |
| Connective tissue-activating peptide III | P02775 | 5473 | PPBP | 3.746 |
| Prostaglandin G/H synthase 2 | P35354 | 5743 | PTGS2 | 3.686 |
| Amyloid beta A4 protein | P05067 | 351 | APP | 3.667 |
| GTP-binding nuclear protein Ran | P62826 | 5901 | RAN | 3.605 |
| NAD-dependent protein deacetylase sirtuin-2 | Q8IXJ6 | 22933 | SIRT2 | 3.54 |
| Protein S100-A12 | P80511 | 6283 | S100A12 | 3.498 |
| Plasminogen activator inhibitor 1 | P05121 | 5054 | SERPINE1 | 3.367 |
| SUMO-conjugating enzyme UBC9 | P63279 | 7329 | UBE2I | 3.336 |
| Bactericidal permeability-increasing protein | P17213 | 671 | BPI | 3.321 |
| 6-phosphogluconate dehydrogenase, decarboxylating | P52209 | 5226 | PGD | 3.204 |
| Platelet-derived growth factor subunit B | P01127 | 5155 | PDGFB | 3.158 |
| Tyrosine-protein phosphatase non-receptor type 6 | P29350 | 5777 | PTPN6 | 3.117 |
| Metalloproteinase inhibitor 3 | P35625 | 7078 | TIMP3 | 3.113 |
| NudC domain-containing protein 3 | Q8IVD9 | 23386 | NUDCD3 | 3.112 |
| SPARC | P09486 | 6678 | SPARC | 3.046 |
| Protein 4.1 | P11171 | 2035 | EPB41 | 2.967 |
| Sex hormone-binding globulin | P04278 | 6462 | SHBG | 2.905 |
| Death-associated protein kinase 2 | Q9UIK4 | 23604 | DAPK2 | 2.834 |
| Hepatoma-derived growth factor-related protein 2 | Q7Z4V5 | 84717 | HDGFRP2 | 2.777 |
| Proto-oncogene vav | P15498 | 7409 | VAV1 | 2.723 |

(continued)

| Protein name | UniProt ID | Entrez Gene ID | Entrez Gene Symbol | Aptamer signal: Average value of 3 patients / Average value of 3 healthy individuals |
|---|---|---|---|---|
| Serine/threonine-protein kinase pim-1 | P11309 | 5292 | PIM1 | 2.694 |
| Heterogeneous nuclear ribonucleoprotein A/B | Q99729 | 3182 | HNRNPAB | 2.685 |
| Proliferation-associated protein 2G4 | Q9UQ80 | 5036 | PA2G4 | 2.654 |
| Extracellular superoxide dismutase [Cu-Zn] | P08294 | 6649 | SOD3 | 2.654 |
| Angiopoietin-1 | Q15389 | 284 | ANGPT1 | 2.643 |
| Acid sphingomyelinase-like phosphodiesterase 3a | Q92484 | 10924 | SMPDL3A | 2.631 |
| Peroxiredoxin-6 | P30041 | 9588 | PRDX6 | 2.624 |
| AMP Kinase (alpha1beta1gamma1) | Q13131 Q9Y478 P54619 | 5562 5564 5571 | PRKAA1 PRKAB1 PRKAG1 | 2.577 |
| Serum amyloid A-1 protein | P0DJI8 | 6288 | SAA1 | 2.556 |
| Insulin-like growth factor-binding protein 2 | P18065 | 3485 | IGFBP2 | 2.52 |
| Histone H2B type 2-E | Q16778 | 8349 | HIST2H2BE | 2.491 |
| Fibroblast growth factor 5 | P12034 | 2250 | FGF5 | 2.491 |
| Non-histone chromosomal protein HMG-14 | P05114 | 3150 | HMGN1 | 2.467 |
| Calcium/calmodulin-dependent protein kinase type II subunit delta | Q13557 | 817 | CAMK2D | 2.464 |
| Interstitial collagenase | P03956 | 4312 | MMP1 | 2.454 |
| ICOS ligand | O75144 | 23308 | ICOSLG | 2.444 |
| Calcium/calmodulin-dependent protein kinase type II subunit beta | Q13554 | 816 | CAMK2B | 2.425 |
| Inosine-5'-monophosphate dehydrogenase 2 | P12268 | 3615 | IMPDH2 | 2.398 |
| Dickkopf-related protein 4 | Q9UBT3 | 27121 | DKK4 | 2.375 |
| Glia-derived nexin | P07093 | 5270 | SERPINE2 | 2.365 |
| 14-3-3 protein beta/alpha | P31946 | 7529 | YWHAB | 2.357 |
| Macrophage-capping protein | P40121 | 822 | CAPG | 2.352 |
| ADP-ribosyl cyclase/cyclic ADP-ribose hydrolase 1 | P28907 | 952 | CD38 | 2.349 |
| SH2 domain-containing protein 1A | O60880 | 4068 | SH2D1A | 2.324 |
| Vacuolar protein sorting-associated protein VTA1 homolog | Q9NP79 | 51534 | VTA1 | 2.319 |
| Interleukin-1 beta | P01584 | 3553 | IL1B | 2.312 |
| Chloride intracellular channel protein 1 | O00299 | 1192 | CLIC1 | 2.306 |

(continued)

| Protein name | UniProt ID | Entrez Gene ID | Entrez Gene Symbol | Aptamer signal: Average value of 3 patients / Average value of 3 healthy individuals |
|---|---|---|---|---|
| MAP kinase-activated protein kinase 3 | Q16644 | 7867 | MAPKAPK3 | 2.298 |
| Mitogen-activated protein kinase 1 | P28482 | 5594 | MAPK1 | 2.295 |
| Choline/ethanolamine kinase | Q9Y259 | 1120 | CHKB | 2.293 |
| Creatine kinase B-type | P12277 | 1152 | CKB | 2.287 |
| DNA topoisomerase 1 | P11387 | 7150 | TOP1 | 2.286 |
| C-C motif chemokine 5 | P13501 | 6352 | CCL5 | 2.28 |
| C3a anaphylatoxin | P01024 | 718 | C3 | 2.276 |
| Copine-1 | Q99829 | 8904 | CPNE1 | 2.274 |
| Chymase | P23946 | 1215 | CMA1 | 2.27 |
| Mitogen-activated protein kinase kinase kinase 7:TGF-beta-activated kinase 1 and MAP3K7-binding protein 1 fusion | O43318 Q15750 | 6885 10454 | MAP3K7 TAB1 | 2.27 |
| Dickkopf-related protein 1 | O94907 | 22943 | DKK1 | 2.266 |
| Ephrin type-B receptor 4 | P54760 | 2050 | EPHB4 | 2.261 |
| 40S ribosomal protein S7 | P62081 | 6201 | RPS7 | 2.253 |
| Intercellular adhesion molecule 1 | P05362 | 3383 | ICAM1 | 2.248 |
| Ubiquitin+1, truncated mutation for UbB | P62979 | 6233 | RPS27A | 2.222 |
| Ubiquitin-conjugating enzyme E2 N | P61088 | 7334 | UBE2N | 2.219 |
| Moesin | P26038 | 4478 | MSN | 2.201 |
| Small ubiquitin-related modifier 3 | P55854 | 6613 | SUMO3 | 2.196 |
| G2/mitotic-specific cyclin-B1 | P14635 | 891 | CCNB1 | 2.19 |
| ATP-dependent RNA helicase DDX19B | Q9UMR2 | 11269 | DDX19B | 2.178 |
| Tyrosine-protein kinase CSK | P41240 | 1445 | CSK | 2.17 |
| Alpha-1-antitrypsin | P01009 | 5265 | SERPINA1 | 2.162 |
| Adenylate kinase isoenzyme 1 | P00568 | 203 | AK1 | 2.145 |
| Mothers against decapentaplegic homolog 3 | P84022 | 4088 | SMAD3 | 2.137 |
| Brain-derived neurotrophic factor | P23560 | 627 | BDNF | 2.136 |
| Atrial natriuretic factor | P01160 | 4878 | NPPA | 2.131 |
| Annexin A1 | P04083 | 301 | ANXA1 | 2.13 |
| Signal transducer and activator of transcription 1-alpha/beta | P42224 | 6772 | STAT1 | 2.12 |
| Angiopoietin-related protein 4 | Q9BY76 | 51129 | ANGPTL4 | 2.104 |
| Cadherin-12 | P55289 | 1010 | CDH12 | 2.098 |
| Stress-induced-phosphoprotein 1 | P31948 | 10963 | STIP1 | 2.096 |
| Glycylpeptide N-tetradecanoyltransferase 1 | P30419 | 4836 | NMT1 | 2.091 |

(continued)

| Protein name | UniProt ID | Entrez Gene ID | Entrez Gene Symbol | Aptamer signal: Average value of 3 patients / Average value of 3 healthy individuals |
|---|---|---|---|---|
| Peroxi redoxi n-1 | Q06830 | 5052 | PRDX1 | 2.077 |
| Oxidized low-density lipoprotein receptor 1 | P78380 | 4973 | OLR1 | 2.075 |
| VPS10 domain-containing receptor SorCS2 | Q96PQ0 | 57537 | SORCS2 | 2.059 |
| FACT complex subunit SSRP1 | Q08945 | 6749 | SSRP1 | 2.049 |
| Carbonic anhydrase 1 | P00915 | 759 | CA1 | 2.035 |
| Mitogen-activated protein kinase 11 | Q15759 | 5600 | MAPK11 | 2.034 |
| Triosephosphate isomerase | P60174 | 7167 | TPI1 | 2.029 |
| Neurexophilin-1 | P58417 | 30010 | NXPH1 | 2.018 |
| Platelet-derived growth factor subunit A | P04085 | 5154 | PDGFA | 2.017 |
| Interleukin-22 receptor subunit alpha-1 | Q8N6P7 | 58985 | IL22RA1 | 2.004 |

[Table 4B]

| Protein name | UniProt ID | Entrez Gene ID | Entrez Gene Symbol | Aptamer signal: Average value of 3 patients / Average value of 3 healthy individuals |
|---|---|---|---|---|
| Cystatin-SN | P01037 | 1469 | CST1 | 0.499 |
| Interleukin-22 | Q9GZX6 | 50616 | IL22 | 0.497 |
| Tyrosine-protein kinase Fyn | P06241 | 2534 | FYN | 0.497 |
| Biglycan | P21810 | 633 | BGN | 0.495 |
| Sonic hedgehog protein | Q15465 | 6469 | SHH | 0.492 |
| Cathepsin L2 | 060911 | 1515 | CTSV | 0.49 |
| Interferon alpha/beta receptor 1 | P17181 | 3454 | IFNAR1 | 0.486 |
| Ectodysplasin-A, secreted form | Q92838 | 1896 | EDA | 0.485 |
| Dynein light chain 1, cytoplasmic | P63167 | 8655 | DYNLL1 | 0.483 |
| Cytochrome c | P99999 | 54205 | CYCS | 0.482 |
| Lactoperoxidase | P22079 | 4025 | LPO | 0.48 |
| Tumor necrosis factor receptor superfamily member 19L | Q969Z4 | 84957 | RELT | 0.48 |
| Growth factor receptor-bound protein 2 | P62993 | 2885 | GRB2 | 0.467 |
| Ectonucleotide pyrophosphatase/ phosphodiesterase family member 7 | Q6UWV6 | 339221 | ENPP7 | 0.466 |
| Glucagon | P01275 | 2641 | GCG | 0.466 |
| Eukaryotic translation initiation factor 4 gamma 2 | P78344 | 1982 | EIF4G2 | 0.465 |
| C-X-C motif chemokine 10 | P02778 | 3627 | CXCL10 | 0.457 |

(continued)

| Protein name | UniProt ID | Entrez Gene ID | Entrez Gene Symbol | Aptamer signal: Average value of 3 patients / Average value of 3 healthy individuals |
|---|---|---|---|---|
| Platelet-derived growth factor receptor beta | P09619 | 5159 | PDGFRB | 0.453 |
| von Willebrand factor | P04275 | 7450 | VWF | 0.452 |
| Iduronate 2-sulfatase | P22304 | 3423 | IDS | 0.443 |
| cGMP-specific 3',5'-cyclic phosphodiesterase | O76074 | 8654 | PDE5A | 0.443 |
| Small glutamine-rich tetratricopeptide repeat-containing protein alpha | O43765 | 6449 | SGTA | 0.441 |
| Interleukin-25 | Q9H293 | 64806 | IL25 | 0.433 |
| MHC class I polypeptide-related sequence B | Q29980 | 4277 | MICB | 0.433 |
| C-C motif chemokine 7 | P80098 | 6354 | CCL7 | 0.429 |
| Lactadherin | Q08431 | 4240 | MFGE8 | 0.427 |
| Macrophage metalloelastase | P39900 | 4321 | MMP12 | 0.425 |
| Ubiquitin-like protein ISG15 | P05161 | 9636 | ISG15 | 0.423 |
| Fatty acid-binding protein, liver | P07148 | 2168 | FABP1 | 0.418 |
| Properdin | P27918 | 5199 | CFP | 0.417 |
| Stromelysin-2 | P09238 | 4319 | MMP10 | 0.415 |
| Protein FAM107B | Q9H098 | 83641 | FAM107B | 0.413 |
| Myosin-binding protein C, slow-type | Q00872 | 4604 | MYBPC1 | 0.413 |
| Serine/threonine-protein kinase Chk2 | O96017 | 11200 | CHEK2 | 0.41 |
| Xaa-Pro aminopeptidase 1 | Q9NQW7 | 7511 | XPNPEP1 | 0.395 |
| NT-3 growth factor receptor | Q16288 | 4916 | NTRK3 | 0.394 |
| Interleukin-5 receptor subunit alpha | Q01344 | 3568 | IL5RA | 0.392 |
| Ephrin type-A receptor 2 | P29317 | 1969 | EPHA2 | 0.391 |
| Peptidyl-prolyl cis-trans isomerase F, mitochondrial | P30405 | 10105 | PPIF | 0.387 |
| B-cell lymphoma 6 protein | P41182 | 604 | BCL6 | 0.384 |
| Cell adhesion molecule 1 | Q9BY67 | 23705 | CADM1 | 0.384 |
| Kinesin-like protein KIF23 | Q02241 | 9493 | KIF23 | 0.383 |
| Netrin receptor UNC5D | Q6UXZ4 | 137970 | UNC5D | 0.381 |
| Lymphocyte activation gene 3 protein | P18627 | 3902 | LAG3 | 0.377 |
| Serine protease HTRA2, mitochondrial | O43464 | 27429 | HTRA2 | 0.376 |
| Interleukin-22 receptor subunit alpha-2 | Q969J5 | 116379 | IL22RA2 | 0.375 |
| Caspase-3 | P42574 | 836 | CASP3 | 0.374 |
| Platelet-derived growth factor receptor alpha | P16234 | 5156 | PDGFRA | 0.372 |

(continued)

| Protein name | UniProt ID | Entrez Gene ID | Entrez Gene Symbol | Aptamer signal: Average value of 3 patients / Average value of 3 healthy individuals |
|---|---|---|---|---|
| Interleukin-20 | Q9NYY1 | 50604 | IL20 | 0.368 |
| Ferritin | P02794 P02792 | 2495 2512 | FTH1 FTL | 0.367 |
| Ephrin-A5 | P52803 | 1946 | EFNA5 | 0.361 |
| Amphoterin-induced protein 2 | Q86SJ2 | 347902 | AMIGO2 | 0.353 |
| Interleukin-2 | P60568 | 3558 | IL2 | 0.351 |
| Phospholipase A2 | P04054 | 5319 | PLA2G1B | 0.346 |
| Protein kinase C alpha type | P17252 | 5578 | PRKCA | 0.345 |
| Tumor necrosis factor ligand superfamily member 8 | P32971 | 944 | TNFSF8 | 0.343 |
| Endoglin | P17813 | 2022 | ENG | 0.342 |
| Gamma-enolase | P09104 | 2026 | ENO2 | 0.332 |
| C-X-C motif chemokine 9 | Q07325 | 4283 | CXCL9 | 0.329 |
| beta-nerve growth factor | P01138 | 4803 | NGF | 0.327 |
| Hepcidin | P81172 | 57817 | HAMP | 0.323 |
| Inorganic pyrophosphatase | Q15181 | 5464 | PPA1 | 0.319 |
| Kallikrein-8 | O60259 | 11202 | KLK8 | 0.314 |
| Semaphorin-6B | Q9H3T3 | 10501 | SEMA6B | 0.312 |
| Adapter molecule crk | P46108 | 1398 | CRK | 0.307 |
| Phosphoglycerate mutase 1 | P18669 | 5223 | PGAM1 | 0.298 |
| Proprotein convertase subtilisin/kexin type 7 | Q16549 | 9159 | PCSK7 | 0.298 |
| Pancreatic hormone | P01298 | 5539 | PPY | 0.297 |
| Interleukin-1 receptor type 1 | P14778 | 3554 | IL1R1 | 0.296 |
| C-X-C motif chemokine 11 | 014625 | 6373 | CXCL11 | 0.293 |
| Secreted frizzled-related protein 1 | Q8N474 | 6422 | SFRP1 | 0.288 |
| Inhibin beta A chain | P08476 | 3624 | INHBA | 0.284 |
| Immunoglobulin D | P01880 | 3495 50802 3535 | IGHD IGK@ IGL@ | 0.277 |
| Complement C3b | P01024 | 718 | C3 | 0.272 |
| Teratocarcinoma-derived growth factor 1 | P13385 | 6997 | TDGF1 | 0.27 |
| Brother of CDO | Q9BWV1 | 91653 | BOC | 0.269 |
| CD109 antigen | Q6YHK3 | 135228 | CD109 | 0.26 |
| Aminoacylase-1 | Q03154 | 95 | ACY1 | 0.259 |
| 60 kDa heat shock protein, mitochondrial | P10809 | 3329 | HSPD1 | 0.255 |

(continued)

| Protein name | UniProt ID | Entrez Gene ID | Entrez Gene Symbol | Aptamer signal: Average value of 3 patients / Average value of 3 healthy individuals |
|---|---|---|---|---|
| Importin subunit beta-1 | Q14974 | 3837 | KPNB1 | 0.253 |
| C-reactive protein | P02741 | 1401 | CRP | 0.251 |
| Ephrin type-A receptor 1 | P21709 | 2041 | EPHA1 | 0.242 |
| Semaphorin-6A | Q9H2E6 | 57556 | SEMA6A | 0.24 |
| SLIT and NTRK-like protein 5 | 094991 | 26050 | SLITRK5 | 0.233 |
| Leucine-rich repeat transmembrane protein FLRT3 | Q9NZU0 | 23767 | FLRT3 | 0.217 |
| dCTP pyrophosphatase 1 | Q9H773 | 79077 | DCTPP1 | 0.21 |
| Osteopontin | P10451 | 6696 | SPP1 | 0.206 |
| Formimidoyltransferase-cyclodeaminase | O95954 | 10841 | FTCD | 0.191 |
| Ephrin-B2 | P52799 | 1948 | EFNB2 | 0.175 |
| T-lymphocyte surface antigen Ly-9 | Q9HBG7 | 4063 | LY9 | 0.17 |
| Sialic acid-binding Ig-like lectin 14 | Q08ET2 | 100049587 | SIGLEC14 | 0.157 |
| N-acylethanolamine-hydrolyzing acid amidase | Q02083 | 27163 | NAAA | 0.146 |
| Endoplasmic reticulum aminopeptidase 1 | Q9NZ08 | 51752 | ERAP1 | 0.118 |
| Low affinity immunoglobulin gamma Fc region receptor II-a | P12318 | 2212 | FCGR2A | 0.053 |

The selected proteins are promising as biomarkers for diagnosing endometriosis. Figs. 2-1 to 2-10 show the graphs of expression levels of representative proteins among them.

(Example 3) Search for biomarkers by extracellular matrix degradation product analysis using plasma of healthy human individuals and patients with endometriosis

(3-1) Obtainment of human plasma samples

Plasma from healthy individuals and endometriosis patients was purchased from Proteogenex.

(3-2) Extracellular matrix measurement of plasma proteins

Plasma samples (three healthy individuals and three endometriosis patients) were analyzed by the ELISA system with an antibody specific to the cleavage site of the extracellular matrix, and the total amount of extracellular matrix and the degradation/synthesis state of extracellular matrix in the samples were analyzed (Nordic Bioscience). Specifically, with antibodies against the cleavage site contained in degradation products when type III, IV, V, or VI collagen, decorin, or nidogen was degraded, the expression level of each degradation product was analyzed. The result showed that the type V collagen MMP degradation product (C5M) was remarkably increased in two of the three patients (the C5M concentration was 10.2 ng/ml and 11.8 ng/ml in the plasma of the two patients with increased C5M), while the plasma C5M concentration was below the detection limit in all healthy individuals. Thus, it was suggested that C5M is promising as a biomarker for endometriosis. Fig. 3 shows the graph of the plasma concentration of the type V collagen MMP degradation product (C5M).

(Example 4) Search for biomarkers by Luminex xMAP™ technology using plasma of healthy human individuals and patients with endometriosis

(4-1) Obtainment of human plasma samples

Plasma of healthy individuals (five plasma samples at secretory stage and five plasma samples at proliferative stage) was purchased from Proteogenex. As plasma of endometriosis patients, a total of 111 plasma samples were obtained from 37 endometriosis patients, each before surgery, at 3 days after surgery, and at 1 month after surgery.

(4-2) Analysis of plasma proteins by Luminex xMAP™ technology

Proteins in plasma samples were analyzed by Luminex xMAP™ (a trademark of Luminex). The Luminex xMAP™ technology is a technology of staining macrobeads with two fluorescent dyes combined at various concentrations and immobilizing onto each bead a substance that binds to each analysis target, whereby multiple items can be simultaneously analyzed with a small amount of samples using the content of the fluorescent dyes as an identification code.
In this Example, 152 proteins were selected and analyzed using protein measurement panels. Specifically, an antibody that binds to a target protein was immobilized on beads for each panel, a plasma sample was reacted with the antibody on the beads, a reporter antibody (labeled with a fluorescent dye) against the target protein was further reacted, and the expression level of the target protein was analyzed by measuring the fluorescence intensity with two types of lasers by flow cytometry technology (using the Luminex100 device).

(4-3) Selection of biomarker candidates

The plasma concentrations of the obtained various proteins were analyzed by Microsoft Excel. According to the following criterion, 24 proteins for which altered expression was found in endometriosis patients were selected (Table 7A and Table 7B).

$$\frac{\text{The average value of the plasma concentration of the protein in 37 patients with endometriosis before surgery}}{\text{The average value of the plasma concentration of the protein in 10 samples from 5 healthy individuals at secretory and proliferative stages}} \geqq 2 \text{ or } \leqq 0.5$$

Of the 24 proteins selected, 12 proteins showed the highest value in endometriosis patients at the time of surgery and a decreased value one month after surgery, and also showed the lowest value in healthy individuals.

[Table 7A]

| UniProt ID | Protein name | Gene name | Abundance ratio | Patients with endometriosis (before surgery) > Patients with endometriosis (1 month after surgery) > Healthy individuals |
|---|---|---|---|---|
| Q15389 | Angiopoietin-1 (ANG-1) | ANGPT1 | 2.96 | ○ |
| P23560 | Brain-Derived Neurotrophic Factor (BDNF) | BDNF | 4. 03 | ○ |
| 094907 | Dickkopf-related protein 1 (DKK-1) | DKK1 | 2. 11 | ○ |
| P80511 | S100-A12 | S100A12 | 4. 67 | ○ |
| P42830 | Epithelial-Derived Neutrophil-Activating Protein 78 (ENA-78) | CXCL5 | 2. 52 | ○ |
| P02794 | Ferritin (FRTN) | FTH1 | 2.99 | |
| P09341 | Growth-Regulated alpha protein (GRO-alpha) | CXCL1 | 2.23 | ○ |
| P01137 | Latency-Associated Peptide of Transforming Growth Factor beta 1 (LAP TGF-b1) | TGFB1 | 2. 55 | ○ |
| Q8WXL0 | Luteinizing Hormone (LH) | LHB | 2. 26 | ○ |
| G3CBL7 | MHC class I chain-related protein A (MICA) | MICA | 2. 29 | |

(continued)

| UniProt ID | Protein name | Gene name | Abundance ratio | Patients with endometriosis (before surgery) > Patients with endometriosis (1 month after surgery) > Healthy individuals |
|---|---|---|---|---|
| P02775 | Platelet basic protein | PPBP | 2.92 | ○ |
| P01127 | Platelet-Derived Growth Factor BB (PDGF-BB) | PDGFB | 4.25 | ○ |
| P01236 | Prolactin (PRL) | PRL | 3.76 | ○ |
| P0DJI8; P0DJI9-2 | Serum amyloid A-1 protein;Amyloid protein A;Serum amyloid protein A(2-104);Serum amyloid protein A(3-104) ;Serum amyloid protein A(2-103);Serum amyloid protein A (2-102) ;Serum amyloid protein A(4-101); Serum amyloid A-2 protein | SAA1; SAA2 | 3.01 | |
| P13501 | T-Cell-Specific Protein RANTES (RANTES) | CCL5 | 2.18 | ○ |

[Table 7B]

| UniProt ID | Protein name | Gene name | Abundance ratio |
|---|---|---|---|
| P10645 | Chromogranin-A (CgA) | CHGA | 0. 38 |
| Q9GZV9 | Fibroblast growth factor 23 (FGF-23) | FGF23 | 0. 22 |
| P01266 | Thyroglobul in (TG) | TG | 0. 47 |

(Example 5) Search for biomarkers by LC-MS analysis using plasma of healthy human individuals and patients with endometriosis

(5-1) Obtainment of human plasma samples

Plasma of healthy individuals (five plasma samples at secretory stage and five plasma samples at proliferative stage) was purchased from Proteogenex. As plasma of endometriosis patients, a total of 125 plasma samples were obtained from 39 endometriosis patients, each before surgery, at 3 days after surgery, and at 1 month after surgery.

(5-2) LC-MS analysis of plasma proteins

With High Select™ Top14 Abundant Protein Depletion Mini Spin Columns (Thermo), highly abundant proteins in each plasma sample were bound to the column and removed by the method recommended by the manufacturer. The proteins in the flow-through fraction that did not bind to the column were precipitated by the methanol/chloroform method and dissolved in 8 M urea/400 mM ammonium bicarbonate solution. The dissolved proteins were reduced and alkylated, and then digested into peptides with lysyl endopeptidase and trypsin. The peptide solution was demineralized with Monospin C18 (GL Science) by the method recommended by the manufacturer.

The peptides were labeled with TMT10plex by the method recommended by the manufacturer. With AssayMAP reversed phase (RP-S) cartridges (Agilent technologies), the labeled peptides were fractionated by the High pH Reversed-Phase method with triethylamine. Each fraction was analyzed by the LC-MS analysis system in which Orbitrap Fusion Lumos (ThermoFisher scientific) was coupled to the nano-LC system (EASY-nLC™ 1200 system, ThermoFisher Scientific). The analysis samples were peptides obtained from the plasma of each of five healthy individuals (each at secretory stage and proliferative stage; a total of 10 samples) and 39 endometriosis patients (before surgery, at 3 days after surgery, and at 1 month after surgery; a total of 125 samples). The sample prepared by mixing an equal amount of seven samples from healthy individuals and seven samples from endometriosis patients, which were purchased for correction between measurements, was used as a control in each analysis, and 10 samples were used for each analysis. Each sample was analyzed three times, and they were integrated during analysis.

**EP 3 961 217 A1**

The proteins in the samples were identified from the raw data of the LC-MS analysis by Thermo Scientific™ Proteome Discoverer™, and their relative expression levels were analyzed. The database search for protein identification was performed with the following parameters.

Taxonomy: uniprot human
Static modifications: TMT6plex/ +229.163 Da (Any N-Terminus), Carbamidomethyl/ +57.021 Da (C, C-Terminus), TMT6plex / +229.163 Da (K, C-Terminus)
Dynamic Modifications: Oxidation/ +15.995 Da (M), Acetyl/ +42.011Da (N-Terminus)
FDR: Target FDR (Strict)/ 0.01, Target FDR (Relaxed)/ 0.05

The relative expression levels of the target proteins in the plasma samples from endometriosis patients before surgery relative to the target proteins in the plasma samples from healthy individuals were calculated by the analysis software (Proteome Discoverer).

(5-3) Selection of biomarker candidate molecules

The LC-MS result data were analyzed by Proteome Discoverer. From the relative expression level of each protein in the samples (10 samples from five healthy individuals at secretory stage and proliferative stage and samples from 39 endometriosis patients before surgery), 27 proteins for which altered expression was found in endometriosis patients were selected according to the following criteria (Table 8A and Table 8B).

- For the abundance ratio, P-Value ≤ 0.5 in ANOVA and tukey HSD post hoc; and
- Abundance ratios ≤ 0.6250 or ≥ 1.6000

$$\text{Abundance ratio} = \frac{\text{The median value of the relative expression level of the protein in 39 patients with endometriosis before surgery}}{\text{The median value of the relative expression level of the protein in 10 samples from 5 healthy individuals at secretory and proliferative stages}}$$

Of the 27 proteins selected, 16 proteins showed the highest value in endometriosis patients at the time of surgery and a decreased value one month after surgery, and also showed the lowest value in healthy individuals.

[Table 8A]

| UniProt ID | Protein name | Gene name | Abundance ratio | Patients with endometriosis (before surgery) > Patients with endometriosis (1 month after surgery) > Healthy individuals |
|---|---|---|---|---|
| P07996 | Thrombospondin-1 OS=Homo sapiens OX=9606 GN=THBS1 PE=1 SV=2 | THBS1 | 3. 149 | ○ |
| P69905 | Hemoglobin subunit alpha OS=Homo sapiens OX=9606 GN=HBA1 PE=1 SV=2 | HBA1 | 1. 717 | ○ |
| P32119 | Peroxiredoxin-2 OS=Homo sapiens OX=9606 GN=PRDX2 PE=1 SV=5 | PRDX2 | 1. 772 | ○ |
| P00918 | Carbonic anhydrase 2 OS=Homo sapiens OX=9606 GN=CA2 PE=1 SV=2 | CA2 | 1.821 | ○ |
| P30043 | Flavin reductase (NADPH) OS=Homo sapiens OX=9606 GN=BLVRB PE=1 SV=3 | BLVRB | 1.787 | ○ |
| 076011 | Keratin, type I cuticular Ha4 OS=Homo sapiens OX=9606 GN=KRT34 PE=1 SV=2 | KRT34 | 2.091 | ○ |

(continued)

| UniProt ID | Protein name | Gene name | Abundance ratio | Patients with endometriosis (before surgery) > Patients with endometriosis (1 month after surgery) > Healthy individuals |
|---|---|---|---|---|
| P13716 | Delta-aminolevulinic acid dehydratase OS=Homo sapiens OX=9606 GN=ALAD PE=1 SV=1 | ALAD | 1.867 | ○ |
| P00568 | Adenylate kinase isoenzyme 1 OS=Homo sapiens OX=9606 GN=AK1 PE=1 SV=3 | AK1 | 1.786 | ○ |
| P07738 | Bisphosphoglycerate mutase OS=Homo sapiens OX=9606 GN=BPGM PE=1 SV=2 | BPGM | 1. 735 | ○ |
| P02775 | Platelet basic protein OS=Homo sapiens OX=9606 GN=PPBP PE=1 SV=3 | PPBP | 3. 628 | ○ |
| P22392 | Nucleoside diphosphate kinase B OS=Homo sapiens OX=9606 GN=NME2 PE=1 SV=1 | NME2 | 1. 779 | ○ |
| P10124 | Serglycin OS=Homo sapiens OX=9606 GN=SRGN PE=1 SV=3 | SRGN | 3. 181 | ○ |
| AOAOC4DH67 | Immunoglobulin kappa variable 1-8 OS=Homo sapiens OX=9606 GN=IGKV1-8 PE=3 SV=1 | IGKV1-8 | 1. 615 | × |
| P63241 | Eukaryotic translation initiation factor 5A-1 OS=Homo sapiens OX=9606 GN=EIF5A PE=1 SV=2 | EIF5A | 1. 874 | ○ |
| P02776 | Platelet factor 4 OS=Homo sapiens OX=9606 GN=PF4 PE=1 SV=2 | PF4 | 3. 438 | ○ |
| AOAO75B6S2 | Immunoglobulin kappa variable 2D-29 OS=Homo sapiens OX=9606 GN-IGKV2D-29 PE=3 SV=1 | IGKV2D-29 | 2. 174 | × |
| Q9UKU6 | Thyrotropin-releasing hormone-degrading ectoenzyme OS=Homo sapiens OX=9606 GN=TRHDE PE=2 SV=1 | TRHDE | 1. 879 | ○ |
| Q8WUM4 | Programmed cell death 6-interacting protein OS=Homo sapiens OX=9606 GN=PDCD6IP PE=1 SV=1 | PDCD6IP | 1. 644 | × |
| P00441 | Superoxide dismutase [Cu-Zn] OS=Homo sapiens OX=9606 GN=SOD1 PE=1 SV=2 | SOD1 | 2. 01 | ○ |

[Table 8B]

| UniProt ID | Protein name | Gene name | Abundance ratio |
|---|---|---|---|
| P36980 | Complement factor H-related protein 2 OS=Homo sapiens OX=9606 GN=CFHR2 PE=1 SV=1 | CFHR2 | 0. 443 |
| PODOX3 | Immunoglobulin delta heavy chain OS=Homo sapiens OX=9606 PE=1 SV=1 | N/A | 0. 518 |
| P01880 | Immunoglobulin heavy constant delta OS=Homo sapiens OX=9606 GN=IGHD PE=1 SV=3 | IGHD | 0. 456 |
| Q8N6C8 | Leukocyte immunoglobulin-like receptor subfamily A member 3 OS=Homo sapiens OX=9606 GN=LILRA3 PE=1 SV=3 | LILRA3 | 0.318 |
| P47929 | Galectin-7 OS=Homo sapiens OX=9606 GN=LGALS7 PE=1 SV=2 | LGALS7 | 0. 533 |
| O75339 | Cartilage intermediate layer protein 1 OS=Homo sapiens OX=9606 GN=CILP PE=1 SV=4 | CILP | 0. 614 |
| 015389 | Sialic acid-binding Ig-like lectin 5 OS=Homo sapiens OX=9606 GN=SIGLEC5 PE=1 SV=1 | SIGLEC5 | 0. 546 |
| P35247 | Pulmonary surfactant-associated protein D OS=Homo sapiens OX=9606 GN=SFTPD PE=1 SV=3 | SFTPD | 0. 605 |

[Industrial Applicability]

The present disclosure proved that by measuring the concentration of a type V collagen MMP degradation product or the abundance of at least one marker selected from the group consisting of the markers shown in Table 1A, the markers shown in Table 2A, the markers shown in Table 1B, the markers shown in Table 2B, the markers shown in Table 5A, and the markers shown in Table 6A, the markers shown in Table 5B, and the markers shown in Table 6B in a sample obtained from a subject, whether or not the subject is affected by endometriosis can be diagnosed. The invention of the present disclosure allows for the diagnosis of endometriosis by non-invasive means and is highly useful in the diagnosis and treatment of the disease.

**Claims**

1. A method of diagnosing endometriosis, comprising measuring the abundance of at least one marker selected from the group consisting of a type V collagen MMP degradation product, the markers shown in Group 1A, the markers shown in Group 2A, the markers shown in Group 1B, the markers shown in Group 2B, the markers shown in Group 3A, the markers shown in Group 4A, the markers shown in Group 3B, and the markers shown in Group 4B in a sample obtained from a subject.

[Group 1A]

| UniProt ID | Protein name | Gene name |
|---|---|---|
| P02042 | Hemoglobin subunit delta | HBD |
| P68871 | Hemoglobin subunit beta;LVV-hemorphin-7;Spinorphin | HBB |
| P55072 | Transitional endoplasmic reticulum ATPase | VCP |
| P50395;P50395-2 | Rab GDP dissociation inhibitor beta | GDI2 |
| O43852-9;O43852-5;O43852-2; O43852;O43852-4;O43852-3; 043852-12;043852-13; 043852-14;043852-7;043852-15; 043852-11;O43852-8;O43852-10; O43852-6 | Calumenin | CALU |
| P04040 | Catalase | CAT |
| Q06830;Q13162 | Peroxiredoxin-1 ;Peroxiredoxin-4 | PRDX1; PRDX 4 |
| P69905 | Hemoglobin subunit alpha | HBA1 |
| Q13404-8;Q15819;Q13404; Q13404-7;Q13404-2;Q13404-1 | Ubiquitin-conjugating enzyme E2 variant 1;Ubiquitin-conjugating enzyme E2 variant 2 | UBE2V1; UBE2 V2 |
| P06703 | Protein S100-A6 | S100A6 |
| P60174-1;P60174;P60174-4 | Triosephosphate isomerase | TPI1 |
| P62937;P62937-2 | Peptidyl-prolyl cis-trans isomerase A;Peptidyl-prolyl cis-trans isomerase A, N-terminally processed | PPIA |
| P49746-2;P49746 | Thrombospondin-3 | THBS3 |
| P09486 | SPARC | SPARC |
| Q14766-3;Q14766-2;Q14766-5; Q14766;Q14766-4 | Latent-transforming growth factor beta-binding protein 1 | LTBP1 |
| P01137 | Transforming growth factor beta-1;Latency-associated peptide | TGFB1 |
| P61088;Q5JXB2 | Ubiquitin-conjugating enzyme E2 N;Putative ubiquitin-conjugating enzyme E2 N-like | UBE2N; UBE2 NL |
| P00558-2;P00558 | Phosphoglycerate kinase 1 | PGK1 |
| P07996;P07996-2 | Thrombospondin-1 | THBS1 |
| P02776 | Platelet factor 4;Platelet factor 4, short form | PF4 |
| P05067-7;P05067-11;P05067-8; P05067-9; P05067; P05067-10; P05067-3;P05067-4;P05067-5; P05067-6 | Amyloid beta A4 protein;N-APP;Soluble APP-alpha;Soluble APP-beta;C99;Beta-amyloid protein 42;Beta-amyloid protein 40;C83;P3(42);P3(40);C80;Gamm a-secretase C-terminal fragment 59;Gamma-secretase C-terminal fragment 57; Gamma-secretase C-terminal fragment 50;C31 | APP |
| P00568 | Adenylate kinase isoenzyme 1 | AK1 |
| P00918 | Carbonic anhydrase 2 | CA2 |
| P32119 | Peroxiredoxin-2 | PRDX2 |

(continued)

| UniProt ID | Protein name | Gene name |
|---|---|---|
| P16070-18;P16070-12; P16070-14;P16070-13; P16070-11;P16070-10; P16070-16;P16070-8;P16070-17; P16070-6;P16070-4;P16070-3; P16070-7;P16070-5;P16070; P16070-15;P16070-9 | CD44 antigen | CD44 |
| Q13201 | Multimerin-1 ;Platelet glycoprotein la*;155 kDa platelet multimerin | MMRN1 |
| P27348 | 14-3-3 protein theta | YWHAQ |
| 000592-2;000592 | Podocalyxin | PODXL |
| P10599-2;P10599 | Thioredoxin | TXN |
| P00915 | Carbonic anhydrase 1 | CA1 |
| P62158 | Calmodulin | CALM1 |
| P58546 | Myotrophin | MTPN |
| P62258;P62258-2 | 14-3-3 protein epsilon | YWHAE |
| 094919 | Endonuclease domain-containing 1 protein | ENDOD1 |
| P37837 | Transaldolase | TALDO1 |
| P07738 | Bisphosphoglycerate mutase | BPGM |
| P30043 | Flavin reductase (NADPH) | BLVRB |
| P02775 | Platelet basic protein;Connective tissue-activating peptide III; TC-2;Connective tissue-activating peptide III(1-81);Beta-thromboglobulin;Neutrophil-activating peptide 2(74); Neutrophil-activating peptide 2(73);Neutrophil-activating peptide 2;TC-1;Neutrophil-activating peptide 2(1-66); Neutrophil-activating peptide 2(1-63) | PPBP |
| P15311 | Ezrin | EZR |
| P0DJI8;P0DJI9-2 | Serum amyloid A-1 protein;Amyloid protein A;Serum amyloid protein A(2-104);Serum amyloid protein A(3-104);Serum amyloid protein A(2-103);Serum amyloid protein A(2-102); Serum amyloid protein A(4-101);Serum amyloid A-2 protein | SAA1 ; SAA2 |
| P10124 | Serglycin | SRGN |
| P13798 | Acylamino-acid-releasing enzyme | APEH |
| P23528 | Cofilin-1 | CFL1 |
| P30041 | Peroxiredoxin-6 | PRDX6 |
| Q8WUM4;Q8WUM4-2 | Programmed cell death 6-interacting protein | PDCD6IP |
| P05090 | Apolipoprotein D | APOD |
| Q99497 | Protein deglycase DJ-1 | PARK7 |
| P35579;P35579-2 | Myosin-9 | MYH9 |
| Q13228;Q13228-4;Q13228-2; Q13228-3 | Selenium-binding protein 1 | SELENBP1 |
| P13716;P13716-2 | Delta-aminolevulinic acid dehydratase | ALAD |
| Q9ULI3-2;Q9ULI3 | Protein HEG homolog 1 | HEG1 |

(continued)

| UniProt ID | Protein name | Gene name |
|---|---|---|
| P07384 | Calpain-1 catalytic subunit | CAPN1 |
| P18065 | Insulin-like growth factor-binding protein 2 | IGFBP2 |
| P04083 | Annexin A1 | ANXA1 |
| P0DMV8-2;PODMV9;P0DMV8 | Heat shock 70 kDa protein 1A;Heat shock 70 kDa protein 1B | HSPA1A; HSP A1B |
| P07911-3;P07911;P07911-5; P07911-4 | Uromodulin;Uromodulin, secreted form | UMOD |
| P15924;P15924-2;P15924-3 | Desmoplakin | DSP |

[Group 1B]

| UniProt ID | Protein name | Gene name |
|---|---|---|
| Q9C0H2-3 | Protein tweety homolog 3 | TTYH3 |

[Group 2A]

| Protein name | UniProt ID | Entrez Gene ID | Entrez Gene Symbol |
|---|---|---|---|
| Pulmonary surfactant-associated protein D | P35247 | 6441 | SFTPD |
| Histone H1.2 | P16403 | 3006 | HIST1H1C |
| Sialic acid-binding Ig-like lectin 9 | Q9Y336 | 27180 | SIGLEC9 |
| Heterogeneous nuclear ribonucleoproteins A2/B1 | P22626 | 3181 | HNRNPA2B1 |
| Hexokinase-2 | P52789 | 3099 | HK2 |
| High mobility group protein B1 | P09429 | 3146 | HMGB1 |
| Thrombospondin-1 | P07996 | 7057 | THBS1 |
| 3-hydroxy-3-methylglutaryl-coenzyme A reductase | P04035 | 3156 | HMGCR |
| Platelet factor 4 | P02776 | 5196 | PF4 |
| Protein S100-A9 | P06702 | 6280 | S100A9 |
| 40S ribosomal protein S3a | P61247 | 6189 | RPS3A |
| Annexin A6 | P08133 | 309 | ANXA6 |
| Inosine-5'-monophosphate dehydrogenase 1 | P20839 | 3614 | IMPDH1 |
| Tyrosine-protein kinase Fgr | P09769 | 2268 | FGR |
| Serine/threonine-protein kinase 17B | O94768 | 9262 | STK17B |
| Neutrophil-activating peptide 2 | P02775 | 5473 | PPBP |
| Estradiol 17-beta-dehydrogenase 1 | P14061 | 3292 | HSD17B1 |
| Connective tissue-activating peptide III | P02775 | 5473 | PPBP |
| Prostaglandin G/H synthase 2 | P35354 | 5743 | PTGS2 |
| Amyloid beta A4 protein | P05067 | 351 | APP |
| GTP-binding nuclear protein Ran | P62826 | 5901 | RAN |

(continued)

| Protein name | UniProt ID | Entrez Gene ID | Entrez Gene Symbol |
|---|---|---|---|
| NAD-dependent protein deacetylase sirtuin-2 | Q8IXJ6 | 22933 | SIRT2 |
| Protein S100-A12 | P80511 | 6283 | S100A12 |
| Plasminogen activator inhibitor 1 | P05121 | 5054 | SERPINE1 |
| SUMO-conjugating enzyme UBC9 | P63279 | 7329 | UBE2I |
| Bactericidal permeability-increasing protein | P17213 | 671 | BPI |
| 6-phosphogluconate dehydrogenase, decarboxylating | P52209 | 5226 | PGD |
| Platelet-derived growth factor subunit B | P01127 | 5155 | PDGFB |
| Tyrosine-protein phosphatase non-receptor type 6 | P29350 | 5777 | PTPN6 |
| Metalloproteinase inhibitor 3 | P35625 | 7078 | TIMP3 |
| NudC domain-containing protein 3 | Q8IVD9 | 23386 | NUDCD3 |
| SPARC | P09486 | 6678 | SPARC |
| Protein 4.1 | P11171 | 2035 | EPB41 |
| Sex hormone-binding globulin | P04278 | 6462 | SHBG |
| Death-associated protein kinase 2 | Q9UIK4 | 23604 | DAPK2 |
| Hepatoma-derived growth factor-related protein 2 | Q7Z4V5 | 84717 | HDGFRP2 |
| Proto-oncogene vav | P15498 | 7409 | VAV1 |
| Serine/threonine-protein kinase pim-1 | P11309 | 5292 | PIM1 |
| Heterogeneous nuclear ribonucleoprotein A/B | Q99729 | 3182 | HNRNPAB |
| Proliferation-associated protein 2G4 | Q9UQ80 | 5036 | PA2G4 |
| Extracellular superoxide dismutase [Cu-Zn] | P08294 | 6649 | SOD3 |
| Angiopoietin-1 | Q15389 | 284 | ANGPT1 |
| Acid sphingomyelinase-like phosphodiesterase 3a | Q92484 | 10924 | SMPDL3A |
| Peroxiredoxin-6 | P30041 | 9588 | PRDX6 |
| AMP Kinase (alpha1beta1gamma1) | Q13131 Q9Y478 P54619 | 5562 5564 5571 | PRKAA1 PRKAB1 PRKAG1 |
| Serum amyloid A-1 protein | P0DJI8 | 6288 | SAA1 |
| Insulin-like growth factor-binding protein 2 | P18065 | 3485 | IGFBP2 |
| Histone H2B type 2-E | Q16778 | 8349 | HIST2H2BE |
| Fibroblast growth factor 5 | P12034 | 2250 | FGF5 |
| Non-histone chromosomal protein HMG-14 | P05114 | 3150 | HMGN1 |
| Calcium/calmodulin-dependent protein kinase type II subunit delta | Q13557 | 817 | CAMK2D |
| Interstitial collagenase | P03956 | 4312 | MMP1 |
| ICOS ligand | O75144 | 23308 | ICOSLG |
| Calcium/calmodulin-dependent protein kinase type II subunit beta | Q13554 | 816 | CAMK2B |
| Inosine-5'-monophosphate dehydrogenase 2 | P12268 | 3615 | IMPDH2 |
| Dickkopf-related protein 4 | Q9UBT3 | 27121 | DKK4 |

(continued)

| Protein name | UniProt ID | Entrez Gene ID | Entrez Gene Symbol |
|---|---|---|---|
| Glia-derived nexin | P07093 | 5270 | SERPINE2 |
| 14-3-3 protein beta/alpha | P31946 | 7529 | YWHAB |
| Macrophage-capping protein | P40121 | 822 | CAPG |
| ADP-ribosyl cyclase/cyclic ADP-ribose hydrolase 1 | P28907 | 952 | CD38 |
| SH2 domain-containing protein 1A | O60880 | 4068 | SH2D1A |
| Vacuolar protein sorting-associated protein VTA1 homolog | Q9NP79 | 51534 | VTA1 |
| Interleukin-1 beta | P01584 | 3553 | IL1B |
| Chloride intracellular channel protein 1 | O00299 | 1192 | CLIC1 |
| MAP kinase-activated protein kinase 3 | Q16644 | 7867 | MAPKAPK3 |
| Mitogen-activated protein kinase 1 | P28482 | 5594 | MAPK1 |
| Choline/ethanolamine kinase | Q9Y259 | 1120 | CHKB |
| Creatine kinase B-type | P12277 | 1152 | CKB |
| DNA topoisomerase 1 | P11387 | 7150 | TOP1 |
| C-C motif chemokine 5 | P13501 | 6352 | CCL5 |
| C3a anaphylatoxin | P01024 | 718 | C3 |
| Copine-1 | Q99829 | 8904 | CPNE1 |
| Chymase | P23946 | 1215 | CMA1 |
| Mitogen-activated protein kinase kinase kinase 7:TGF-beta-activated kinase 1 and MAP3K7-binding protein 1 fusion | 043318 Q15750 | 6885 10454 | MAP3K7 TAB1 |
| Dickkopf-related protein 1 | O94907 | 22943 | DKK1 |
| Ephrin type-B receptor 4 | P54760 | 2050 | EPHB4 |
| 40S ribosomal protein S7 | P62081 | 6201 | RPS7 |
| Intercellular adhesion molecule 1 | P05362 | 3383 | ICAM1 |
| Ubiquitin+1, truncated mutation for UbB | P62979 | 6233 | RPS27A |
| Ubiquitin-conjugating enzyme E2 N | P61088 | 7334 | UBE2N |
| Moesin | P26038 | 4478 | MSN |
| Small ubiquitin-related modifier 3 | P55854 | 6613 | SUMO3 |
| G2/mitotic-specific cyclin-B1 | P14635 | 891 | CCNB1 |
| ATP-dependent RNA helicase DDX19B | Q9UMR2 | 11269 | DDX19B |
| Tyrosine-protein kinase CSK | P41240 | 1445 | CSK |
| Alpha-1-antitrypsin | P01009 | 5265 | SERPINA1 |
| Adenylate kinase isoenzyme 1 | P00568 | 203 | AK1 |
| Mothers against decapentaplegic homolog 3 | P84022 | 4088 | SMAD3 |
| Brain-derived neurotrophic factor | P23560 | 627 | BDNF |
| Atrial natriuretic factor | P01160 | 4878 | NPPA |
| Annexin A1 | P04083 | 301 | ANXA1 |
| Signal transducer and activator of transcription 1-alpha/beta | P42224 | 6772 | STAT1 |

(continued)

| Protein name | UniProt ID | Entrez Gene ID | Entrez Gene Symbol |
|---|---|---|---|
| Angiopoietin-related protein 4 | Q9BY76 | 51129 | ANGPTL4 |
| Cadherin-12 | P55289 | 1010 | CDH12 |
| Stress-induced-phosphoprotein 1 | P31948 | 10963 | STIP1 |
| Glycylpeptide N-tetradecanoyltransferase 1 | P30419 | 4836 | NMT1 |
| Peroxiredoxin-1 | Q06830 | 5052 | PRDX1 |
| Oxidized low-density lipoprotein receptor 1 | P78380 | 4973 | OLR1 |
| VPS10 domain-containing receptor SorCS2 | Q96PQ0 | 57537 | SORCS2 |
| FACT complex subunit SSRP1 | Q08945 | 6749 | SSRP1 |
| Carbonic anhydrase 1 | P00915 | 759 | CA1 |
| Mitogen-activated protein kinase 11 | Q15759 | 5600 | MAPK11 |
| Triosephosphate isomerase | P60174 | 7167 | TPI1 |
| Neurexophilin-1 | P58417 | 30010 | NXPH1 |
| Platelet-derived growth factor subunit A | P04085 | 5154 | PDGFA |
| Interleukin-22 receptor subunit alpha-1 | Q8N6P7 | 58985 | IL22RA1 |

[Group 2B]

| Protein name | UniProt ID | Entrez Gene ID | Entrez Gene Symbol |
|---|---|---|---|
| Cystatin-SN | P01037 | 1469 | CST1 |
| Interleukin-22 | Q9GZX6 | 50616 | IL22 |
| Tyrosine-protein kinase Fyn | P06241 | 2534 | FYN |
| Biglycan | P21810 | 633 | BGN |
| Sonic hedgehog protein | Q15465 | 6469 | SHH |
| Cathepsin L2 | 060911 | 1515 | CTSV |
| Interferon alpha/beta receptor 1 | P17181 | 3454 | IFNAR1 |
| Ectodysplasin-A, secreted form | Q92838 | 1896 | EDA |
| Dynein light chain 1, cytoplasmic | P63167 | 8655 | DYNLL1 |
| Cytochrome c | P99999 | 54205 | CYCS |
| Lactoperoxidase | P22079 | 4025 | LPO |
| Tumor necrosis factor receptor superfamily member 19L | Q969Z4 | 84957 | RELT |
| Growth factor receptor-bound protein 2 | P62993 | 2885 | GRB2 |
| Ectonucleotide pyrophosphatase/phosphodiesterase family member 7 | Q6UWV6 | 339221 | ENPP7 |
| Glucagon | P01275 | 2641 | GCG |
| Eukaryotic translation initiation factor 4 gamma 2 | P78344 | 1982 | EIF4G2 |
| C-X-C motif chemokine 10 | P02778 | 3627 | CXCL10 |

(continued)

| Protein name | UniProt ID | Entrez Gene ID | Entrez Gene Symbol |
|---|---|---|---|
| Platelet-derived growth factor receptor beta | P09619 | 5159 | PDGFRB |
| von Willebrand factor | P04275 | 7450 | VWF |
| Iduronate 2-sulfatase | P22304 | 3423 | IDS |
| cGMP-specific 3',5'-cyclic phosphodiesterase | O76074 | 8654 | PDE5A |
| Small glutamine-rich tetratricopeptide repeat-containing protein alpha | O43765 | 6449 | SGTA |
| Interleukin-25 | Q9H293 | 64806 | IL25 |
| MHC class I polypeptide-related sequence B | Q29980 | 4277 | MICB |
| C-C motif chemokine 7 | P80098 | 6354 | CCL7 |
| Lactadherin | Q08431 | 4240 | MFGE8 |
| Macrophage metalloelastase | P39900 | 4321 | MMP12 |
| Ubiquitin-like protein ISG15 | P05161 | 9636 | ISG15 |
| Fatty acid-binding protein, liver | P07148 | 2168 | FABP1 |
| Properdin | P27918 | 5199 | CFP |
| Stromelysin-2 | P09238 | 4319 | MMP10 |
| Protein FAM107B | Q9H098 | 83641 | FAM107B |
| Myosin-binding protein C, slow-type | Q00872 | 4604 | MYBPC1 |
| Serine/threonine-protein kinase Chk2 | O96017 | 11200 | CHEK2 |
| Xaa-Pro aminopeptidase 1 | Q9NQW7 | 7511 | XPNPEP1 |
| NT-3 growth factor receptor | Q16288 | 4916 | NTRK3 |
| Interleukin-5 receptor subunit alpha | Q01344 | 3568 | IL5RA |
| Ephrin type-A receptor 2 | P29317 | 1969 | EPHA2 |
| Peptidyl-prolyl cis-trans isomerase F, mitochondrial | P30405 | 10105 | PPIF |
| β-cell lymphoma 6 protein | P41182 | 604 | BCL6 |
| Cell adhesion molecule 1 | Q9BY67 | 23705 | CADM1 |
| Kinesin-like protein KIF23 | Q02241 | 9493 | KIF23 |
| Netrin receptor UNC5D | Q6UXZ4 | 137970 | UNC5D |
| Lymphocyte activation gene 3 protein | P18627 | 3902 | LAG3 |
| Serine protease HTRA2, mitochondrial | O43464 | 27429 | HTRA2 |
| Interleukin-22 receptor subunit alpha-2 | Q969J5 | 116379 | IL22RA2 |
| Caspase-3 | P42574 | 836 | CASP3 |
| Platelet-derived growth factor receptor alpha | P16234 | 5156 | PDGFRA |
| Interleukin-20 | Q9NYY1 | 50604 | IL20 |
| Ferritin | P02794 P02792 | 2495 2512 | FTH1 FTL |
| Ephrin-A5 | P52803 | 1946 | EFNA5 |
| Amphoterin-induced protein 2 | Q86SJ2 | 347902 | AMIGO2 |
| Interleukin-2 | P60568 | 3558 | IL2 |

(continued)

| Protein name | UniProt ID | Entrez Gene ID | Entrez Gene Symbol |
|---|---|---|---|
| Phospholipase A2 | P04054 | 5319 | PLA2G1B |
| Protein kinase C alpha type | P17252 | 5578 | PRKCA |
| Tumor necrosis factor ligand superfamily member 8 | P32971 | 944 | TNFSF8 |
| Endoglin | P17813 | 2022 | ENG |
| Gamma-enolase | P09104 | 2026 | ENO2 |
| C-X-C motif chemokine 9 | Q07325 | 4283 | CXCL9 |
| beta-nerve growth factor | P01138 | 4803 | NGF |
| Hepcidin | P81172 | 57817 | HAMP |
| Inorganic pyrophosphatase | Q15181 | 5464 | PPA1 |
| Kallikrein-8 | O60259 | 11202 | KLK8 |
| Semaphorin-6B | Q9H3T3 | 10501 | SEMA6B |
| Adapter molecule crk | P46108 | 1398 | CRK |
| Phosphoglycerate mutase 1 | P18669 | 5223 | PGAM1 |
| Proprotein convertase subtilisin/kexin type 7 | Q16549 | 9159 | PCSK7 |
| Pancreatic hormone | P01298 | 5539 | PPY |
| Interleukin-1 receptor type 1 | P14778 | 3554 | IL1R1 |
| C-X-C motif chemokine 11 | 014625 | 6373 | CXCL11 |
| Secreted frizzled-related protein 1 | Q8N474 | 6422 | SFRP1 |
| Inhibin beta A chain | P08476 | 3624 | INHBA |
| Immunoglobulin D | P01880 | 3495 50802 3535 | IGHD IGK@ IGL@ |
| Complement C3b | P01024 | 718 | C3 |
| Teratocarcinoma-derived growth factor 1 | P13385 | 6997 | TDGF1 |
| Brother of CDO | Q9BWV1 | 91653 | BOC |
| CD109 antigen | Q6YHK3 | 135228 | CD109 |
| Aminoacylase-1 | Q03154 | 95 | ACY1 |
| 60 kDa heat shock protein, mitochondrial | P10809 | 3329 | HSPD1 |
| Importin subunit beta-1 | Q14974 | 3837 | KPNB1 |
| C-reactive protein | P02741 | 1401 | CRP |
| Ephrin type-A receptor 1 | P21709 | 2041 | EPHA1 |
| Semaphorin-6A | Q9H2E6 | 57556 | SEMA6A |
| SLIT and NTRK-like protein 5 | 094991 | 26050 | SLITRK5 |
| Leucine-rich repeat transmembrane protein FLRT3 | Q9NZU0 | 23767 | FLRT3 |
| dCTP pyrophosphatase 1 | Q9H773 | 79077 | DCTPP1 |
| Osteopontin | P10451 | 6696 | SPP1 |
| Formimidoyltransferase-cyclodeaminase | O95954 | 10841 | FTCD |

(continued)

| Protein name | UniProt ID | Entrez Gene ID | Entrez Gene Symbol |
|---|---|---|---|
| Ephrin-B2 | P52799 | 1948 | EFNB2 |
| T-lymphocyte surface antigen Ly-9 | Q9HBG7 | 4063 | LY9 |
| Sialic acid-binding Ig-like lectin 14 | Q08ET2 | 100049587 | SIGLEC14 |
| N-acylethanolamine-hydrolyzing acid amidase | Q02083 | 27163 | NAAA |
| Endoplasmic reticulum aminopeptidase 1 | Q9NZ08 | 51752 | ERAP1 |
| Low affinity immunoglobulin gamma Fc region receptor II-a | P12318 | 2212 | FCGR2A |

[Group 3A]

| UniProt ID | Protein name | Gene name |
|---|---|---|
| Q15389 | Angiopoietin-1 (ANG-1) | ANGPT1 |
| P23560 | Brain-Derived Neurotrophic Factor (BDNF) | BDNF |
| 094907 | Dickkopf-related protein 1 (DKK-1) | DKK1 |
| P80511 | S100-A12 | S100A12 |
| P42830 | Epithelial-Derived Neutrophil-Activating Protein 78 (ENA-78) | CXCL5 |
| P02794 | Ferritin (FRTN) | FTH1 |
| P09341 | Growth-Regulated alpha protein (GRO-alpha) | CXCL1 |
| P01137 | Latency-Associated Peptide of Transforming Growth Factor beta 1 (LAP TGF-b1) | TGFB1 |
| Q8WXLO | Luteinizing Hormone (LH) | LHB |
| G3CBL7 | MHC class I chain-related protein A (MICA) | MICA |
| P02775 | Platelet basic protein | PPBP |
| PO1127 | Platelet-Derived Growth Factor BB (PDGF-BB) | PDGFB |
| P01236 | Prolactin (PRL) | PRL |
| PODJI8; PODJI9-2 | Serum amyloid A-1 protein;Amyloid protein A;Serum amyloid protein A(2-104); Serum amyloid protein A(3-104);Serum amyloid protein A(2-103);Serum amyloid protein A(2-102);Serum amyloid protein A(4-101);Serum amyloid A-2 protein | SAA1 ; SAA2 |
| P13501 | T-Cell-Specific Protein RANTES (RANTES) | CCL5 |

[Group 3B]

| UniProt ID | Protein name | Gene name |
|---|---|---|
| P10645 | Chromogranin-A (CgA) | CHGA |
| Q9GZV9 | Fibroblast growth factor 23 (FGF-23) | FGF23 |
| P01266 | Thyroglobulin (TG) | TG |

[Group 4A]

| UniProt ID | Protein name | Gene name |
|---|---|---|
| P07996 | Thrombospondin-1 OS=Homo sapiens OX=9606 GN=THBS1 PE=1 SV=2 | THBS1 |

(continued)

| UniProt ID | Protein name | Gene name |
|---|---|---|
| P69905 | Hemoglobin subunit alpha OS=Homo sapiens OX=9606 GN=HBA1 PE=1 SV=2 | HBA1 |
| P32119 | Peroxiredoxin-2 OS=Homo sapiens OX=9606 GN=PRDX2 PE=1 SV=5 | PRDX2 |
| P00918 | Carbonic anhydrase 2 OS=Homo sapiens OX=9606 GN=CA2 PE=1 SV=2 | CA2 |
| P30043 | Flavin reductase (NADPH) OS=Homo sapiens OX=9606 GN=BLVRB PE=1 SV=3 | BLVRB |
| O76011 | Keratin, type I cuticular Ha4 OS=Homo sapiens OX=9606 GN=KRT34 PE=1 SV=2 | KRT34 |
| P13716 | Delta-aminolevulinic acid dehydratase OS=Homo sapiens OX=9606 GN=ALAD PE=1 SV=1 | ALAD |
| P00568 | Adenylate kinase isoenzyme 1 OS=Homo sapiens OX=9606 GN=AK1 PE=1 SV=3 | AK1 |
| P07738 | Bisphosphoglycerate mutase OS=Homo sapiens OX=9606 GN=BPGM PE=1 SV=2 | BPGM |
| P02775 | Platelet basic protein OS=Homo sapiens OX=9606 GN=PPBP PE=1 SV=3 | PPBP |
| P22392 | Nucleoside diphosphate kinase B OS=Homo sapiens OX=9606 GN=NME2 PE=1 SV=1 | NME2 |
| P10124 | Serglycin OS=Homo sapiens OX=9606 GN=SRGN PE=1 SV=3 | SRGN |
| AOAOC4DH67 | Immunoglobulin kappa variable 1-8 OS=Homo sapiens OX=9606 GN=IGKV1-8 PE=3 SV=1 | IGKV1-8 |
| P63241 | Eukaryotic translation initiation factor 5A-1 OS=Homo sapiens OX=9606 GN=EIF5A PE=1 SV=2 | EIF5A |
| P02776 | Platelet factor 4 OS=Homo sapiens OX-9606 GN=PF4 PE=1 SV=2 | PF4 |
| A0A075B6S2 | Immunoglobulin kappa variable 2D-29 OS=Homo sapiens OX=9606 GN=IGKV2D-29 PE=3 SV=1 | IGKV2D-29 |
| Q9UKU6 | Thyrotropin-releasing hormone-degrading ectoenzyme OS=Homo sapiens OX=9606 GN=TRHDE PE=2 SV=1 | TRHDE |
| Q8WUM4 | Programmed cell death 6-interacting protein OS=Homo sapiens OX=9606 GN=PDCD6IP PE=1 SV=1 | PDCD6IP |
| P00441 | Superoxide dismutase [Cu-Zn] OS=Homo sapiens OX=9606 GN=SOD1 PE=1 SV=2 | SOD1 |

[Group 4B]

| UniProt ID | Protein name | Gene name |
|---|---|---|
| P36980 | Complement factor H-related protein 2 OS=Homo sapiens OX=9606 GN=CFHR2 PE=1 SV=1 | CFHR2 |
| P0D0X3 | Immunoglobulin delta heavy chain OS=Homo sapiens OX=9606 PE=1 SV=1 | N/A |
| P01880 | Immunoglobulin heavy constant delta OS=Homo sapiens OX=9606 GN=IGHD PE=1 SV=3 | IGHD |
| Q8N6C8 | Leukocyte immunoglobulin-like receptor subfamily A member 3 OS=Homo sapiens OX=9606 GN=LILRA3 PE=1 SV=3 | LILRA3 |
| P47929 | Galectin-7 OS=Homo sapiens OX=9606 GN=LGALS7 PE=1 SV=2 | LGALS7 |

(continued)

| UniProt ID | Protein name | Gene name |
|---|---|---|
| O75339 | Cartilage intermediate layer protein 1 OS=Homo sapiens OX=9606 GN=CILP PE=1 SV=4 | CILP |
| O15389 | Sialic acid-binding Ig-like lectin 5 OS=Homo sapiens OX=9606 GN=SIGLEC5 PE=1 SV=1 | SIGLEC5 |
| P35247 | Pulmonary surfactant-associated protein D OS=Homo sapiens OX=9606 GN=SFTPD PE=1 SV=3 | SFTPD |

2. A method of determining whether or not a subject is affected by endometriosis, comprising measuring the abundance of at least one marker selected from the markers set forth in claim 1 in a sample obtained from the subject.

3. A method of determining the extent of endometrial fibrosis in a subject, comprising measuring the abundance of at least one marker selected from the markers set forth in claim 1 in a sample obtained from the subject.

4. A method of determining the extent of endometrial adhesion in a subject, comprising measuring the abundance of at least one marker selected from the markers set forth in claim 1 in a sample obtained from the subject.

5. A method of predicting the degree of pain due to endometriosis in a subject affected or suspected of being affected by endometriosis, comprising measuring the abundance of at least one marker selected from the markers set forth in claim 1 in a sample obtained from the subject.

6. A method of determining the degree of progression of endometriosis in a subject affected or suspected of being affected by endometriosis, comprising measuring the abundance of at least one marker selected from the markers set forth in claim 1 in a sample obtained from the subject.

7. A method of monitoring pathological conditions of endometriosis in a subject affected or suspected of being affected by endometriosis, comprising measuring each of the abundance of at least one marker selected from the markers set forth in claim 1 in each of a plurality of samples collected from the subject at different points of time.

8. The method of any one of claims 1 to 7, wherein the abundance of the marker is a concentration of a polypeptide of the marker in a blood sample.

9. A kit for diagnosing endometriosis, comprising a reagent for measuring the abundance of at least one marker selected from the markers set forth in claim 1.

10. A kit for determining whether or not a subject is affected by endometriosis, comprising a reagent for measuring the abundance of at least one marker selected from the markers set forth in claim 1.

11. A kit for determining the extent of endometrial fibrosis in a subject, comprising a reagent for measuring the abundance of at least one marker selected from the markers set forth in claim 1.

12. A kit for determining the extent of endometrial adhesion in a subject, comprising a reagent for measuring the abundance of at least one marker selected from the markers set forth in claim 1.

13. A kit for predicting the degree of pain due to endometriosis in a subject affected or suspected of being affected by endometriosis, comprising a reagent for measuring the abundance of at least one marker selected from the markers set forth in claim 1.

14. A kit for determining the degree of progression of endometriosis in a subject affected or suspected of being affected by endometriosis, comprising a reagent for measuring the abundance of at least one marker selected from the markers set forth in claim 1.

15. A kit for monitoring pathological conditions of endometriosis in a subject affected or suspected of being affected by endometriosis, comprising a reagent for measuring the abundance of at least one marker selected from the markers set forth in claim 1, and instructions stating that the abundance of at least one marker selected from the markers set forth in claim 1 in each of a plurality of samples collected from the subject at different points of time is compared.

CALU:O43852

FIG. 1-1

CAT:P04040

FIG. 1-2

FIG. 1-3

HBB:P68871

FIG. 1-4

FIG. 1-5

FIG. 1-6

PRDX1;PRDX4:Q06830

FIG. 1-7

FIG. 1-8

FIG. 1-9

UBE2V1;UBE2V2:Q13404

FIG. 1-10

FIG. 2-1

FIG. 2-2

FIG. 2-3

IL1B:P01584

FIG. 2-4

FIG. 2-5

FIG. 2-6

FIG. 2-7

FIG. 2-8

FIG. 2-9

FIG. 2-10

FIG. 3

**INTERNATIONAL SEARCH REPORT**

| International application No. |
|---|
| PCT/JP2020/017595 |

**A. CLASSIFICATION OF SUBJECT MATTER**
G01N 33/68(2006.01)i
FI: G01N33/68
According to International Patent Classification (IPC) or to both national classification and IPC

**B. FIELDS SEARCHED**

Minimum documentation searched (classification system followed by classification symbols)
G01N33/68

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2003-531580 A (METRIOGENE BIOSCIENCES INC.) 28.10.2003 (2003-10-28) entire text, all drawings | 1-15 |
| A | JP 2002-325600 A (SCHERING AG) 12.11.2002 (2002-11-12) entire text, all drawings | 1-15 |
| A | JP 2009-168646 A (FUJI PHARMA CO., LTD.) 30.07.2009 (2009-07-30) entire text, all drawings | 1-15 |
| A | US 2016/0251718 A1 (THE REGENTS OF THE UNIVERSITY OF CALIFORNIA) 01.09.2016 (2016-09-01) entire text, all drawings | 1-15 |
| A | FASSBENDER, Amelie et al., "Update on Biomarkers for the Detection of Endometriosis", BioMed Research International, 2015, vol. 2015, Article ID 130854, 1-14 entire text, all drawings | 1-15 |

☒ Further documents are listed in the continuation of Box C.  ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 16 July 2020 (16.07.2020) | 28 July 2020 (28.07.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2020/017595

C (Continuation). DOCUMENTS CONSIDERED TO BE RELEVANT

| Category* | Citation of document, with indication, where appropriate, of the relevant passages | Relevant to claim No. |
|---|---|---|
| A | IRUNGU, Stella et al., "Discovery of non-invasive biomarkers for the diagnosis of endometriosis", Clinical Proteomics, 06 April 2019, 16:14, 1-16 entire text, all drawings | 1-15 |
| A | GRANDE, Giuseppe et al., "Cervical mucus proteome in endometriosis", Clinical Proteomics, 02 February 2017, 14:7, 1-11 entire text, all drawings | 1-15 |
| A | EK, Mal in et al., "AXINI in Plasma or Serum Is a Potential New Biomarker for Endometriosis", International Journal of Molecular Sciences, 07 January 2019, 20(1), 189, 1-12 entire text, all drawings | 1-15 |

Form PCT/ISA/210 (continuation of second sheet) (January 2015)

**EP 3 961 217 A1**

<table>
<tr><td rowspan="2" style="text-align:center;">INTERNATIONAL SEARCH REPORT</td><td>International application No.</td></tr>
<tr><td>PCT/JP2020/017595</td></tr>
</table>

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.: Claims 1-15 (parts using collagen type V MMP degradation products)

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.

PCT/JP2020/017595

<Continuation of Box No. III>

A technical feature of a method for diagnosing endometriosis comprising a step for measuring the existential quantity of markers in a sample obtained from a subject set forth in claim 1 does not make a contribution over the prior art in light of the disclosure of document 1 (see claims, etc.), and thus said technical feature cannot be considered a special technical feature. Therefore, claims 1-15 set forth at least 267 independent inventions including a step for measuring the existential quantity of 267 markers (excluding duplication) of "type V MMP degradation products, a marker represented by Group 1A, a marker represented by Group 2A, a marker represented by Group 1B, a marker represented by Group 2B, a marker represented by Group 3A, a marker represented by Group 4A, a marker represented by Group 3B, a marker represented by Group 4B."

Document 1: JP 2003-531580 A (METRIOGENE BIOSCIENCES INC.) 28.10.2003

INTERNATIONAL SEARCH REPORT

Information on patent family members

International application No.

PCT/JP2020/017595

| Patent Documents referred in the Report | Publication Date | Patent Family | Publication Date |
|---|---|---|---|
| JP 2003-531580 A | 28 Oct. 2003 | US 2002/0127555 A1 entire text, all drawings US 2006/0057584 A1 US 2012/0208869 A1 WO 2001/062959 A2 EP 1290218 A2 DE 60129018 T2 AU 3717801 A CA 2399259 A1 BR 108685 A AT 365227 T ES 2287100 T3 ES 2336817 T3 | |
| JP 2002-325600 A | 12 Nov. 2002 | US 2003/0077589 A1 entire text, all drawings EP 1191107 A2 DE 10048633 A1 | |
| JP 2009-168646 A | 30 Jul. 2009 | (Family: none) | |
| US 2016/0251718 A1 | 01 Sep. 2016 | WO 2015/050875 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018025982 A

**Non-patent literature cited in the description**

- **GIUDICE LC.** Endometriosis. *N Engl J Med,* 2010, vol. 362, 2389-98
- **ROGERS PA et al.** Research priorities for endometriosis. *Reprod Sci,* 2017, vol. 24, 202-226
- **BESTE MT et al.** Molecular network analysis of endometriosis reveals a role for c-Jun-regulated macrophage activation. *Sci Transl Med,* 2014, vol. 6, 222-216
- **TAYLOR HS et al.** Treatment of Endometriosis-Associated Pain with Elagolix, an Oral GnRH Antagonist. *N Engl J Med.,* 2017, vol. 377, 28-40
- **KOHLER G et al.** A dose-ranging study to determine the efficacy and safety of 1, 2, and 4mg of dienogest daily for endometriosis. *Int J Gynaecol Obstet.,* 2010, vol. 108, 21-5
- **GREENE R et al.** Diagnostic experience among 4,334 women reporting surgically diagnosed endometriosis. *Fertility and Sterility,* 2009, vol. 91, 32-39
- **MANDERSON L et al.** Circuit breaking: Pathways of treatment seeking for women with endometriosis in Australia. *Qualitative Health Research,* 2008, vol. 18, 522-534
- **FASSBENDER A et al.** Update on biomarkers for the detection of endometriosis. *Biomed Res Int,* 2015, vol. 2015, 130854
- *Molecular and Cellular Endocrinology,* 2016, vol. 428, 1-16
- *Clin Biochem,* May 2012, vol. 45 (7-8), 541-6